# EUROPEAN PATENT APPLICATION

(11) **EP 1 604 983 A1**
(43) Date of publication of application: **14.12.2005**
(21) Application number: 04721010.9
(22) Date of filing: 16.03.2004
(51) Int. Cl.: C07D 217/26, C07D 403/06, C07D 403/12, C07D 491/113, A61K 31/472, A61K 31/4741, A61P 43/00, A61P 29/00, A61P 35/00, A61P 15/00, A61P 15/08, A61P 15/10, A61P 15/14, A61P 37/00, A61P 5/48, A61P 3/10, A61P 3/02, A61P 3/06, A61P 17/00, A61P 19/02, A61P 19/08

(54) **RECEPTOR ANTAGONIST**

(30) Priority: 17.03.2003 JP 2003072709
(71) Applicant: Takeda Pharmaceutical Company Limited, Osaka 541-0045 (JP)
(72) Inventor: ITOH, Fumio, Tsukuba-shi, Ibaraki 3050821 (JP); HINUMA, Shuji, Tsukuba-shi, Ibaraki 3050821 (JP); KANZAKI, Naoyuki, c/o Takeda Pharmaceutical, Yodogawa-ku, Osaka-shi, Osaka (JP); BANNO, Yoshihiro, c/o Takeda Pharmaceutical, Yodogawa-ku, Osaka-shi, Osaka (JP); YOSHIDA, Hiromi, Yuki-gun, Ibar aki 3002741 (JP); MATSUMOTO, Hirokazu, Tsukuba-shi, Ibaraki 3050821 (JP)
(74) Representative: Rickard, Timothy Mark Adrian
(86) International application number: PCT/JP2004/003496
(87) International publication number: WO 2004/083184

(57) **Abstract**

A compound represented by the formula (I): 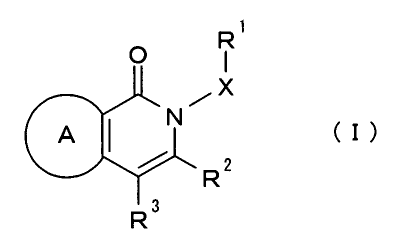 wherein ring A represents an aromatic ring; X represents a bond, oxygen, NR⁴ (R⁴ represents hydrogen, a hydrocarbon group, or a heterocyclic group), or alkylene; R¹ represents a hydrocarbon group, or a heterocyclic group; R² represents -COYR⁵ (Y represents a bond, alkylene, oxygen, sulfur, or NR⁶ (R⁶ represents hydrogen, a hydrocarbon group, or a heterocyclic group), and R⁵ represents a hydrocarbon group, or a heterocyclic group), a hydrocarbon group, or a heterocyclic group; and R³ represents a hydrocarbon group, a heterocyclic group, optionally substituted hydroxy, optionally substituted amino, or -S(O)ₙR⁷ (R⁷ represents a hydrocarbon group, or a heterocyclic group, and n is 0 to 2), a salt of the compound, or a prodrug or either is useful as an agent for modulating the function of an RFRP receptor.

## Description

### Technical Field

The present invention relates to an agent for modulating the function of an RFRP receptor which has an isoquinoline skeleton and is useful as a medicament such as an analgesic.

### Background Art

Secretion peptides called RFRP-1, RFRP-2 and RFRP-3, and a G protein conjugated-type receptor protein OT7T022 (hereinafter, abbreviated as RFRP receptor) to which the peptide is bound are known (WO 00/29441).

It is known that RFRP-1, RFRP-2 and RFRP-3 have prolactin secretion modulating activity (WO 01/66134).

It is known that RFRP-1 inhibits analgesic activity of morphine (Journal of Biological Chemistry, vol. 276, No. 40, pp. 36961-36969, 2001).

It is known that an isoquinoline compound has PDE V inhibitory activity, ACAT inhibitory activity, tachykinin antagonism (e.g., analgesic activity), antispastic activity, dipeptidyl peptidase (DPP) IV inhibitory activity and the like (JP 10-298164 A, JP 2000-72675 A, JP 2000-72751 A, EP-481383, EP-566069, EP-585913, EP-6344022, EP-652218, WO02/62764, Arch. Pharm., 324, 809-814 (1991)), but it is not known that an isoquinoline compound is bound to an RFRP receptor.

An object of the present invention is to provide a synthetic compound having excellent antagonism for an RFRP receptor.

### Disclosure of the Invention

In order to achieve the aforementioned object, the present inventors have studied intensively and, as a result, have found that a compound having an isoquinoline skeleton or a salt thereof has unexpectedly excellent RFRP receptor antagonism based on its specific chemical structure, further has excellent nature in physical properties as a medicament such as stability, and becomes a drug which is safe and useful as an analgesic, resulting in completion of the present invention based on these findings.

That is, the present invention relates to:
(1) An agent for modulating the function of an RFRP receptor, which comprises a compound represented by the formula: 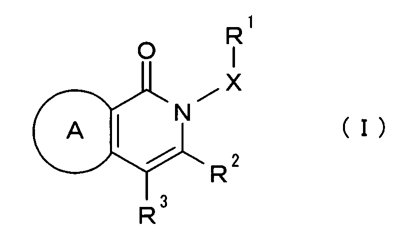 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof;
(2) The agent according to the above (1), wherein R³ is an optionally substituted hydroxy group;
(3) The agent according to the above (1), which comprises a compound represented by the formula: 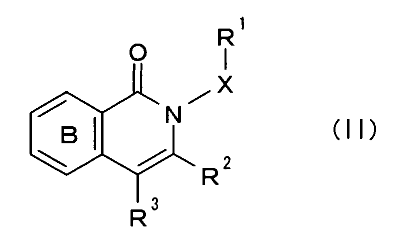 wherein a ring B represents an optionally substituted benzene ring; and the other symbols are as defined in the above (1), or a salt thereof, or a prodrug thereof;
(4) The agent according to the above (1), which comprises a compound represented by the formula: 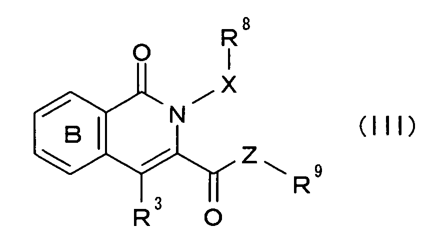 wherein a ring B represents an optionally substituted benzene ring; Z represents a bond, an optionally substituted alkylene group, O, S or NR¹⁰ (R¹⁰ represents a hydrogen atom, an optionally substituted hydrocarbon group, or an optionally substituted heterocyclic group); R⁸ and R⁹ each represents an optionally substituted branched hydrocarbon group; and the other symbols are as defined in the above (1), or a salt thereof, or a prodrug thereof;
(5) The agent according to the above (1), which comprises a compound represented by the formula: 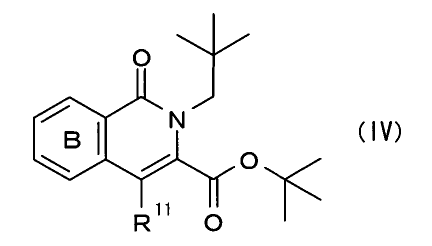 wherein a ring B represents an optionally substituted benzene ring; and R¹¹ represents an optionally substituted hydroxy group, or a salt thereof, or a prodrug thereof;
(6) The agent according to the above (1), which is an analgesic, an agent for promoting analgesic activity of other analgesic drugs, or an agent for avoiding resistance due to other analgesic drugs;
(7) The agent according to the above (1), which is an agent for modulating the prolactin secretion;
(8) The agent according to the above (1), which is an agent for preventing or treating hyperprolactinemia, pituitary gland tumor, diencephalons tumor, emmeniopathy, stress, autoimmune disease, prolactinoma, infertility, impotence, amenorrhea, galactic leakage, acromegaly, Chiari-Frommel syndrome, Argonz-del Castilo syndrome, Forbes-Albright syndrome, breast cancer lymphoma, Sheehan's syndrome or spermatogenesis abnormality;
(9) The agent according to the above (1), which is a pancreatic glucagon secretion inhibiting agent, a blood glucose lowering agent or a urine production inhibiting agent;
(10) The agent according to the above (1), which is an agent for preventing or treating diabetes, glucose tolerance disorder, ketosis, acidosis, diabetic neuropathy, diabetic nephropathy, diabetic retinopathy, pollakiuria, nocturnal enarusis, hyperlipemia, sexual function disorder, skin disease, arthritis, osteopenia, arteriosclerosis, thrombotic disease, maldigestion or memory and learning disabilities;
(11) The agent according to the above (1), which is a bladder constriction inhibiting agent;
(12) The agent according to the above (1), which is an agent for preventing or treating urine incontinence, lower uropathy, urge micturition due to excessive active bladder, or hypotonic bladder accompanied with excessive active bladder;
(13) A compound represented by the formula: 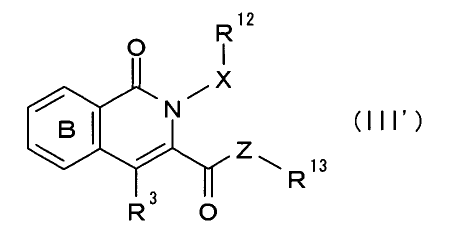 wherein a ring B represents an optionally substituted benzene ring; X represents a bond, O, NR⁴ (R⁴ represents a hydrogen atom, an optionally substituted hydrocarbon group, and an optionally substituted heterocyclic group), or an optionally substituted alkylene group; Z represents a bond, an optionally substituted alkylene group, O, S or NR¹⁰ (R¹⁰ represents a hydrogen atom, an optionally substituted hydrocarbon group, or an optionally substituted heterocyclic group); R¹² and R¹³ each represents an optionally substituted C₃ or higher hydrocarbon group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, provided that tert-butyl 6-fluoro-4-hydroxy-2-neopentyl-1-oxo-1,2-dihydro-3-isoquinolinecarboxylate, tert-butyl 4-butoxy-6-fluoro-2-neopentyl-1-oxo-1,2-dihydoro-3-isoquinolinecarboxylate, tert-butyl 7-benzyloxy-4-hydroxy-2-isobutyl-1-oxo-1,2-dihydro-3-isoquinolinecarboxylate and tert-butyl 6-benzyloxy-4-hydroxy-2-isobutyl-1-oxo-1,2-dihydro-3-isoquinolinecarboxylate are excluded;
(14) The compound according to the above (13), wherein X is a methylene group;
(15) The compound according to the above (13), wherein Z is an oxygen atom;
(16) The compound according to the above (13), wherein R¹² is a tert-butyl group;
(17) The compound according to the above (13), wherein R¹³ is a tert-butyl group;
(18) The compound according to the above (13), wherein R³ is an optionally substituted hydroxy group;
(19) The compound according to the above (13), which is represented by the formula: 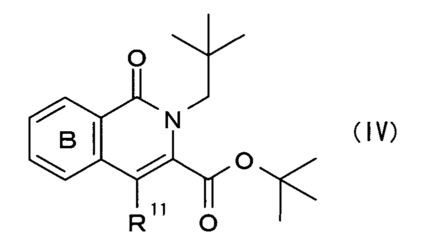 wherein a ring B represents an optionally substituted benzene ring; and R¹¹ represents an optionally substituted hydroxy group;
(20) (i) Ethyl 7-bromo-4-hydroxy-2-neopentyl-1-oxo-1,2-dihydroisoquinoline-3-carboxylate, (ii) ethyl 8-hydroxy-6-neopentyl-5-oxo-5,6-dihydro[1,3]dioxolo[4,5-g]isoquinoline-7-carboxylate, (iii) N-{2-[benzyl(methyl)amino]ethyl}-6,7-dichloro-4-methoxy-2-neopentyl-1-oxo-1,2-dihydro-3-isoquinolinecarboxamide, (iv) methyl 6,7-dichloro-4-hydroxy-2-neopentyl-1-oxo-1,2-dihydro-3-isoquinolinecarboxylate, (v) methyl 6,7-dichloro-4-methoxy-2-neopentyl-1-oxo-1,2-dihydro-3-isoquinolinecarboxylate, or a salt thereof;
(21) A prodrug of the compound according to the above (13) or (20);
(22) A drug comprising the compound according to the above (13) or (20) or a prodrug thereof;
(23) The drug according to the above (22), which is an agent for preventing or treating RFRP-relating disease states or diseases involving RFRP;
(24) A method of modulating the function of an RFRP receptor, which comprises administering an effective amount of a compound represented by the formula: 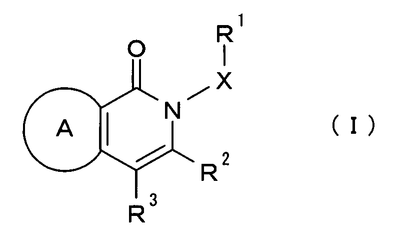 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof to a mammal;
(25) Use of a compound represented by the formula: 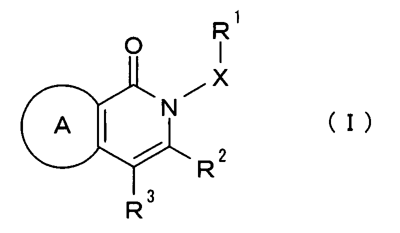 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof for preparing an agent for modulating the function of an RFRP receptor; and the like.

Further, the present invention relates to:
(26) the agent according to the above (1), wherein
   a ring A is (i) an aromatic hydrocarbon ring of a carbon number of 6 to 14, or (ii) a 5- to 14- membered aromatic heterocyclic ring containing 1 to 4 of 1 or 2 kinds of heteroatoms selected from a nitrogen atom, a sulfur atom and an oxygen atom in addition to a carbon atom, which may be substituted with a substituent selected from a substituent A group,
   the substituent A group is a group consisting of:
   (i) a halogen atom,
   (ii) a nitro group,
   (iii) a cyano group
   (iv) a straight or branched C₁₋₁₅ alkyl group, a C₃₋₁₀ cycloalkyl group, a C₂₋₁₈ alkenyl group, a C₃₋₁₀ cycloalkenyl group, a C₂₋₈ alkynyl group, a C₇₋₁₆ aralkyl group, a C₆₋₁₄ aryl group, a biphenyl group, or a tolyl group, which may be substituted with a substituent selected from a substituent B group [a nitro group, a hydroxyl group, an oxo group, a cyano group, a carbamoyl group, a mono- or di-C₁₋₆ alkyl-carbamoyl group (the alkyl group may be substituted with a halogen atom, a hydroxyl group, or a C₁₋₆ alkoxy group), a mono- or di-C₂₋₆ alkenyl-carbamoyl group (the alkenyl group may be substituted with a halogen atom, a hydroxyl group, or a C₁₋₆ alkoxy group), a mono- or diphenyl-carbamoyl group, a mono- or di-benzyl-carbamoyl group, a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-phenylamino-carbamoyl group, a carboxyl group, a C₁₋₆ alkoxy-carbonyl group, a sulfo group, a halogen atom, an optionally halogenated C₁₋₆ alkoxy group, a C₁₋₆ alkoxy group optionally substituted with a hydroxyl group, a C₁₋₆ alkoxy group optionally substituted with a carboxyl group, a C₁₋₆ alkoxy group optionally substituted with a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, a phenoxy group, a phenoxy-C₁₋₆ alkyl group, a phenoxy-C₁₋₆ alkoxy group, a C₁₋₆ alkylcarbonyl-oxy group, a carbamoyloxy group, a mono- or di-C₁₋₆ alkyl-carbamoyloxy group, an optionally halogenated phenyl group, an optionally halogenated phenyl-C₁₋₆ alkyl group, an optionally halogenated phenyl-C₂₋₆ alkenyl group, an optionally halogenated phenoxy group, a pyridyloxy group, a C₃₋₁₀ cycloalkyl group, a C₃₋₁₀ cycloalkyl-C₁₋₆ alkoxy group, a C₃₋₁₀ cycloalkyl-C₁₋₆ alkyl group, an optionally halogenated C₁₋₆ alkyl group, an optionally halogenated C₂₋₆ alkenyl group, an optionally halogenated C₁₋₆ alkylthio group, a C₁₋₆ alkyl group optionally substituted with a hydroxyl group, a C₁₋₆ alkylthio group optionally substituted with a hydroxy group, a mercapto group, a thioxo group, a benzyloxy group (optionally substituted with a substituent selected from a halogen atom, a carboxyl group and a C₁₋₆ alkoxy-carbonyl group) or a benzylthio group (optionally substituted with a substituent selected from a halogen atom, a carboxyl group and a C₁₋₆ alkoxy-carbonyl group), an optionally halogenated phenylthio group, a pyridylthio group, a phenylthio-C₁₋₆ alkyl group, a pyridylthio-C₁₋₆ alkyl group, an optionally halogenated C₁₋₆ alkylsulfinyl group, a phenylsulfinyl group, a phenylsulfinyl-C₁₋₆ alkyl group, an optionally halogenated C₁₋₆ alkylsulfonyl group, a phenylsulfonyl group, a phenylsulfonyl-C₁₋₆ alkyl group, an amino group, an aminosulfonyl group, a mono- or di-C₁₋₆ alkylaminosulfonyl group (the alkyl group may be substituted with a halogen atom, a hydroxy group, or a C₁₋₆ alkoxy group), a C₁₋₁₀ acylamino group (the C₁₋₁₀ acyl may be substituted with a halogen atom, a hydroxy group, or a carboxyl group), a benzoylamino group, a C₁₋₆ alkylsulfonylamino group, a C₆₋₁₀ arylsulfonylamino group, a benzyloxycarbonylamino group, an optionally halogenated C₁₋₆ alkoxycarbonylamino group, a carbamoylamino group, a mono- or di-C₁₋₆ alkylcarbamoylamino group, a mono- or di-C₁₋₆ alkylamino group (the alkyl group may be substituted with a halogen atom, a hydroxy group, or a C₁₋₆ alkoxy group), a mono- or di-C₁₋₆ alkanoylamino group (the alkanoyl group may be substituted with a halogen atom, a hydroxy group, or a C₁₋₆ alkoxy group), a phenylamino group, a benzylamino group, a C₁₋₆ alkyl(C₇₋₁₆ aralkyl) amino group, a C₁₋₆ alkanoyl(C₇₋₁₆ aralkyl) amino group, a 4- to 6-membered cyclic amino group, a 4- to 6-membered cyclic amino-carbonyl group, a 4- to 6-membered cyclic amino carbonyl-oxy group, a 4- to 6-membered cyclic amino-carbonyl-amino group, a 4- to 6-membered cyclic amino-sulfonyl group, a 4- to 6-membered cyclic amino-C₁₋₆ alkyl group, a C₁₋₆ acyl group (optionally substituted with a substituent selected from a halogen atom, a carboxyl group and a C₁₋₆ alkoxy-carbonyl group), a benzoyl group (optionally substituted with a substituent selected from a halogen atom, a carboxyl group and a C₁₋₆ alkoxy-carbonyl group), a benzoyl group optionally substituted with a halogen atom, a 5- to 10-membered heterocyclic group containing at least one 1 to 3 kinds of heteroatoms selected from an oxygen atom, a sulfur atom and a nitrogen atom (the heterocyclic group may be substituted with a C₁₋₆ alkyl group), a 5- to 10-membered heterocyclic-carbonyl group containing at least one 1 to 3 kinds of heteroatoms selected from an oxygen atom, a sulfur atom and a nitrogen atom (the heterocyclic group may be substituted with a C₁₋₆ alkyl group), a hydroxyimino group, a C₁₋₆ alkoxyimino group, an aryl group, an optionally halogenated straight or branched C₁₋₄ alkylenedioxy group, an ureido group, and a C₁₋₆ alkyl-ureido group] (hereinafter, optionally substituted hydrocarbon group),
   (v) a 5- to 16-membered aromatic heterocyclic group containing at least one 1 to 3 kinds of heteroatoms selected from an oxygen atom, a sulfur atom, and a nitrogen atom, or a saturated or unsaturated non-aromatic heterocyclic group, which may be substituted with a substituent selected from the substituent B group (hereinafter, optionally substituted heterocyclic group),
   (vi) a hydroxy group optionally substituted with a substituent selected from the group consisting of:
      (a) the aforementioned optionally substituted hydrocarbon group,
      (b) a group represented by R^{A}CO-, R^{A}OCO-, R^{A}SO₂-, R^{A}SO-or R^{A}OPO(OR^{B})- (R^{A} represents (aa) a hydrogen atom, (bb) the aforementioned optionally substituted hydrocarbon group or (cc) the aforementioned optionally substituted heterocyclic group, and R^{B} represents (aa) a hydrogen atom or (bb) the aforementioned optionally substituted hydrocarbon group) (hereinafter, acyl group),
      (c) a group represented by the formula -COOR^{C} (R^{C} represents (aa) a hydrogen atom, (bb) the aforementioned optionally substituted hydrocarbon group or (cc) the aforementioned optionally substituted heterocyclic group) (hereinafter, optionally esterified carboxyl group),
      (d) a carbamoyl group optionally substituted with a substituent selected from the group consisting of:
         (aa) the aforementioned optionally substituted hydrocarbon group,
         (bb) the aforementioned acyl group,
         (cc) the aforementioned optionally esterified carboxyl group,
         (dd) a carbamoyl group optionally substituted with 1 to 2 substituents selected from a C₁₋₆ alkyl group and a C₆₋₁₄ aryl group, and
         (ee) the aforementioned optionally substituted heterocyclic group
            (hereinafter, optionally substituted carbamoyl group), and
         (e) the aforementioned optionally substituted heterocyclic group
            (hereinafter, optionally substituted hydroxyl group),
   (vii) a thiol group optionally substituted with a substituent selected from the group consisting of:
      (a) the aforementioned optionally substituted hydrocarbon group,
      (b) the aforementioned acyl group,
      (c) the aforementioned optionally esterified carboxyl group,
      (d) the aforementioned optionally substituted carbamoyl group, and
      (e) the aforementioned optionally substituted heterocyclic group
         (hereinafter, optionally substituted thiol group),
   (viii) a sulfinyl group substituted with a substituent selected from the group consisting of:
      (a) the aforementioned optionally substituted hydroxy group,
      (b) the following optionally substituted amino group,
      (c) the aforementioned optionally substituted hydrocarbon group, and
      (d) the aforementioned optionally substituted heterocyclic group
         (hereinafter, substituted sulfinyl group),
   (ix) a sulfonyl group substituted with a substituent selected from the group consisting of:
      (a) the aforementioned optionally substituted hydroxy group,
      (b) the following optionally substituted amino group,
      (c) the aforementioned optionally substituted hydrocarbon group, and
      (d) the aforementioned optionally substituted heterocyclic group
         (hereinafter, substituted sulfonyl group),
   (x) an amino group optionally substituted with a substituent selected from the group consisting of:
      (a) the aforementioned optionally substituted hydrocarbon group,
      (b) the aforementioned acyl group,
      (c) the aforementioned optionally esterified carboxyl group,
      (d) the aforementioned optionally substituted carbamoyl group, and
      (e) the aforementioned optionally substituted heterocyclic group, (hereinafter, optionally substituted amino group),
   (xi) the aforementioned acyl group,
   (xii) the aforementioned optionally substituted carbamoyl group,
   (xiii) the aforementioned optionally esterified carboxyl group, and
   (xiv) a C₁₋₃ alkylenedioxy group,
   X is:
      (i) a bond,
      (ii) O,
      (iii) NR⁴
         (R⁴ is
         (a) a hydrogen atom,
         (b) the aforementioned optionally substituted hydrocarbon group, or
         (c) the aforementioned optionally substituted heterocyclic group), or
      (iv) a C₁₋₆ alkylene group optionally substituted with a substituent selected from the substituent B group,
         R¹ is:
         (i) the aforementioned optionally substituted hydrocarbon group, or
         (ii) the aforementioned optionally substituted heterocyclic group,
         R² is:
         (i) a group represented by the formula -COYR⁵
            (Y is
            (a) a bond
            (b) an C₁₋₆ alkylene group optionally substituted with a substituent selected from the substituent B group,
            (c) O
            (d) S, or
            (e) NR⁶ (R⁶ is
               (aa) a hydrogen atom
               (bb) the aforementioned optionally substituted hydrocarbon group, or
               (cc) the aforementioned optionally substituted heterocyclic group)
               R⁵ is

            (a) the aforementioned optionally substituted hydrocarbon group, or
            (b) the aforementioned optionally substituted heterocyclic group)
         (ii) the aforementioned optionally substituted hydrocarbon group, or
         (iii) the aforementioned optionally substituted heterocyclic group, and
            R³ is:
            (i) the aforementioned optionally substituted hydrocarbon group,
            (ii) the aforementioned optionally substituted heterocyclic group,
            (iii) the aforementioned optionally substituted hydroxy group,
            (iv) the aforementioned optionally substituted amino group, or
            (v) a group represented by the formula -S(O)ₙR⁷
               (R⁷ is
               (a) the aforementioned optionally substituted hydrocarbon group, or
               (b) the aforementioned optionally substituted heterocyclic group, and
   n is an integer of 0 to 2);
(27) The compound according to the above (13), wherein
   a ring B is a benzene ring optionally substituted with a substituent selected from the substituent A group,
   X is:
      (i) a bond,
      (ii) O,
      (iii) NR⁴
         (R⁴ is
         (a) a hydrogen atom,
         (b) the aforementioned optionally substituted hydrocarbon group, or
         (c) the aforementioned optionally substituted heterocyclic group), or
         (iv) a C₁₋₆ alkylene group optionally substituted with a substituent selected from the substituent B group,
   Z is:
      (i) a bond
      (ii) an C₁₋₆ alkylene group optionally substituted with a substituent selected from the substituent B group,
      (iii) O
      (iv) S, or
      (v) NR¹⁰ (R¹⁰ is
         (a) a hydrogen atom
         (b) the aforementioned optionally substituted hydrocarbon group, or
         (c) the aforementioned optionally substituted heterocyclic group)
   R¹² and R¹³ each is:
      (i) a straight or branched C₃₋₁₅ alkyl group or (ii) a C₃₋₁₀ cycloalkyl group, optionally substituted with a substituent selected from the substituent B group, and
         R³ is:
         (i) the aforementioned optionally substituted hydrocarbon group,
         (ii) the aforementioned optionally substituted heterocyclic group,
         (iii) the aforementioned optionally substituted hydroxy group,
         (iv) the aforementioned optionally substituted amino group, or
         (v) a group represented by the formula -S(O)ₙR⁷
            (R⁷ is

      (a) the aforementioned optionally substituted hydrocarbon group, or
      (b) the aforementioned optionally substituted heterocyclic group, and
n is an integer of 0 to 2), or a salt thereof; and the like.

### Brief Description of Drawings

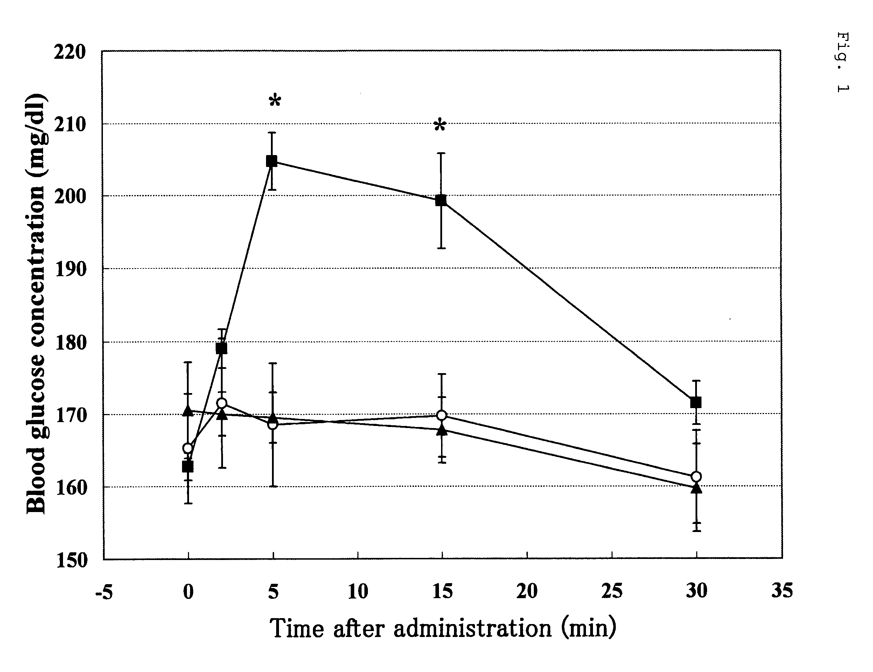

Fig. 1 shows the results of investigation of a variation in a blood glucose concentration upon intravenous administration of RFRP-1 to a rat under no anesthesia. In Fig. 1, (-○-) represents a blood glucose concentration of a physiological saline administration group, (-▲-) represents a blood glucose concentration of an RFRP-1 1 nmol/kg administration group, and (-■-) represents a blood concentration of an RFRP-1 10 nmol/kg administration group. A value indicates mean ± standard deviation (mean ± SE) (n = 4). * indicates that a P value is 0.05 or less as compared with a physiological saline administration group.

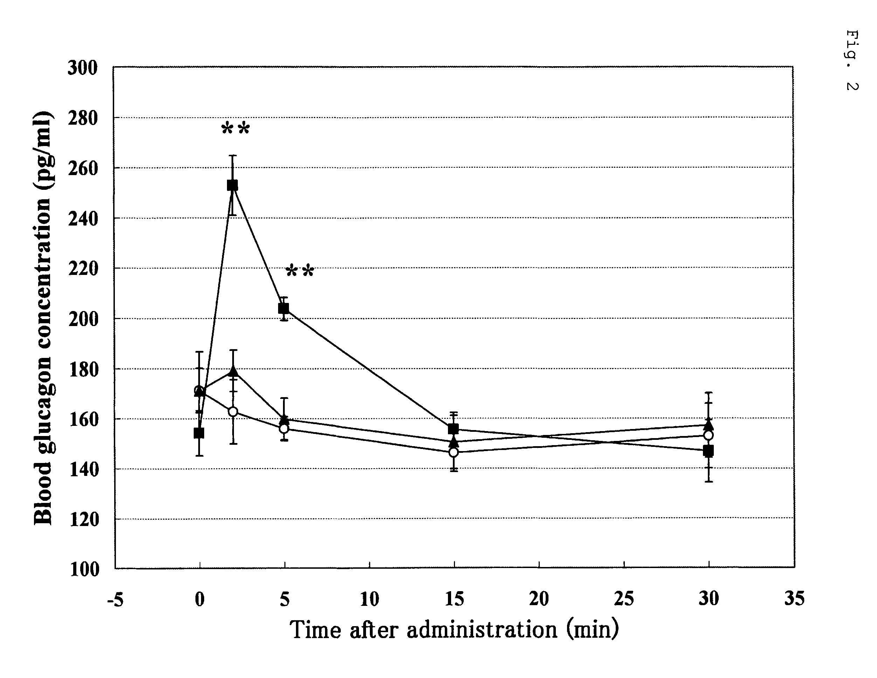

Fig. 2 shows the results of investigation of a variation in a blood glucagon concentration upon intravenous administration of RFRP-1 to a rat under no anesthesia. In Fig. 2, (-O-) represents a blood glucagon concentration of a physiological saline administration group, (-▲-) represents a blood glucagon concentration of an RFRP-1 1 nmol/kg administration group, and (-■-) represents a blood glucagon concentration of an RFRP-1 10 nmol/kg administration group. A value indicates mean ± standard deviation (mean ± SE) (n = 4). ** indicates that a P value is 0.01 or less as compared with a physiological saline administration group.

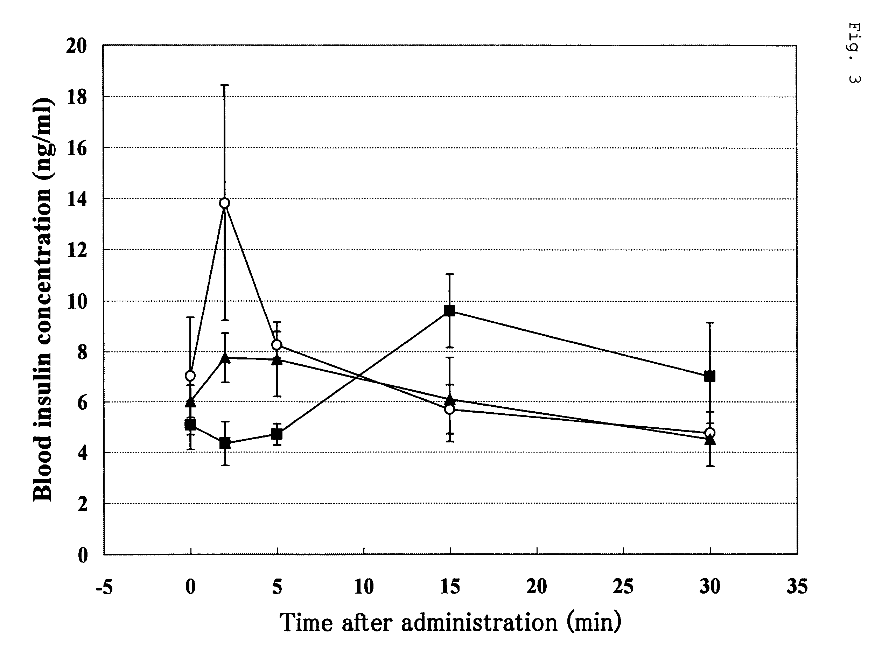

Fig. 3 shows the results of investigation of a variation in a blood insulin concentration upon intravenous administration of RFRP-1 to a rat under no anesthesia. In Fig. 3, (-○-) represents a blood insulin concentration of a physiological saline administration group, (-▲-) represents a blood insulin concentration of an RFRP-1 1 nmol/kg administration group, and (-■-) represents a blood insulin concentration of an RFRP-1 10 nmol/kg administration group. A value indicates mean ± standard deviation (mean ± SE) (n = 4).

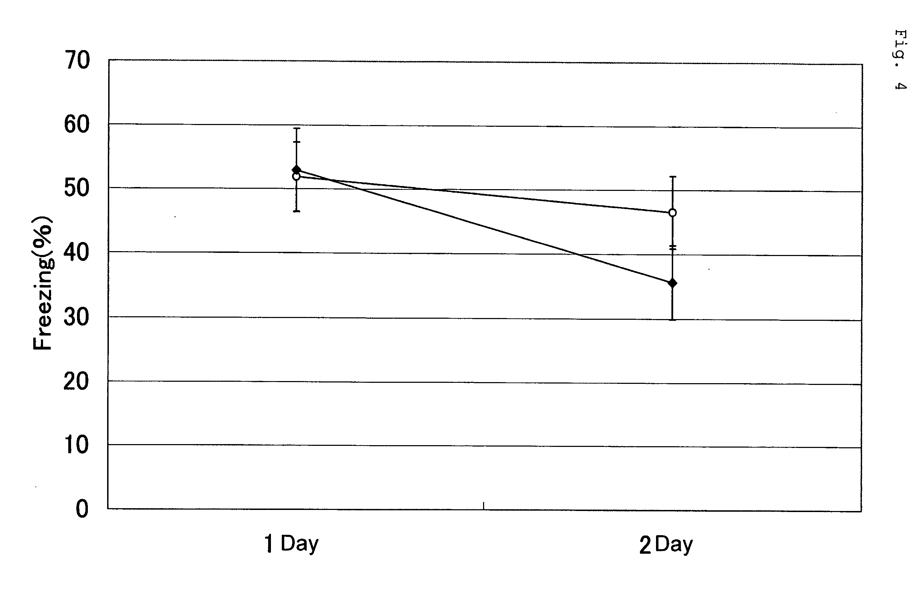

Fig. 4 indicates a ratio of freezing in cued fear conditioning when RFRP-1 (-◆-) and a physiological saline (-○-) are administered in a ventricle. An ordinate axis indicates freezing (%) of one day and two day after administration, respectively, expressed by mean ± standard error.

### Best Mode for Carrying Out the Invention

In the above formulas, the ring A represents an optionally substituted aromatic ring.

As the aromatic ring represented by the ring A, an aromatic hydrocarbon ring or an aromatic heterocyclic ring is used.

As the aromatic hydrocarbon ring, an aromatic hydrocarbon ring of a carbon number of 6 to 14 such as a benzene ring, and a naphthalene ring is used and, inter alia, a benzene ring is preferably used.

As the aromatic heterocyclic ring, for example, a 5-to 14-membered (monocyclic, dicyclic or tricyclic), preferably 5- to 10-membered, more preferably 5- to 6-membered aromatic heterocyclic ring containing 1 to 4 of 1 or 2 kinds of heteroatoms selected from a nitrogen atom, a sulfur atom and an oxygen atom in addition to carbon atoms is used. As the "5- to 14-membered (preferably 5- to 10-membered) aromatic heterocyclic ring", an aromatic heterocyclic ring such as thiophene, furan, oxazole, benzo[b]thiophene, benzo[b]furan, benzimidazole, benzoxazole, benzothiazole, benzisothiazole, naphtha[2,3-b]thiophene, pyrrole, imidazole, pyrazole, pyridine, pyrazine, pyrimidine, pyridazine, indole, isoindole, 1H-indazole, purine, 4H-quinolizine, isoquinoline, quinoline, phthalazine, naphthyridine, quinoxaline, quinazoline, cinnoline, carbazole, β-carboline, phenanthridine, acridine, phenazine, thiazole, isothiazole, phenothiazine, isooxazole, furazane, and phenoxazine, or a ring formed by fusing these rings (preferably monocycle) with one to plural (preferably 1 or 2) aromatic rings (e.g. benzene ring etc.) is used. Inter alia, a monocyclic aromatic heterocyclic ring having no basicity is preferable and, for example, a monocyclic aromatic heterocyclic ring such as thiophene, benzo[b]thiophene, benzo[b]furan, benzoxazole, benzothiazole, benzisothiazole, naphtha[2,3-b]thiophene, furan, indole, carbazole, thiazole, isothiazole, isooxazole, or a ring formed by fusing these rings (preferably monocycle) with one to plural (preferably 1 or 2) aromatic rings having no basicity (e.g. benzene ring etc.), etc. are used and, particularly thiophene is preferably used.

The ring B represents an optionally substituted benzene ring.

Examples of the substituent which may be possessed by the ring A or the ring B include a halogen atom (e.g. fluorine, chlorine, bromine, iodine), a nitro group, a cyano group, an optionally substituted hydrocarbon group, an optionally substituted heterocyclic group, an optionally substituted hydroxy group, an optionally substituted thiol group, a substituted sulfinyl group, a substituted sulfonyl group, an optionally substituted amino group, an acyl group, an optionally substituted carbamoyl group, an optionally esterified carboxyl group, and a C₁₋₃ alkylenedioxy group (hereinafter, substituent A group).

Examples of the "hydrocarbon group" of the "optionally substituted hydrocarbon group" as a substituent which may be possessed by the ring A or the ring B include an alkyl group, a cycloalkyl group, an alkenyl group, a cycloalkenyl group, an alkynyl group, an aralkyl group, an aryl group.

As the "alkyl group", a "straight or branched C₁₋₁₅ alkyl group" such as methyl, ethyl, propyl, isopropyl, butyl, isobutyl, sec-butyl, tert-butyl, pentyl, neopentyl, hexyl, heptyl, octyl, nonyl, decyl, undecyl, tridecyl, tetradecyl, and pentadecyl, preferably, a C₁₋₈ alkyl group is used. Further preferably, a C₁₋₆ alkyl group is used. Further preferably, a C₁₋₄ alkyl group is used.

As the "cycloalkyl group", a "C₃₋₁₀ cycloalkyl group" such as cyclopropyl, cyclobutyl, cyclopentyl, cyclohexyl, cycloheptyl, cyclooctyl, and adamantyl is used. More preferably, a C₃₋₈ cycloalkyl group is used. Further preferably, a C₅₋₇ cycloalkyl group is used.

As the "alkenyl group", a "C₂₋₁₈ alkenyl group" such as vinyl, allyl, isopropenyl, 3-butenyl, 3-octenyl, and 9-octadecenyl is used. Further preferably, a C₂₋₆ alkenyl group is used. More preferably, a C₂₋₄ alkenyl group is used.

As the "cycloalkenyl group", a "C₃₋₁₀ cycloalkenyl group" such as cyclopropenyl, cyclobutenyl, cyclopentenyl, cyclohexenyl, cycloheptenyl, and cyclooctenyl is used. More preferably, a C₃₋₈ cycloalkenyl group is used. Further preferably, a C₅₋₇ cycloalkenyl group is used.

As the "alkynyl group", a "C₂₋₈ alkynyl group" such as ethynyl, 1-propynyl, propargyl, 1-butynyl, 2-butynyl, 1-pentynyl, 2-pentynyl, and 3-pentynyl is used. More preferably, a C₂₋₆ alkynyl group is used. Further preferably, a C₂₋₄ alkynyl group is used.

As the "aralkyl group", a C₇₋₁₄ aralkyl group is used. Specifically, a phenyl-C₁₋₆ alkyl group such as benzyl, phenethyl, 3-phenylpropyl, and 4-phenylbutyl, and a naphthyl-C₁₋₆ alkyl group such as (1-naphthyl)methyl, 2-(1-naphthyl)ethyl, and 2-(2-naphthyl)ethyl are used.

As the "aryl group", an aromatic monocyclic, dicyclic or tricyclic C₆₋₁₄ aryl group such as phenyl, 1-naphthyl, 2-naphthyl, phenthryl, and anthryl, a biphenyl group, and a tolyl group are used. Preferably, a C₆₋₁₀ aryl group such as phenyl and naphthyl is used. Further preferably, phenyl is used.

Examples of the substituent which may be possessed by the "hydrocarbon group" in the "optionally substituted hydrocarbon group" as the substituent which may be possessed by the ring A or the ring B include (i) a nitro group, (ii) a hydroxy group, an oxo group, (iii) a cyano group, (iv) a carbamoyl group, (v) a mono- or di-C₁₋₆ alkyl-carbamoyl group (e.g. N-methylcarbamoyl, N-ethylcarbamoyl, N,N-dimethylcarbamoyl, N,N-diethylcarbamoyl etc.; the alkyl group may be substituted with a halogen atom, a hydroxy group, or a C₁₋₆ alkoxy group), a mono- or di-C₂₋₄ alkenyl-carbamoyl group (e.g. N-allylcarbamoyl etc.; the alkenyl group may be substituted with a halogen atom, a hydroxy group, a C₁₋₆ alkoxy group etc.), a mono- or di-phenylcarbamoyl group, a mono- or di-benzyl-carbamoyl group, a C₁₋₆ alkoxy-carbonyl-carbamoyl group, a C₁₋₆ alkylsulfonyl-carbamoyl group, a C₁₋₆ alkoxy-carbamoyl group, an amino-carbamoyl group, a mono- or di-C₁₋₆ alkylamino-carbamoyl group, a mono- or di-phenylamino-carbamoyl group, (vi) a carboxyl group, (vii) a C₁₋₆ alkoxy-carbonyl group (e.g. methoxycarbonyl, ethoxycarbonyl, propoxycarbonyl, isopropoxycarbonyl etc.), (viii) a sulfo group, (ix) a halogen atom (e.g. fluorine, chlorine, bromine, iodine), (x) an optionally halogenated C₁₋₆ alkoxy group (e.g. methoxy, ethoxy, propoxy, isopropoxy etc.), a C₁₋₆ alkoxy group optionally substituted with a hydroxy group, a C₁₋₆ alkoxy group optionally substituted with a carboxyl group, a C₁₋₆ alkoxy group optionally substituted with a C₁₋₆ alkoxy-carbonyl group, a C₁₋₆ alkoxy-C₁₋₆ alkoxy group, a C₁₋₆ alkoxy-C₁₋₆ alkoxy-C₁₋₆ alkoxy group, (xi) a phenoxy group, a phenoxy-C₁₋₆ alkyl group, a phenoxy-C₁₋₆ alkoxy group, a C₁₋₆ alkylcarbonyl-oxy group, a carbamoyloxy group, a mono- or di-C₁₋₆ alkyl-carbamoyloxy group, (xii) an optionally halogenated phenyl group, an optionally halogenated phenyl-C₁₋₆ alkyl group, an optionally halogenated phenyl-C₂₋₆ alkenyl group, an optionally halogenated phenoxy group (e.g. o-, m- or p-chlorophenoxy, o-, m- or p-bromophenoxy etc.), a pyridyloxy group, a C₃₋₁₀ cycloalkyl group, a C₃₋₁₀ cycloalkyl-C₁₋₆ alkoxy group, a C₃₋₁₀ cycloalkyl-C₁₋₆ alkyl group, (xiii) an optionally halogenated C₁₋₆ alkyl group (e.g. methyl, ethyl, propyl, isopropyl, butyl etc.), an optionally halogenated C₂₋₆ alkenyl group (e.g. vinyl, allyl, 2-butenyl, 3-butenyl etc.), an optionally halogenated C₁₋₆ alkylthio group (e.g. methylthio, ethylthio, n-propylthio, isopropylthio, n-butylthio etc.), a C₁₋₆ alkyl group optionally substituted with a hydroxy group, a C₁₋₆ alkylthio group optionally substituted with a hydroxy group, (xiv) a mercapto group, a thioxo group, (xv) a benzyloxy group or a benzylthio group, each optionally substituted with a substituent selected from a halogen atom, a carboxyl group and a C₁₋₆ alkoxy-carbonyl group, (xvi) an optionally halogenated phenylthio group, a pyridylthio group, a phenylthio-C₁₋₆ alkyl group, a pyridylthio-C₁₋₆ alkyl group, (xvii) an optionally halogenated C₁₋₆ alkylsulfinyl group (e.g. methylsulfinyl, ethylsulfinyl etc.), a phenylsulfinyl group, a phenylsulfinyl-C₁₋₆ alkyl group, (xviii) an optionally halogenated C₁₋₆ alkylsulfonyl group (e.g. methylsulfonyl, ethylsulfonyl etc.), a phenylsulfonyl group, a phenylsulfonyl-C₁₋₆ alkyl group, (xix) an amino group, an aminosulfonyl group, a mono- or di-C₁₋₆ alkylaminosulfonyl group (e.g. methylaminosulfonyl, ethylaminosulfonyl, N,N-dimethylaminosulfonyl, N,N-diethylaminosulfonyl etc.; the alkyl group may be substituted with a halogen atom, a hydroxy group, a C₁₋₆ alkoxy group etc.), (xx) a C₁₋₁₀ acylamino group [e.g. a C₁₋₆ alkanoylamino group (e.g. formylamino, acetylamino, trifluoroacetylamino, propionylamino, pivaloylamino etc.), benzoylamino, C₁₋₆ alkylsulfonylamino (e.g. methanesulfonylamino, trifluoromethanesulfonylamino etc.), C₆₋₁₀ arylsulfonylamino (e.g. benzenesulfonylamino, toluenesulfonylamino etc.); C₁-₁₀ acyl may be substituted with a halogen atom, a hydroxy group, a carboxyl group etc.], benzyloxycarbonylamino, optionally halogenated C₁₋₆ alkoxycarbonylamino, a carbamoylamino group, a mono- or di-C₁₋₆ alkylcarbamoylamino group, (xxi) a mono- or di-C₁₋₆ alkylamino group (e.g. methylamino, ethylamino, dimethylamino, diethylamino etc.; the alkyl group may be substituted with a halogen atom, a hydroxy group, a C₁₋₆ alkoxy group etc.), a mono- or di-C₁₋₆ alkanoylamino group (e.g. formylamino, acetylamino etc.; the alkanoyl group may be substituted with a halogen atom, a hydroxy group, a C₁₋₆ alkoxy group etc.), phenylamino, benzylamino, a C₁₋₆ alkyl(C₇₋₁₆ aralkyl)amino group (e.g. C₁₋₆ alkyl (benzyl) amino etc.), a C₁₋₆ alkanoyl (C₇₋₁₆ aralkyl) amino group (e.g. C₁₋₆ alkanoyl(benzyl)amino etc.), (xxii) a 4- to 6-membered cyclic amino group (e.g. 1-azetidinyl, 1-pyrrolidinyl, piperidino, morpholino, thiomorpholino, 1-piperazinyl etc., a 4- to 6-membered cyclic amino-carbonyl group (e.g. 1-azetidinylcarbonyl, 1-pyrrolidinylcarbonyl, piperidinocarbonyl, morpholinocarbonyl, thiomorpholinocarbonyl, 1-piperazinylcarbonyl etc.), a 4-to 6-membered cyclic amino-carbonyl-oxy group (e.g. 1-pyrrolidinylcarbonyloxy, piperidinocarbonyloxy, morpholinocarbonyloxy, thiomorpholinocarbonyloxy, 1-piperazinylcarbonyloxy etc.), a 4- to 6-membered cyclic amino-carbonyl-amino group (e.g. 1-pyrrolidinylcarbonylamino, piperidinocarbonylamino, morpholinocarbonylamino, thiomorpholinocarbonylamino, 1-piperazinylcarbonylamino etc.), a 4- to 6-membered cyclic amino-sulfonyl group (e.g. 1-pyrrolidinylsulfonyl, piperidinosulfonyl, morpholinosulfonyl, thiomorpholinosulfonyl, 1-piperazinylsulfonyl etc.), a 4-to 6-membered cyclic amino-C₁₋₆ alkyl group, (xxiii) a C₁₋₆ acyl group (e.g. an optionally halogenated C₂₋₆ alkanoyl group such as formyl, acetyl etc.) or a benzoyl group, each optionally substituted with a substituent selected from a halogen atom, a carboxyl group and a C₁₋₆ alkoxy-carbonyl group, (xxiv) a benzoyl group optionally substituted with a substituent selected from a halogen atom, (xxv) a 5- to 10-membered heterocyclic group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl etc.; the heterocyclic group may be substituted with a C₁₋₆ alkyl group), (xxvi) a 5- to 10-membered heterocyclic carbonyl group containing at least one (preferably 1 to 4, further preferably 1 to 2) of 1 to 3 kinds (preferably 1 or 2 kinds) of heteroatoms selected from an oxygen atom, a sulfur atom and a nitrogen atom (e.g. 2- or 3-thienylcarbonyl, 2- or 3-furylcarbonyl, 3-, 4- or 5-pyrazolylcarbonyl, 2-, 4- or 5-thiazolylcarbonyl, 3-, 4- or 5-isothiazolylcarbonyl, 2-, 4- or 5-oxazolylcarbonyl, 1,2,3- or 1,2,4-triazolylcarbonyl, 1H- or 2H-tetrazolylcarbonyl, 2-, 3- or 4-pyridylcarbonyl, 2-, 4- or 5-pyrimidylcarbonyl, 3- or 4-pyridazinylcarbonyl, quinolylcarbonyl, isoquinolylcarbonyl, indolylcarbonyl etc.; the heterocyclic group may be substituted with a C₁₋₆ alkyl group), (xxvii) a hydroxyimino group, a C₁₋₆ alkoxyimino group, an aryl group (e.g., 1- or 2-naphthyl, etc.) (xxviii) an optionally halogenated straight or branched C₁₋₆ alkylenedioxy group (e.g. methylenedioxy, ethylenedioxy, propylenedioxy, tetrafluoroethylenedioxy etc.), (xxiv) an ureido group, and (xxx) a C₁₋₆ alkyl-ureido group (e.g. methylureido, ethylureido etc.) (referred to as substituent B group). The "hydrocarbon group" may have 1 to 5 of these substituents at replaceable positions and, when it has 2 or more substituents, they may be the same or different.

Examples of the "heterocyclic group" of the "optionally substituted heterocyclic group" as the substituent which may be possessed by the ring A or the ring B include a 5- to 16-membered monocyclic to tricyclic aromatic heterocyclic group, and a saturated or unsaturated non-aromatic heterocyclic group (aliphatic heterocyclic group), containing at least one (preferably 1 to 4, further preferably 1 to 2) of 1 to 3 kinds (preferably 1 or 2 kinds) of heteroatoms selected from an oxygen atom, a sulfur atom and a nitrogen atom, as an atom constituting a ring system (ring atom).

Examples of the "aromatic heterocyclic group" include a 5- or 6-membered aromatic monocyclic heterocyclic group such as furyl, thienyl, pyrrolyl, oxazolyl, isooxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, and triazinyl, and a 8- to 16-membered (preferably 8- to 12-membered) aromatic fused heterocyclic group (preferably a heterocyclic ring in which 1 to 2 (preferably 1) of the aforementioned 5- or 6-membered aromatic monocyclic heterocyclic groups are fused with 1 to 2 (preferably 1) of benzene rings are fused, or a heterocyclic group in which 2 to 3 (preferably 2) of the same or different heterocyclic rings of the aforementioned 5- or 6-membered aromatic monocyclic heterocyclic groups are fused, more preferably a heterocyclic ring in which the aforementioned 5- or 6-membered aromatic monocyclic heterocyclic group is fused with a benzene ring) such as benzofuranyl, isobenzofuranyl, benzo[b]thienyl, indolyl, isoindolyl, 1H-indazolyl, benzimidazolyl, benzooxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyridyl, purinyl, pteridinyl, carbazolyl, α-carbolinyl, β-carbolinyl, γ-carbolinyl, acridinyl, phenoxazinyl, phenothiazinyl, phenazinyl, phenoxathiyl, thianthrenyl, phenatrizinyl, phenathrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, benzo[1,2,5]thiadiazolyl, and benzo[1,2,5]oxadiazolyl.

Examples of the "non-aromatic heterocyclic group" include a 3- to 8-membered (preferably 5- to 6-membered) saturated or unsaturated (preferably saturated) non-aromatic monocyclic heterocyclic group (aliphatic monocyclic heterocyclic group) such as oxiranyl, azetidinyl, oxetanyl, thietanyl, pyrrolidinyl (preferably, 1-pyrrolidinyl), tetrahydrofuryl, thiolanyl, piperidinyl (preferably, 1-piperidinyl or 4-piperidinyl), tetrahydropyranyl, morpholinyl, thiomorpholinyl, piperazinyl, 1-azabicyclo[2.2.2]octo-3-yl, a heterocyclic group in which 1 to 2 (preferably 1) of the aforementioned non-aromatic monocyclic heterocyclic groups are fused with 1 to 2 (preferably 1) of benzene rings such as 2,3-dihydroindolyl, and 1,3-dihydroisoindolyl, a heterocyclic group in which 1 to 2 (preferably 1) of the aforementioned non-aromatic monocyclic heterocyclic groups are fused with 1 to 2 (preferably 1) of the aforementioned 5- to 6-memebred aromatic monocyclic heterocyclic groups, and a non-aromatic heterocyclic group in which a part or all of double bonds of the aforementioned aromatic monocyclic heterocyclic group or aromatic fused heterocyclic group are saturated such as 1,2,3,4-tetrahydroquinolyl, and 1,2,3,4-tetrahydroisoquinolyl.

As the "heterocyclic group" in the "optionally substituted heterocyclic group", a 5- or 6-membered aromatic monocyclic heterocyclic group is preferable.

As the substituent which may be possessed by the "heterocyclic group", the same number of the same groups as those for the substituent (substituent B group) which may be possessed by the "hydrocarbon group" in the "optionally substituted hydrocarbon group" as the substituent which may be possessed by the ring A are used.

Examples of the "optionally substituted amino group", the "optionally substituted hydroxy group" and the "optionally substituted thiol group" as the substituent which may be possessed by the ring A or the ring B include an "amino group", a "hydroxy group" and a "thiol group", each optionally having a substituent such as an optionally substituted hydrocarbon group, an acyl group, an optionally esterified carboxyl group, an optionally substituted carbamoyl group, and an optionally substituted heterocyclic group.

As the "hydrocarbon group" in the "optionally substituted hydrocarbon group" and the "heterocyclic group" in the "optionally substituted heterocyclic group", the same groups as the "hydrocarbon group" in the "optionally substituted hydrocarbon group" and the "heterocyclic group" in the "optionally substituted heterocyclic group" as the substituent which may be possessed by the ring A are used, respectively. Further, as the substituents possessed by the "optionally substituted hydrocarbon group" and the "optionally substituted heterocyclic group", the same number of the same groups as the substituents of the "optionally substituted hydrocarbon group" and the "optionally substituted heterocyclic group" as the substituent which may be possessed by the ring A are used, respectively.

As the "acyl group" and the "optionally esterified carboxyl group", the same groups as the "optionally esterified carboxyl group" and the "acyl group" as the substituent which may be possessed by the ring A as described hereinafter are used, respectively.

As the "optionally substituted carbamoyl group", the same group as the "optionally substituted carbamoyl group" as the substituent which may be possessed by the ring A as described hereinafter is used.

Specifically, preferable examples of the "optionally substituted amino group", the "optionally substituted hydroxy group" and the "optionally substituted thiol group" as the substituent which may be possessed by the ring A or the ring B include an "amino group", a "hydroxy group" and a "thiol group" optionally having a substituent such as (i) a lower alkyl (e.g. C₁₋₆ alkyl such as methyl, ethyl, propyl, isopropyl, butyl, isobutyl, tert-butyl, pentyl, hexyl etc.) optionally substituted with a substituent selected from a halogen atom (e.g. fluorine, chlorine, bromine, iodine etc.), optionally halogenated C₁₋₆ alkoxy (e.g. methoxy, ethoxy, trifluoromethoxy, 2,2,2-trifluoroethoxy, trichloromethoxy, 2,2,2-trichloroethoxy etc.), optionally substituted phenyl (preferably, phenyl optionally substituted with a substituent selected from an optionally halogenated C₁₋₆ alkyl group, an optionally halogenated C₁₋₆ alkoxy group, a carboxyl group and a halogen atom), and a 5- to 10-membered heterocyclic group (e.g. 2- or 3-thienyl, 2- or 3-furyl, 3-, 4- or 5-pyrazolyl, 2-, 4- or 5-thiazolyl, 3-, 4- or 5-isothiazolyl, 2-, 4- or 5-oxazolyl, 1,2,3- or 1,2,4-triazolyl, 1H- or 2H-tetrazolyl, 2-, 3- or 4-pyridyl, 2-, 4- or 5-pyrimidyl, 3- or 4-pyridazinyl, quinolyl, isoquinolyl, indolyl etc.; the heterocyclic ring may be substituted with a C₁₋₄ alkyl group etc.), (ii) acyl (e.g. C₁₋₆ alkanoyl (e.g. formyl, acetyl, propionyl, pivaloyl etc.), benzoyl, C₁₋₆ alkylsulfonyl (e.g. methanesulfonyl etc.), benzenesulfonyl etc.), optionally halogenated C₁₋₆ alkoxycarbonyl (e.g. methoxycarbonyl, ethoxycarbonyl, trifluoromethoxycarbonyl, 2,2,2-trifluoroethoxycarbonyl, trichloromethoxycarbonyl, 2,2,2-trichloroethoxycarbonyl etc.), C₁₋₆ alkoxycarbonyl optionally substituted with phenyl (e.g. benzyloxycarbonyl etc.), an optionally substituted carbamoyl group (e.g. a carbamoyl group optionally substituted with 1 to 2 substituents such as a lower (C₁₋₆)alkyl group, a C₆₋₁₄ aryl group (e.g. a phenyl group) etc., such as carbamoyl, N-methylcarbamoyl, N,N-dimethylcarbamoyl, phenylcarbamoyl etc.), (iii) a heterocyclic group (the same group as the "heterocyclic group" in the "optionally substituted heterocyclic group" as the substituent which may be possessed by the ring A), (iv) an aryl group optionally substituted with a substituent selected from a halogen atom (e.g. fluorine, chlorine, bromine, iodine etc.), optionally halogenated C₁₋₆ alkoxy (e.g. methoxy, ethoxy, trifluoromethoxy, 2,2,2-trifluoroethoxy, trichloromethoxy, 2,2,2-trichloroethoxy etc.), optionally halogenated C₁₋₆ alkyl (e.g. methyl, ethyl, trifluoromethyl, 2,2,2-trifluoroethyl, trichloroehtyl, 2,2,2-trichloroethyl) (the same group as the "aryl group" in the "optionally substituted hydrocarbon group" as the substituent possessed by the ring A), and the like. In addition, two substituents in N,N-di-substituted amino may be taken together with a nitrogen atom to form a "cyclic amino group" and, as the "cyclic amino group", a 3- to 8-membered (preferably 5- to 8-membered more preferably 5- or 6-membered) cyclic amino group such as 1-azetidinyl, 1-pyrrolidinyl, piperidino, morpholino, thiomorpholino (a sulfur atom may be oxidized), 1-piperazinyl, and 1-piperazinyl which may have a lower alkyl (e.g. C₁₋₆ alkyl such as methyl, ethyl, propyl, isopropyl, butyl, tert-butyl, pentyl, hexyl etc.), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.), acyl (e.g. formyl, acetyl, benzoyl, methylsulfonyl, benzenesulfonyl, ethoxycarbonyl, benzyloxycarbonyl) at a 4-position are used.

The "substituted sulfinyl group" and the "substituted sulfonyl group" as the substituent which may be possessed by the ring A or the ring B represent a sulfinyl group or a sulfonyl group substituted with a substituent such as the "optionally substituted hydroxy group", the "optionally substituted amino group", the "optionally substituted hydrocarbon group" or the "optionally substituted heterocyclic group", respectively. As the "hydrocarbon group" in the "optionally substituted hydrocarbon group", the same group as the "hydrocarbon group" in the "optionally substituted hydrocarbon group" as the substituent which may be possessed by the ring A is used. As the "heterocyclic group" in the "optionally substituted heterocyclic group", the same group as the "heterocyclic group" in the "optionally substituted heterocyclic group" as the substituent which may be possessed by the ring A is used. As the substituent optionally substituting at the hydroxy group and the amino group which are the substituent of the "substituted sulfinyl group" and the "substituted sulfonyl group", the same group as the substituent which may be possessed by the "hydroxy group" in the "optionally substituted hydroxy group" or by the "amino group" in the "optionally substituted amino group" as the substituent which may be possessed by the ring A is used, and preferable examples include a C₁₋₆ alkyl group, a C₃₋₈ cycloalkyl group, a C₂₋₄ alkenyl group, a C₆₋₁₀ aryl group, an acyl group, an amino group and a heterocyclic group (the same group as the "heterocyclic group" in the "optionally substituted heterocyclic group" as the substituent which may be possessed by the ring A). In addition, as the substituent in the "optionally substituted hydrocarbon group" and the "optionally substituted heterocyclic group" which are the substituent of the "substituted sulfinyl group" and the "substituted sulfonyl group", the same number of the same groups as those for the substituent (substituent B group) in the "optionally substituted hydrocarbon group" and the "optionally substituted heterocyclic group" as the substituent which may be possessed by the ring A are used.

As the "acyl group" as the substituent which may be possessed by the ring A or the ring B, an acyl group obtained by removing a OH group from carboxylic acid such as R^{A}COOH, sulfonic acid such as R^{A}SO₃H, sulfinic acid such as R^{A}SO₂H, or phosphoric acid such as R^{A}OPO(OR^{B})OH (R^{A} represents a hydrogen atom, an optionally substituted hydrocarbon group, or an optionally substituted heterocyclic group, and R^{B} represents a hydrogen atom, or an optionally substituted hydrocarbon group) is used and, specifically, R^{A}CO, R^{A}OCO, R^{A}SO₂, R^{A}SO, and R^{A}OPO(OR^{B}) (the symbols in the formulas are as defined above) are used.

As the "hydrocarbon group" in the "optionally substituted hydrocarbon group" and the "heterocyclic group" in the "optionally substituted heterocyclic group" represented by R^{A} (and R^{B}), the same groups as the "hydrocarbon group" in the "optionally substituted hydrocarbon group" and the "heterocyclic group" in the "optionally substituted heterocyclic group" as the substituent which may be possessed by the ring A are used, respectively. In addition, as the substituent in the "optionally substituted hydrocarbon group" and the "optionally substituted heterocyclic group", the same number of the same groups as those for the substituent (substituent B group) in the "optionally substituted hydrocarbon group" and the "optionally substituted heterocyclic group" as the substituent which may be possessed by the ring A are used, respectively.

Examples of R^{A}CO include formyl, acetyl, propionyl, butyryl, isobutyryl, valeryl, isovaleryl, pivaloyl, hexanoyl, cyclobutanecarbonyl, cyclopentanecarbonyl, cyclohexanecarbonyl, crotonyl, benzoyl, nicotinoyl, isonicotinoyl, and trifluoroacetyl and, inter alia, R^{A}CO in which R^{A} is a lower (C₁₋₆)alkyl group such as acetyl, propionyl, butyryl, valeryl is more preferable.

Examples of R^{A}OCO include methoxycarbonyl, ethoxycarbonyl, propoxycarbonyl, and tert-butoxycarbonyl and, inter alia, R^{A}OCO in which R^{A} is a lower (C₁₋₆)alkyl group such as tert-butoxycarbonyl is more preferable.

Examples of R^{A} of R^{A}SO₂ preferably include a halogenated C₁₋₆ alkyl group (e.g. methyl, trifluoromethyl), or a phenyl group optionally substituted with a substituent selected from a halogen atom, C₁₋₆ alkyl and C₁₋₆ alkoxy (e.g. phenyl, p-tolyl).

Examples of the "optionally substituted carbamoyl group" as the substituent which may be possessed by the ring A or the ring B include N-mono-substituted carbamoyl and N,N-di-substituted carbamoyl in addition to unsubstituted carbamoyl.

Examples of the substituent which may be possessed by the "carbamoyl group" in the "optionally substituted carbamoyl group" include the same group as the substituent of the "amino group" of the "optionally substituted amino group" as the substituent which may be possessed by the ring A ("optionally substituted hydrocarbon group", "acyl group", "optionally substituted alkoxycarbonyl group", "optionally substituted carbamoyl group" (preferably a carbamoyl group optionally substituted with 1 to 2 substituents such as a lower (C₁₋₆)alkyl group, and a C₆₋₁₄ aryl group (e.g. a phenyl group), such as carbamoyl, N-methylcarbamoyl, N,N-dimethylcarbamoyl, phenylcarbamoyl etc.), "optionally substituted heterocyclic group" etc.), and a "carbamoyl group" having the "optionally substituted amino group" (i.e. "optionally substituted carbazoyl group"), and a "carbamoyl group" having the "optionally substituted hydroxy group" (i.e. "optionally substituted N-hydroxycarbamoyl group") may be used. Alternatively, two substituents in N,N-di-substituted carbamoyl may be taken together with the nitrogen atom to form cyclic amino and, as cyclic amino carbonyl in such the case, 3- to 8-membered (preferably 5- to 6-membered) cyclic aminocarbonyl such as 1-azetidinylcarbonyl, 1-pyrrolidinylcarbonyl, piperidinocarbonyl, morpholinocarbonyl, thiomorpholinocarbonyl (a sulfur atom may be oxidized), 1-piperazinylcarbonyl, and 1-piperazinylcarbonyl optionally having lower alkyl (e.g. C₁₋₆ alkyl such as methyl, ethyl, propyl, isopropyl, butyl, tert-butyl, pentyl, hexyl etc.), aralkyl (e.g. C₇₋₁₀ aralkyl such as benzyl, phenethyl etc.), aryl (e.g. C₆₋₁₀ aryl such as phenyl, 1-naphthyl, 2-naphthyl etc.), or an acyl group (e.g. formyl, acetyl, benzoyl, methoxycarbonyl, benzyloxycarbonyl, methylsulfonyl etc.) at a 4-position is used.

Examples of the "optionally esterified carboxyl group" as the substituent which may be possessed by the ring A or the ring B include a group represented by the formula - COOR^{C} (R^{C} represents a hydrogen atom, or an optionally substituted hydrocarbon group, or an optionally substituted heterocyclic group) and, inter alia, free carboxyl, lower alkoxycarbonyl, aryloxycarbonyl, aralkyloxycarbonyl, heterocyclic oxycarbonyl, and heterocyclic methyloxycarbonyl are preferably used.

As "the hydrocarbon group" in the "optionally substituted hydrocarbon group" and the "heterocyclic group" in the "optionally substituted heterocyclic group" represented by R^{c}, the same groups as those for the "hydrocarbon group" in the "optionally substituted hydrocarbon group" and the "heterocyclic group" in the "optionally substituted heterocyclic group" as the substituent which may be possessed by the ring A are used, respectively. In addition, as the substituent which may be possessed by the "hydrocarbon group" or the "heterocyclic group", the same number of the same groups as those for a substituent (substituent B group) which may be possessed by the "hydrocarbon group" in the "optionally substituted hydrocarbon group" and the "heterocyclic group" in the "optionally substituted heterocyclic group" as the substituent which may be possessed by the ring A are used, respectively.

Examples of the "lower alkoxycarbonyl" include C₁₋₆ alkoxycarbonyl such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, and neopentyloxycarbonyl and, inter alia, C₁₋₃ alkoxycarbonyl such as methoxycarbonyl, ethoxycarbonyl, and propoxycarbonyl is preferable.

The "lower alkoxycarbonyl" may have a substituent at the "lower alkyl" part of the "lower alkoxy" and, as the substituent, the same number of the same groups as those listed as the substituent which may be possessed by the "hydrocarbon group" in the "optionally substituted hydrocarbon group" as the substituent (substituent B group) which may be possessed by the ring A are used.

As the "aryloxycarbonyl", C₇₋₁₂ aryloxycarbonyl such as phenoxycarbonyl, 1-naphthoxycarbonyl, and 2-naphthoxycarbonyl is preferable.

As the "aralkyloxycarbonyl", C₇₋₁₅ aralkyloxycarbonyl such as benzyloxycarbonyl, and phenethyloxycarbonyl (preferably, C₆₋₁₀ aryl-C₁₋₆ alkoxy-carbonyl etc.) is preferable.

As the heterocyclic ring in the "heterocyclic oxycarbonyl" and the "heterocyclic methyloxycarbonyl", the same groups as those for the "heterocyclic" in the "optionally substituted heterocyclic group" as the substituent which may be possessed by the ring A are used and, for example, pyridyl, quinolyl, indolyl, piperidinyl, and tetrahydropyranyl are preferably used.

The "aryloxycarbonyl", the "aralkyloxycarbonyl" and the "heterocyclic oxycarbonyl" may have a substituent, respectively. As the substituent thereof, the same number of the same groups as groups listed as the substituent (substituent B group) which may be possessed by the "hydrocarbon group" in the "optionally substituted hydrocarbon group" as a substituent which may be possessed by the ring A are used.

As the "C₁₋₃ alkylenedioxy group" as a substituent which may be possessed by the ring A or the ring B, methylenedioxy, and ethylenedioxy are used.

As the substituent which may be possessed by the ring A or the ring B, a halogen atom (e.g. fluorine, chlorine, bromine, iodine), a C₁₋₆ alkyl group (e.g. methyl, ethyl, propyl, isopropyl, butyl etc.) optionally substituted with a halogen atom (e.g. fluorine etc.), a C₁₋₆ alkoxy group, an optionally substituted C₆₋₁₄ aryl group (preferably, phenyl optionally substituted with a substituent selected from an optionally halogenated C₁₋₆ alkyl group, a carboxyl group and a halogen atom, etc.), an optionally substituted C₇₋₁₆ aralkyloxy group (preferably, benzyloxy optionally substituted with a substituent selected from a C₁₋₆ alkyl group, a carboxyl group and a halogen atom, etc.), a C₁₋₃ alkylenedioxy group (e.g. methylenedioxy, ethylenedioxy, etc.) are preferably used. Inter alia, preferable examples include a halogen atom (e.g. fluorine, chlorine, bromine, iodine), a C₁₋₆ alkyl group (e.g. methyl, ethyl, propyl, isopropyl, butyl etc.) optionally substituted with a halogen atom (e.g. fluorine etc.), a C₁₋₆ alkoxy group, an optionally substituted C₇₋₁₆ aralkyloxy group (preferably, benzyloxy optionally substituted with a substituent selected from a C₁₋₆ alkyl group, a carboxyl group and a halogen atom, etc.), and a C₁₋₃ alkylenedioxy group (e.g. methylenedioxy, ethylenedioxy, etc.).

The ring A or the ring B may have 1 to 3 substituents at positions where can be substituted, respectively. When the ring has 2 or more substituents, they may be the same or different. Inter alia, it is preferable that the 1-isoquinoline skeleton is substituted at 6- and/or 7-position.

In the aforementioned formulas, as the ring A or the ring B, a benzene ring represented by the formula: 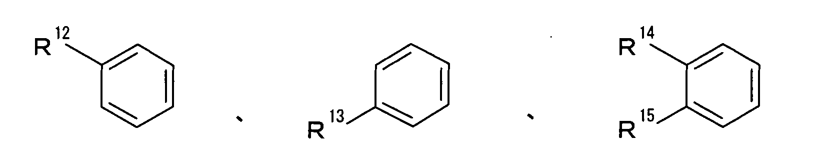 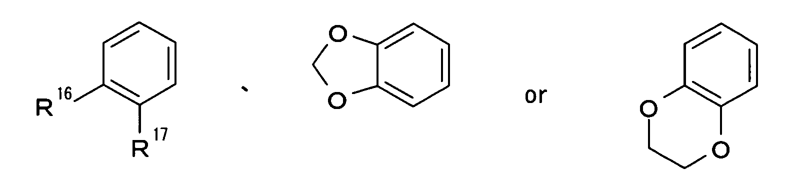 wherein R¹² represents a hydrogen atom, a halogen atom, a C₁₋₆ alkyl group optionally substituted with a halogen atom, a C₆₋₁₄ aryl group, or a C₇₋₁₆ aralkyloxy group; R¹³ represents a halogen atom, a C₁₋₆ alkyl group optionally substituted with a halogen atom, or a C₇₋₁₆ aralkyloxy group; R¹⁴ and R¹⁵ represent a halogen atom, or a C₁₋₆ alkoxy group, respectively; and R¹⁶ and R¹⁷ represent a C₁₋₆ alkoxy group, respectively, is preferably used. Inter alia, a benzene ring represented by the formula: 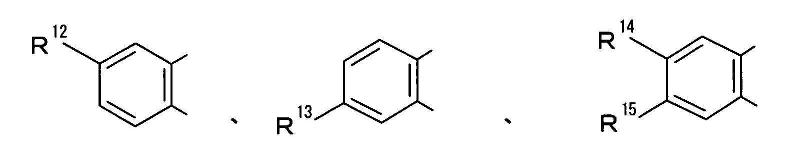 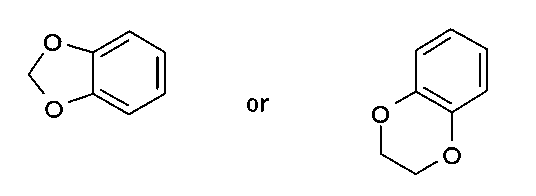 wherein R¹² represents a hydrogen atom, a halogen atom, a C₁₋₆ alkyl group optionally substituted with a halogen atom, a C₆₋₁₄ aryl group, or a C₇₋₁₆ aralkyloxy group; R¹³ represents a halogen atom, a C₁₋₆ alkyl group optionally substituted with a halogen atom, or a C₇₋₁₆ aralkyloxy group; R¹⁴ and R¹⁵ represent a halogen atom, or a C₁₋₆ alkoxy group, respectively, is preferably used. As R¹², a hydrogen atom, a halogen atom, a C₁₋₆ alkyl group optionally substituted with a halogen atom or a C₇₋₁₆ aralkyloxy group is preferable.

In the aforementioned formulas, X represents a bond, NR⁴ (R⁴ represents a hydrogen atom, an optionally substituted hydrocarbon group, or an optionally substituted heterocyclic group), or an optionally substituted alkylene group.

As the "optionally substituted hydrocarbon group" and the "optionally substituted heterocyclic group" represented by R⁴, the same groups as the "optionally substituted hydrocarbon group" and the "optionally substituted heterocyclic group" as the substituent which may be possessed by the ring A are used.

As the "alkylene group" of the "optionally substituted alkylene group" represented by X, for example, a C₁₋₆ alkylene group such as methylene, ethylene, propylene, etc. is used and, inter alia, methylene is preferable.

As the substituent of the "alkylene group", the same group as the substituent of the "optionally substituted hydrocarbon" (substituent B group) as the substituent which may be possessed by the ring A is used.

As X, an optionally substituted alkylene group is preferable, inter alia, a C₁₋₆ alkylene group such as methylene, ethylene, propylene, etc. is preferable, and methylene is particularly preferable.

In the aforementioned formulas, R¹ represents an optionally substituted hydrocarbon group or an optionally substituted heterocyclic group.

As the "optionally substituted hydrocarbon group" represented by R¹, the same group as the "optionally substituted hydrocarbon group" as the substituent which may be possessed by the ring A is used.

As the "optionally substituted heterocyclic group" represented by R¹, the same group as the "optionally substituted heterocyclic group" as the substituent which may be possessed by the ring A is used.

The "hydrocarbon group" and the "heterocyclic group" may have 1 to 5 (preferably 1 to 3) substituents at positions where can be substituted, respectively. When 2 or more substituents are possessed, they may be the same or different.

As R¹, an optionally substituted C₁₋₆ alkyl group, an optionally substituted C₃₋₁₀ cycloalkyl group, an optionally substituted C₆₋₁₄ aryl group, etc. are preferable.

As the "optionally substituted C₁₋₆ alkyl group", for example, a C₁₋₆ alkyl group (e.g. methyl, ethyl, propyl, isopropyl, butyl, isobutyl, sec-butyl, tert-butyl, etc.) optionally substituted with a halogen atom (e.g. fluorine etc.), C₁₋₆ alkoxy-carbonyl (e.g. methoxycarbonyl, ethoxycarbonyl, isopropoxycarbonyl, etc.), etc. is used and, inter alia, a branched C₃₋₆ alkyl group such as isobutyl, sec-butyl, and tert-butyl is preferable.

As the "optionally substituted C₃₋₁₀ cycloalkyl", for example, a C₃₋₁₀ cycloalkyl group such as cyclopropyl, cyclobutyl, cyclopentyl, cyclohexyl, adamantyl, etc. optionally substituted with an optionally halogenated C₁₋₆ alkyl group is preferable.

As the "optionally substituted C₆₋₁₄ aryl group", for example, a C₆₋₁₄ aryl (e.g. phenyl) optionally substituted with C₁₋₃ alkylenedioxy (e.g. methylenedioxy) is used.

In the aforementioned formulas, R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group.

As the "optionally substituted hydrocarbon group" represented by R², R⁵ or R⁶, the same group as the "optionally substituted hydrocarbon group" which is the substituent of the ring A is used.

As the "optionally substituted heterocyclic group" represented by R², R⁵ or R⁶, the same group as the "optionally substituted heterocyclic group" which is the substituent of the ring A is used.

As the "alkylene group" of the "optionally substituted alkylene group" represented by Y, a C₁₋₆ alkylene group such as methylene, ethylene, and propylene is used and, inter alia, methylene is preferable.

As the substituent which may be possessed by the "alkylene", the same group as the "substituent" (substituent B group) which may be possessed by the "optionally substituted hydrocarbon group" as the substituent which may be possessed by the ring A is used.

As R², the group represented by the formula -COYR⁵ is preferable.

As Y, O or NR⁶ is preferably, in particular, O is preferable.

As R⁶, for example, a hydrogen atom or a C₁₋₆ alkyl group such as methyl and ethyl is preferable.

As R⁵, an optionally substituted C₁₋₆ alkyl group, an optionally substituted C₃₋₁₀ cycloalkyl group (preferably a C₃₋₈ cycloalkyl group), an optionally substituted C₇₋₁₆ aralkyl group, an optionally substituted heterocyclic group, etc. are preferable. As the substituent which may be possessed by the "C₁₋₆ alkyl group", the "C₃₋₁₀ cycloalkyl group", the "C₇₋₁₆ aralkyl group" or the heterocyclic group, the same group as the "substituent" (substituent B group) which may be possessed by the "hydrocarbon group" of the "optionally substituted hydrocarbon group" as the substituent which may be possessed by the ring A is used.

As the "optionally substituted C₁₋₆ alkyl group", for example, a C₁₋₆ alkyl group (e.g. methyl, ethyl, propyl, isopropyl, butyl, isobutyl, sec-butyl, tert-butyl, etc.) optionally substituted with a halogen atom (e.g. fluorine etc.), an amino group, a mono- or di-C₁₋₆ alkylamino group (e.g. dimethylamino), a C₁₋₆ alkyl(C₇₋₁₆ aralkyl) amino group (e.g. methyl(benzyl)amino), a 4- to 6-membered cyclic amino group (e.g. 1-azetidinyl, 1-pyrrolidinyl, piperidino, morpholino, thiomorpholino, 1-piperazinyl etc., in particular, 1-pyrrolidinyl), etc. is preferable, inter alia, an unsubstituted C₁₋₆ alkyl group is preferable, and a branched C₃₋₆ alkyl such as isobutyl, sec-butyl, tert-butyl, etc. is preferable.

As the "optionally substituted C₃₋₁₀ cycloalkyl group", for example, cyclopropyl, cyclobutyl, cyclopentyl, cyclohexyl, adamantyl, etc., each of which may be substituted with an optionally halogenated C₁₋₆ alkyl is preferable, inter alia, a C₃₋₈ cycloalkyl group is preferable, and a C₅₋₇ cycloalkyl group is particularly preferable.

As the "optionally substituted C₇₋₁₆ aralkyl group", for example, a C₇₋₁₆ aralkyl (e.g. benzyl) optionally substituted with C₁₋₆ alkyl (e.g. methyl, ethyl, propyl, etc.) optionally substituted with a halogen atom is preferable.

As the "optionally substituted heterocyclic group", for example, 1-azabicyclo[2.2.2]oct-3-yl is preferable.

In particular, as R⁵, a branched C₃₋₆ alkyl group such as isobutyl, sec-butyl, tert-butyl, or a C₃₋₁₀ cycloalkyl group such as adamantyl is preferable, inter alia, a branched C₃₋₆ alkyl group such as isobutyl, sec-butyl, and tert-butyl is preferable.

In the aforementioned formulas, R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2).

As the "optionally substituted hydrocarbon group", the "optionally substituted heterocyclic group", the "optionally substituted hydroxy group" and "the optionally substituted amino group" represented by R³, the same groups as the "optionally substituted hydrocarbon group", the "optionally substituted heterocyclic group", the "optionally substituted hydroxy group" and "the optionally substituted amino group" which are the substituents of the ring A are used.

As the "optionally substituted hydrocarbon group" and the "optionally substituted heterocyclic group" represented by R⁷, the same groups as the "optionally substituted hydrocarbon group" and the "optionally substituted heterocyclic group" which are the substituents of the ring A are used. n represents the integer of 0 to 2, inter alia 0 is preferable.

As R³, an optionally substituted hydroxy group, an optionally substituted amino group or an optionally substituted hydrocarbon group is preferable, inter alia, an optionally substituted hydroxy group is preferable.

As the "optionally substituted hydroxy group", a hydroxy group optionally substituted with an optionally substituted hydrocarbon group is preferable. Specifically, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbonyl, ethoxycarbonyl, propoxycarbonyl, isopropoxycarbonyl, etc.), mono- or di-C₁₋₆ alkylamino (e.g. dimethylamino), a 4- or 6-membered cyclic amino group (e.g. 1-azetidinyl, 1-pyrrolidinyl, piperidino, morpholino, thiomorpholino, 1-piperazinyl etc., in particular, 1-pyrrolidinyl), a 5- to 16-membered aromatic heterocyclic group containing at least one of 1 to 3 kinds of heteroatoms selected from an oxygen atom, a sulfur atom and a nitrogen atom (e.g. indolyl), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₆₋₁₄ aryl group (e.g. phenyl), (iv) a C₇₋₁₆ aralkyl group (e.g. benzyl), (v) R^{AA}SO₂- (R^{AA} represents an optionally halogenated C₁₋₆ alkyl group (e.g. methyl, trifluoromethyl, etc.), and the like is preferably used. Inter alia, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbony, ethoxycarbonyl, propoxycarbonyl, isopropoxycarbonyl, etc.), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₇₋₁₆ aralkyl group (e.g. benzyl), (iv) R^{AA}SO₂- (R^{AA} represents an optionally halogenated C₁₋₆ alkyl group (e.g. methyl, trifluoromethyl, etc.), and the like is preferably used. As the "optionally substituted amino group", an amino group is preferable. As the "optionally substituted hydrocarbon group", a C₆₋₁₄ aryl group such as phenyl is preferable.

As R³, a hydroxy group is particularly preferable.

As the compound represented by the above formula (I), for example,
(1) the compound represented by the formula: 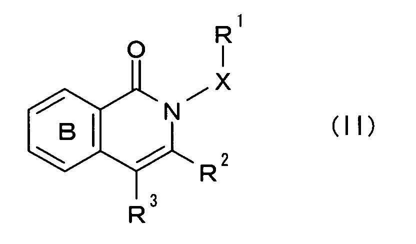 wherein the symbols are as defined above,
(2) the compound represented by the formula: 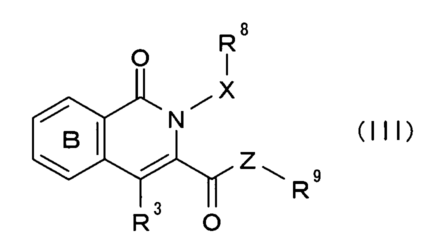 wherein Z represents a bond, an optionally substituted alkylene group, O, S or NR¹⁰ (R¹⁰ represents a hydrogen atom, an optionally substituted hydrocarbon group, or an optionally substituted heterocyclic group); R⁸ and R⁹ each represents an optionally substituted branched hydrocarbon group; and the other symbols are as defined in the above,
(3) the compound represented by the formula: 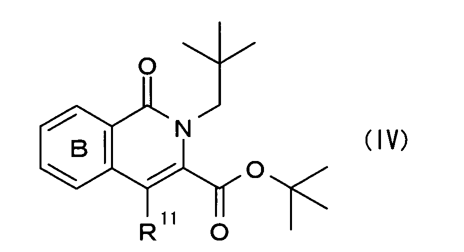
wherein R¹¹ represents an optionally substituted hydroxy group, and the ring B is as defined above, etc. are preferably used.

Z represents a bond, an optionally substituted alkylene group, O, S or NR¹⁰ (R¹⁰ represents a hydrogen atom, an optionally substituted hydrocarbon group, or an optionally substituted heterocyclic group).

As the "alkylene group" of the "optionally substituted alkylene group" represented by Z, for example, a C₁₋₆ alkylene group such as methylene, ethylene, propylene, etc. is used and, inter alia, methylene is preferable.

As the substituent of the "alkylene group", the same group as the substituent of the "optionally substituted hydrocarbon" (substituent B group) as the substituent which may be possessed by the ring A is used.

As the "optionally substituted hydrocarbon group" and the "optionally substituted heterocyclic group" represented by R¹⁰, the same groups as the "optionally substituted hydrocarbon group" and the "optionally substituted heterocyclic group" which are the substituents of the ring A are used.

As Z, O or NR¹⁰ is preferable. As R¹⁰, for example, a hydrogen atom or a C₁₋₆ alkyl group such as methyl or ethyl is preferable, and, a hydrogen atom is particularly preferable.

Among them, as Z, O or NH is preferable, and O is particularly preferable.

As the "branched hydrocarbon group" of the "optionally substituted branched hydrocarbon group" represented by R⁸ or R⁹, a branched C₃₋₆ alkyl group such as, for example, isopropyl, isobutyl, sec-butyl, tert-butyl, neopentyl, etc., an adamantyl group are used.

Besides, R⁸ or R⁹ is not necessarily limited to the "optionally substituted branched hydrocarbon group" in so far as it is a bulky group.

As the substituent of the "branched hydrocarbon group", the same group as the substituent of the "optionally substituted hydrocarbon" (substituent B group) as the substituent which may be possessed by the ring A is used. Inter alia, a halogen atom (e.g. fluorine etc.) is preferable.

As R⁸ and R⁹, an unsubstituted branched C₃₋₆ alkyl group such as isopropyl, isobutyl, sec-butyl, tert-butyl, etc. is preferable.

As the "optionally substituted hydroxy group" represented by R¹¹, the same group as the "optionally substituted hydroxy group" represented by R³ is used, inter alia, a hydroxy group optionally substituted with an optionally substituted hydrocarbon group is preferable.

Specifically, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbony, ethoxycarbonyl, propoxycarbonyl, isopropoxycarbonyl, etc.), mono- or di-C₁₋₆ alkylamino (e.g. dimethylamino), a 4- or 6-membered cyclic amino group (e.g. 1-azetidinyl, 1-pyrrolidinyl, piperidino, morpholino, thiomorpholino, 1-piperazinyl etc., in particular, 1-pyrrolidinyl), a 5- to 16-membered aromatic heterocyclic group containing at least one of 1 to 3 kinds of heteroatoms selected from an oxygen atom, a sulfur atom and a nitrogen atom (e.g. indolyl), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₆₋₁₄ aryl group (e.g. phenyl), (iv) a C₇₋₁₆ aralkyl group (e.g. benzyl), (v) R^{AA}SO₂- (R^{AA} represents an optionally halogenated C₁₋₆ alkyl group (e.g. methyl, trifluoromethyl, etc.), and the like is preferably used. Inter alia, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbony, ethoxycarbonyl, propoxycarbonyl, isopropoxycarbonyl, etc.), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₇₋₁₆ aralkyl group (e.g. benzyl), (iv) R^{AA}SO₂- (R^{AA} represents an optionally halogenated C₁₋₆ alkyl group (e.g. methyl, trifluoromethyl, etc.), and the like is preferably used.

As R¹¹, a hydroxy group is particularly preferable.

As the compound represented by the above formula (I), specifically, the compounds prepared by Reference Examples 1 to 22 or Examples 1 to 28 described hereinafter, etc. are preferable.

Among the compounds represented by the formula (I), the compound represented by the formula: 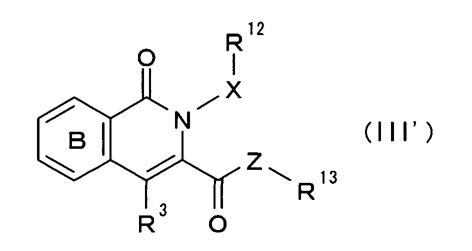 wherein R¹² and R¹³ each represents an optionally substituted C₃ or higher hydrocarbon group; and the other symbols are as defined above (provided that tert-butyl 6-fluoro-4-hydroxy-2-neopentyl-1-oxo-1,2-dihydro-3-isoquinolinecarboxylate, tert-butyl 4-butoxy-6-fluoro-2-neopentyl-1-oxo-1,2-dihydoro-3-isoquinolinecarboxylate, tert-butyl 7-benzyloxy-4-hydroxy-2-isobutyl-1-oxo-1,2-dihydro-3-isoquinolinecarboxylate and tert-butyl 6-benzyloxy-4-hydroxy-2-isobutyl-1-oxo-1,2-dihydro-3-isoquinolinecarboxylate are excluded) is a novel compound.

As the "optionally substituted C₃ or higher hydrocarbon group" represented by R¹² and R¹³, for example, a straight or branched C₃₋₁₅ alkyl group such as propyl, isopropyl, cyclopropyl, butyl, isobutyl, sec-butyl, tert-butyl, pentyl, neopentyl, hexyl, heptyl, octyl, nonyl, decyl, undecyl, tridecyl, tetradecyl, pentadecyl, etc., a C₃₋₁₀ cycloalkyl group such as adamantyl, etc. is used. Inter alia, a C₃₋₈ alkyl group is preferable, and a C₃₋₆ alkyl group is more preferable. As a combination thereof, the case where R¹² is a straight or branched C₃₋₁₅ alkyl group or a C₃₋₁₀ cycloalkyl group and R¹³ is a straight or branched C₃₋₁₅ alkyl group is preferable.

As the "C₃ or higher hydrocarbon group", a branched C₃ or higher hydrocarbon group is preferable. For example, a branched C₃₋₆ alkyl group such as isopropyl, isobutyl, sec-butyl, tert-butyl, neopentyl, etc., an adamantyl group , etc. is used, and tert-butyl is particularly preferable.

As the substituent of the "C₃ or higher hydrocarbon group", the same group as the substituent of the "optionally substituted hydrocarbon" (substituent B group) as the substituent which may be possessed by the ring A is used. Inter alia, a halogen atom (e.g. fluorine etc.) is preferable.

As R¹² and R¹³, an unsubstituted branched C₃₋₆ alkyl group such as isopropyl, isobutyl, sec-butyl, tert-butyl, etc. is preferable

As X, a methylene group is preferable.

As Z, an oxygen atom is preferable.

As R¹², isopropyl tert-butyl or adamantyl is preferable, and tert-butyl is particularly preferable.

As R¹³, tert-butyl is particularly preferable.

As R³, the aforementioned optionally substituted hydroxy group is preferable. Specifically, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbonyl, ethoxycarbonyl, propoxycarbonyl, isopropoxycarbonyl, etc.), mono- or di-C₁₋₆ alkylamino (e.g. dimethylamino), a 4- or 6-membered cyclic amino group (e.g. 1-azetidinyl, 1-pyrrolidinyl, piperidino, morpholino, thiomorpholino, 1-piperazinyl etc., in particular, 1-pyrrolidinyl), a 5- to 16-membered aromatic heterocyclic group containing at least one of 1 to 3 kinds of heteroatoms selected from an oxygen atom, a sulfur atom and a nitrogen atom (e.g. indolyl), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₆₋₁₄ aryl group (e.g. phenyl), (iv) a C₇₋₁₆ aralkyl group (e.g. benzyl), (v) R^{AA}SO₂- (R^{AA} represents an optionally halogenated C₁₋₆ alkyl group (e.g. methyl, trifluoromethyl, etc.), and the like is preferably used. Inter alia, a hydroxy group optionally substituted with a substituent selected from (i) a C₁₋₆ alkyl group (e.g. methyl, ethyl, propyl, isopropyl, butyl, isobutyl, sec-butyl, tert-butyl etc.) optionally substituted with a halogen atom (e.g. fluorine etc.), C₁₋₆ alkoxy-carbonyl (e.g., methoxycarbony, ethoxycarbonyl, propoxycarbonyl, isopropoxycarbonyl, etc.), etc. (ii) a C₃₋₈ cycloalkyl group (e.g. cyclopropyl, cyclobutyl, cyclopentyl, cyclohexyl) optionally substituted with a C₁₋₆ alkyl group (e.g. methyl, ethyl, propyl, isopropyl, butyl, isobutyl, sec-butyl, tert-butyl, etc.), (iii) a C₇₋₁₆ aralkyl group (e.g. benzyl), (iv) R^{AA}SO₂- (R^{AA} represents an optionally halogenated C₁₋₆ alkyl group (e.g. methyl, trifluoromethyl, etc.), and the like is preferable, and a hydroxy group is particularly preferable.

Among them, as the compound (III'), the compound represented by the formula: 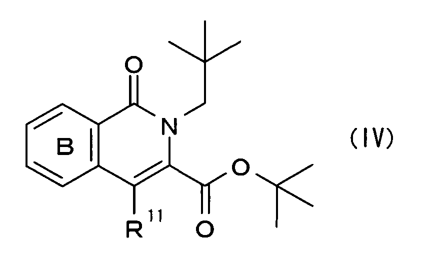 wherein the symbols are as defined above, is preferably used.

Further, among the compound represented by the above formula (I), (i) ethyl 7-bromo-4-hydroxy-2-neopentyl-1-oxo-1,2-dihydroisoquinoline-3-carboxylate or its salt, (ii) ethyl 8-hydroxy-6-neopentyl-5-oxo-5,6-dihydro[1,3]dioxolo[4,5-g]isoquinoline-7-carboxylate or its salt, (iii) N-{2-[benzyl(methyl)amino]ethyl}-6,7-dichloro-4-methoxy-2-neopentyl-1-oxo-1,2-dihydro-3-isoquinolinecarboxamide or its salt (particularly, hydrochloride), (iv) methyl 6,7-dichloro-4-hydroxy-2-neopentyl-1-oxo-1,2-dihydro-3-isoquinolinecarboxylate or its salt, or (v) methyl 6,7-dichloro-4-methoxy-2-neopentyl-1-oxo-1,2-dihydro-3-isoquinolinecarboxylate or its salt is also a novel compound.

A prodrug of the compound represented by the above formula (I) or a salt thereof [hereinafter, referred to as compound (I) in some cases] refers to a compound which is converted into a compound (I) by a reaction with an enzyme or gastric acid under the physiological condition in a living body, that is, a compound which causes oxidation, reduction or hydrolysis enzymatically, and is changed into a compound (I), and a compound which causes hydrolysis with gastric acid, and is changed into a compound (I). Examples of a prodrug of a compound (I) include a compound in which an amino group of a compound (I) is acylated, alkylated, or phosphorylated (e.g. a compound in which an amino group of a compound (I) is eicosanoylated, alanylated, pentylaminocarbonylated, (5-methyl-2-oxo-1, 3-dioxolan-4-yl) methoxycarbonylated, tetrahydrofuranylated, pyrrolidylmethylated, pivaloyloxymethylated, or tert-butylated), a compound in which a hydroxy group of a compound (I) is acylated, alkylated, phosphorylated, or converted into borate (e.g. a compound in which a hydroxy group of a compound (I) is acetylated, palmitoylated, propanoylated, pivaloylated, succinylated, fumarylated, alanylated, or dimethylaminomethylcarbonylated), and a compound in which a carboxyl group of a compound (I) is esterified, or amidated (e.g. a compound in which a carboxy group of a compound (I) is ethyl-esterified, phenyl-esterified, carboxymetyl-esterified, dimethylaminomethyl-esterified, pivaloyloxymethyl-esterified, ethoxycarbonyloxyethyl-esterified, phthalidyl-esterified, (5-methyl-2-oxo-1,3-dioxolan-4-yl)methyl-esterified, cyclohexyloxycarbonylethyl-esterified, or methylamidated). These compounds can be prepared from the compound (I) by the known per se method.

Alternatively, a prodrug of the compound (I) may be a compound which is changed into a compound (I) under the physiological condition, described in "Development of Medicament", vol. 7, Molecular Design, p163-198 published by Hirokawashoten in 1990.

Examples of a salt of the compound (I) include a metal salt, an ammonium salt a salt with an organic base, a salt with an inorganic acid, a salt with an organic acid, and a salt with basic or acidic amino acid. Preferable examples of the metal salt include an alkali metal salt such as a sodium salt, and a potassium salt; an alkaline earth metal salt such as a calcium salt, a magnesium salt, and a barium salt; an aluminum salt. Preferable example of the salt with an organic base include a base with trimethylamine, triethylamine, pyridine, picoline, 2,6-lutidine, ethanolamine, diethanolamine, triethanolamine, cyclohexylamine, dicyclohexylamine, or N,N'-dibenzylethylenediamine. Preferable examples of the salt with an inorganic acid include a salt with hydrochloric acid, hydrobromic acid, nitric acid, sulfuric acid, or phosphoric acid. Preferable examples of the salt with an organic acid include a salt with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid or p-toluenesulfonic acid. Preferable examples of the salt with basic amino acid include a salt with arginine, lysine, or ornithine, and preferable examples of the salt with acidic amino acid include a salt with aspartic acid, or glutamic acid.

Among them, a pharmaceutically acceptable salt is preferable. For example, when a compound has an acidic functional group, examples include an inorganic salt such as an alkali metal salt (e.g. sodium salt, potassium salt etc.) an alkaline earth metal salt (e.g. calcium salt, magnesium salt, barium salt etc.), and an ammonium salt and, when the compound has a basic functional group, examples include a salt with an inorganic acid such as hydrochloric acid, hydrobromic acid, nitric acid, sulfuric acid, and phosphoric acid, and a salt with an organic acid such as acetic acid, phthalic acid, fumaric acid, oxalic acid, tartaric acid, maleic acid, citric acid, succinic acid, methanesulfonic acid, and p-toluenesulfonic acid.

When an optically active form of the compound (I) is required, for example, it can be obtained by using an optically active starting material, or by resolution of a racemic isomer of the compound using a per se known method.

The compound (I) or a salt thereof can be prepared by the methods described, for example, in JP 10-298164 A, JP 2000-72675 A, JP 2000-72751 A, EP-481383, EP-566069, EP-585913, EP-634402, EP-652218, WO02/62764, or methods similar thereto.

Specifically, the compound (I) or a salt thereof can be prepared, for example, by the following Methods A to F. Each compound described in the following reaction schemes may form its salt in so far as the salt does not interfere with the reaction. Examples of such salt include the same salts as those of the compound (I).

### Method A

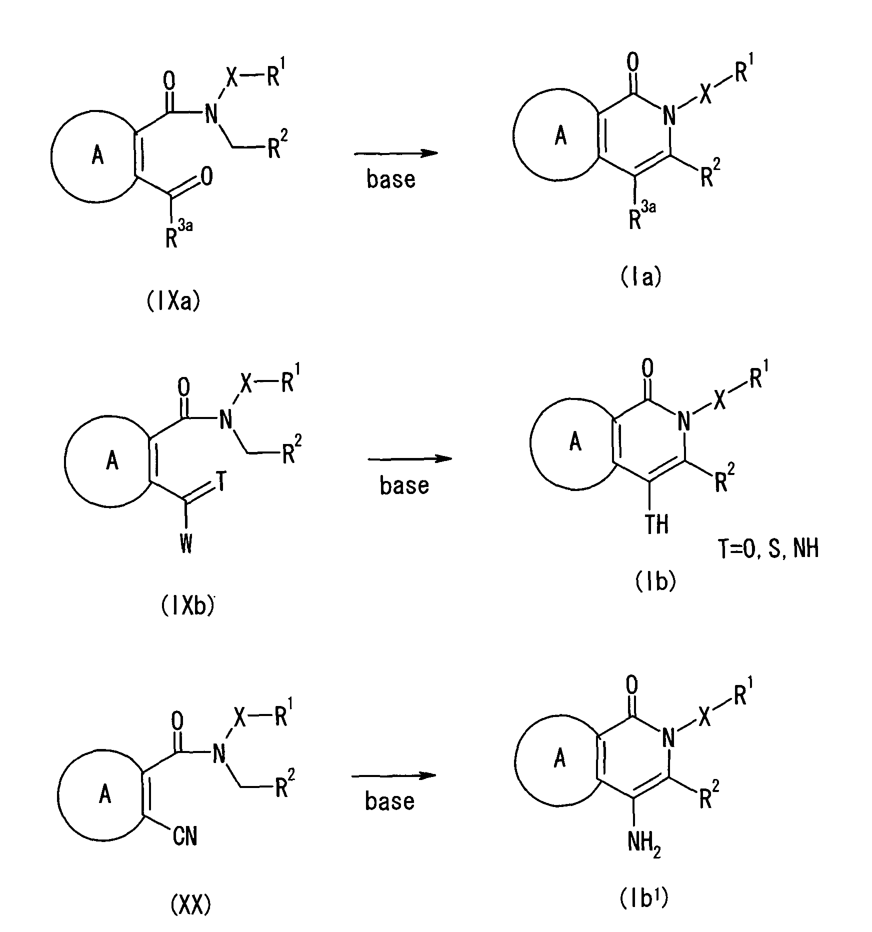

### Method B

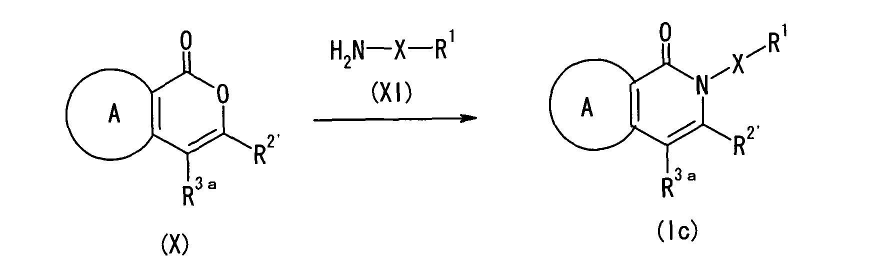

### Method C

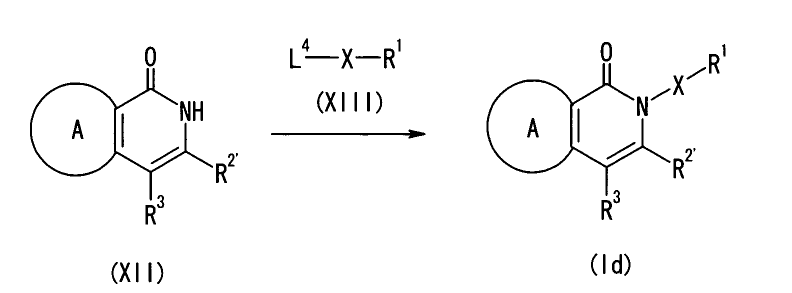

### Method D

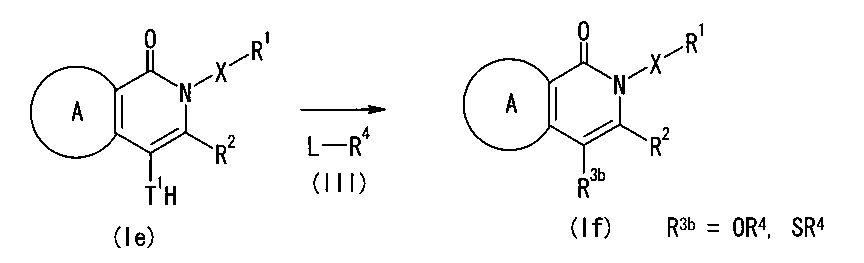

### Method E

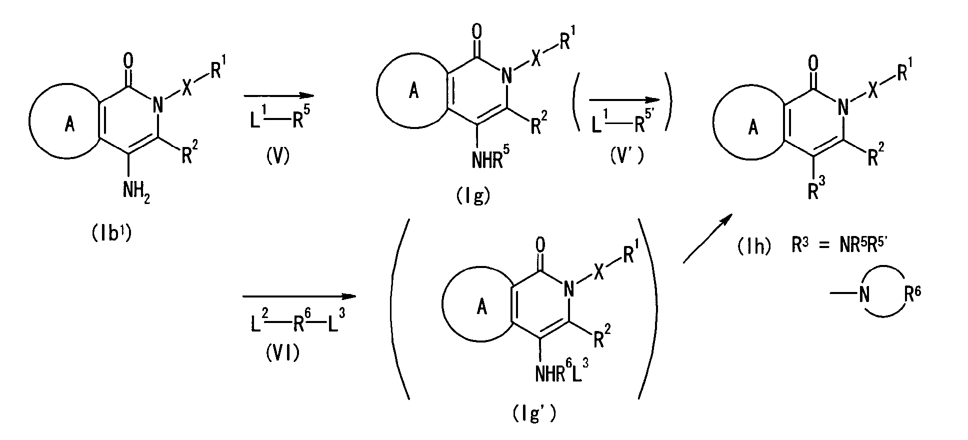

### Method F

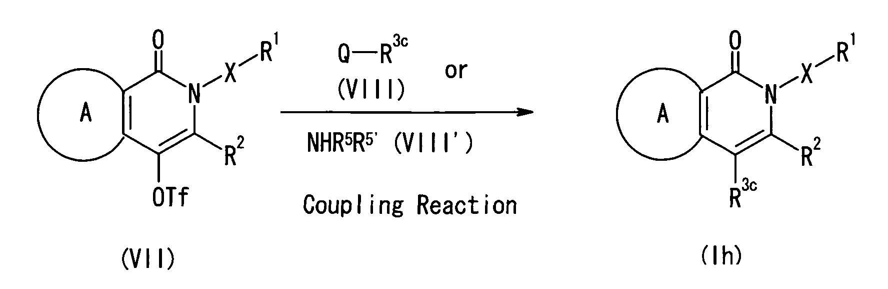

### Method A

In case that R³ of the compound (I) is an optionally substituted hydrocarbon group, an optionally substituted aromatic heterocyclic group, a hydroxy group, a thiol group or an amino group, each of a compound (Ia) which is the compound (I) wherein R³ is an optionally substituted hydrocarbon group or an optionally substituted aromatic heterocyclic group, or a salt thereof, a compound (Ib) which is the compound (I) wherein R³ is a hydroxy group, a thiol group or an amino group, or a salt thereof, and a compound (Ib¹) which is the compound (I) wherein R³ is an amino group, or a salt thereof can be prepared by subjecting a compound (IXa), (IXb) or (XX) represented by the formula (IXa), (IXb) or (XX): 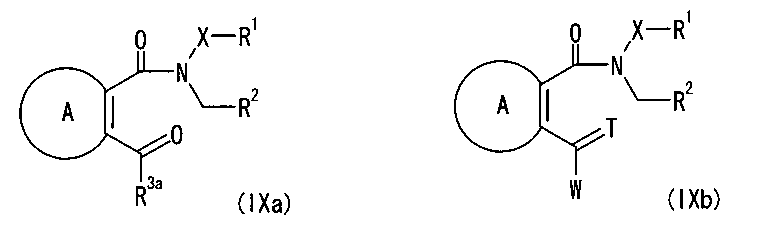 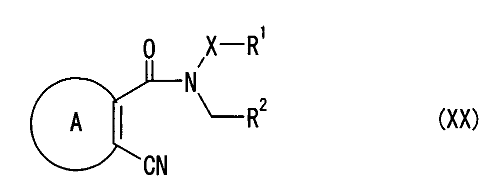 wherein R^{3a} represents an optionally substituted hydrocarbon group or an optionally substituted aromatic heterocyclic group; T represents O, S or NH; W represents an imidazolyl group, a lower alkoxy group or a lower alkylthio group; and the other symbols are as defined above, or a salt thereof, to intramolecular cyclization.

This cyclization is carried out by reacting the compound (IXa) or (IXb) with a base.

Generally, this reaction is carried out in a solvent and a solvent which does not interfere with the reaction is appropriately selected. Examples of the solvent include alcohols such as methanol, ethanol, propanol, isopropanol butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

In this reaction, as the base, for example, alkali metal hydrides such as potassium hydride, sodium hydride, etc.; metal C₁₋₆ alkoxides such as lithium ethoxide, lithium tert-butoxide, sodium methoxide, sodium ethoxide, potassium tert-butoxide, etc.; inorganic bases such as lithium hydroxide, potassium hydroxide, sodium hydroxide, sodium carbonate, potassium carbonate, sodium bicarbonate, etc.; organic amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; organic lithiums such as methyllithium, n-butyllithium, sec-butyllithium, tert-butyllithium, etc.; lithium amides such as lithium diisopropylamide, etc. are used.

In this reaction, the base is used in an amount of about 0.01 to about 100 mole, preferably about 0.1 to about 3 mole relative to 1 mole of the compound (IXa), (IXb) or (XX).

The reaction temperature is about -80°C to about 200°C, preferably about -20°C to about 100°C.

The reaction time varies depending upon the compound (IXa), (IXb) or (XX), the kind of the base catalyst, the kind of the solvent, the reaction temperature, and the like, but usually it is about 1 minute to about 72 hours, preferably about 15 minutes to about 24 hours.

### Method B

In case that R³ of the compound (I) is an optionally substituted hydrocarbon group or an optionally substituted aromatic heterocyclic group, a compound (Ic) which is the compound (I) wherein R³ is an optionally substituted hydrocarbon group or an optionally substituted aromatic heterocyclic group, or a salt thereof can be prepared by reacting a compound (X) represented by the formula (X): 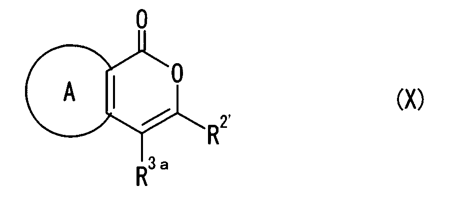 wherein R^{2'} represents an optionally esterified carboxyl group; R^{3a} represents an optionally substituted hydrocarbon group or an optionally substituted aromatic heterocyclic group; and the other symbols are as defined above, or a salt thereof is reacted with an amino compound (XI) represented by the formula (XI):

H²N-X-R¹ (XI)

wherein the symbols are as defined above, or a salt thereof, followed by dehydration.

This reaction is carried out in a solvent and a solvent which does not interfere with the reaction is appropriately selected. Examples of the solvent include alcohols such as methanol, ethanol, propanol, isopropanol butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichloroethylene, 1,2-dichloroethane, etc.; hydrocarbons such as n-hexane, benzene, toluene, etc.; amides such as formamide, N,N-dimethylformamide, N,N-dimethylacetamide, etc.; ketones such as acetone, methyl ethyl ketone, methyl isobutyl ketone, etc.; nitriles such as acetonitrile, propionitrile, etc.; as well as dimethylsulfoxide, sulfolane, hexamethylphosphoramide, water, and the like. These solvents can be used alone or as a mixed solvent thereof.

This reaction is preferably carried out in the presence of a base, as the base, for example, inorganic bases such as sodium carbonate, potassium carbonate, sodium bicarbonate, etc.; amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, etc. are used.

In this reaction, the compound (XI) is used in an amount of about 1 to about 20 mole, preferably about 1 to about 5 mole relative to 1 mole of the compound (X).

The reaction temperature is about -20°C to about 150°C, preferably about 10°C to about 80°C.

The reaction time varies depending upon the kind of compound (X)or (XI), the kind of the solvent, the reaction temperature, and the like, but usually it is about 1 minute to about 72 hours, preferably about 15 minutes to about 24 hours.

Sometimes, the dehydration step in this reaction is completed by simply reacting the compound (X) with the compound (XI) but, usually, the dehydration is carried out with an acid. Examples of the acid include organic acids such as acetic acid, trifluoroacetic acid, methanesulfonic acid, p-toluenesulfonic acid, etc.; mineral acids such as hydrochloric acid, sulfuric acid, nitric acid, phosphoric acid, hydrobromic acid, perchloric acid, etc.; Lewis acids such as aluminum chloride, zinc chloride, boron trifluoride etherate, titanium tetrachloride, etc.

The solvent to be used in the dehydration step is appropriately selected from solvents which do not interfere with the reaction and, as such a solvent, that for the reaction of the compound (X) and the compound (XI) is used.

The reaction temperature is about -20°C to about 200°C, preferably about 0°C to about 120°C.

The reaction time varies depending upon the reaction conditions, and is usually about 1 minute to about 72 hours, preferably about 15 minutes to about 15 hours.

### Method C

In case that X of the compound (I) is a bond or an alkylene group, a compound (Id) which is the compound (I)
wherein X is a bond or an alkylene group can be prepared by reacting a compound (XII) represented by the formula (XII): 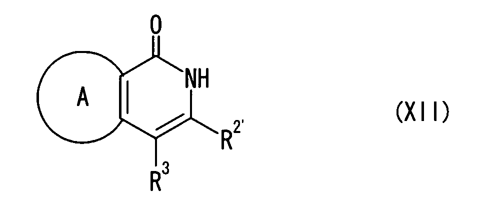 wherein R^{2'} represents an optionally esterified carboxyl group; and the other symbols are as defined above, or a salt with a compound (XIII) represented by the formula (XIII):

L⁴-X¹-R¹ (XIII)

wherein L⁴ is a leaving group (as defined with respect to the above L); and X¹ is a bond or an alkylene group.

This reaction is carried out by using the compound (XII) or a salt thereof and the compound (XIII) or a salt thereof and subjecting them to alkylation.

Generally, this reaction is carried out in a solvent in the presence of a base. Examples of the base include alkali metal hydrides such potassium hydride, sodium hydride, etc.; metal C₁₋₆ alkoxides such as lithium ethoxide, lithium tert-butoxide, sodium methoxide, sodium ethoxide, potassium tert-butoxide, etc.; inorganic bases such as lithium hydroxide, potassium hydroxide, sodium hydroxide, sodium carbonate, potassium carbonate, cesium carbonate, sodium bicarbonate, etc.; organic amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; 2-tert-butylimino-2-diethylamino-1,3-dimethyl-perhydro-1,3,2-diazaphosphorin or a resin thereof; and the like.

As the solvent, a solvent which does not interfere with the reaction is appropriately selected. Examples of the solvent include alcohols such as methanol, ethanol, propanol, isopropanol, butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

In this reaction, the compound (XIII) is used in an amount of about 1 to about 5 mole, preferably about 1 to about 2 mole relative to 1 mole of the compound (XII).

The reaction temperature is about -50°C to about 150°C, preferably about -20°C to about 100°C.

The reaction time varies depending upon the kind of compound (XII) or (XIII), the kind of the solvent and the base, the reaction temperature, and the like, but usually it is about 1 minute to about 100 hours, preferably about 15 minutes to about 48 hours.

### Method D

In case that R³ of the compound (I) is a substituted hydroxy group or a substituted thiol group, the compound (I) can be prepared by reacting a compound (Ie) represented by the formula (Ie): 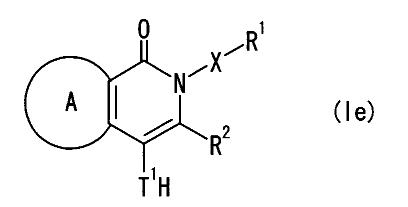 wherein T¹ represents an oxygen atom or a sulfur atom; and the other symbols are as defined above, or a salt thereof is reacted with a compound represented by the formula (III):

L-R⁴ (III)

wherein L represents a leaving group (e.g. a halogen atom (e.g. chlorine, bromine, iodine, etc.), a group represented by the formula R^{L}-SO₂-O- (wherein R^{L} represents a lower alkyl group optionally substituted with a halogen atom, an optionally substituted phenyl group, etc.), etc.) or a hydroxy group; and R⁴ represents a group corresponding to the substituent which is possessed by the hydroxy group or the thiol group of the "optionally substituted hydroxy group" or the "optionally substituted thiol group" represented by the above R³, or a salt thereof.

### Method D-1

In case that L is a hydroxy group, the compound (Ie) can be alkylated in a solvent which does not affect the reaction by Mitsunobu reaction.

In this reaction, a conventional aprotic solvent, for example, aromatic hydrocarbons such as toluene, benzene, etc.; carboxylic acid amides such as N,N-dimethylformamide, N,N-dimethylacetamide, N-methylpyrrolidone, etc.; sulfoxides such as dimethylsulfoxide, etc.; ketones such as acetone, methyl ethyl ketone, methyl isobutyl ketone, etc.; as well as acetonitrile, ethylene glycol dimethyl ether , tetrahydrofuran, dioxane, etc. can be used. Inter alia, tetrahydrofuran is preferable.

In Mitsunobu reaction, a combination of azodicarboxylic acids such as diethyl azodicarboxylate, diisopropyl azodicarboxylate, azodicarboxylic acid bisdimethylamide, etc. and phosphines such as triphenylphosphine, tributylphosphine, trimethylphosphine, etc. are preferably used as a reagent. In addition, phosphoranes such as cyanomethylene tributylphosphorane, etc. can be used alone.

This reaction is preferably carried out by using about 1 to 3 mole of the alcohol and about 1 to 3 mole of the reagent of Mitsunobu reaction relative to 1 mole of the compound (Ie) in tetrahydrofuran, usually, at 0°C to the boiling point of a solvent for 5 to 40 hours, preferably about 0°C to room temperature for about 1 to 20 hours.

### Method D-2

In case that L is a leaving group, examples of the lower alkyl group in the "lower alkyl group optionally substituted with a halogen atom" represented by R^{L} in the above formula include a C₁₋₆ alkyl group such as methyl, ethyl, propyl, isopropyl, butyl, isobutyl, sec-butyl, tert-butyl, pentyl, isopentyl, neopentyl, tert-pentyl, 1-ethylpropyl, hexyl, isohexyl, 1,1-dimethylbutyl, 2,2-diemthylbutyl, 3,3-dimethylbutyl, 2-ethylbutyl, etc. and, inter alia, a C₁₋₄ alkyl group such as methyl, ethyl, propyl, isopropyl, butyl, isobutyl, etc. is preferably. Examples of the lower alkyl substituted with a halogen atom (e.g. fluorine, chlorine, bromine, iodine, etc.) represented by R^{L} include trichloromethyl, trifluoromethyl, etc.

As the substituent of the "optionally substituted phenyl" represented by R^{L}, for example, a lower alkyl group (e.g. methyl, ethyl, propyl, isopropyl, butyl, etc.), a lower alkoxy group (e.g. C₁₋₆ alkoxy such as methoxy, ethoxy, propoxy, isopropoxy, butoxy, etc.), a halogen atom (e.g. fluorine, chlorine, bromine, iodine, etc.), a nitro group, a cyano group, etc. can be used.

This reaction is alkylation and, generally carried out in a solvent which does not affect the reaction in the presence of a base.

Examples of the base to be used in this reaction include alkali metal hydrides such potassium hydride, sodium hydride, etc.; metal C₁₋₆ alkoxides such as lithium ethoxide, lithium tert-butoxide, sodium methoxide, sodium ethoxide, potassium tert-butoxide, etc.; inorganic bases such as lithium hydroxide, potassium hydroxide, sodium hydroxide, sodium carbonate, potassium carbonate, cesium carbonate, sodium bicarbonate, etc.; organic amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; 2-tert-butylimino-2-diethylamino-1,3-dimethyl-perhydro-1,3,2-diazaphosphorin or a resin thereof; and the like.

In addition, in this reaction, an iodide such as potassium iodide, sodium iodide, etc. can be added as a reaction promoter.

This reaction is carried out in a solvent and a solvent which does not interfere with the reaction is appropriately selected. Examples of the solvent include alcohols such as methanol, ethanol, propanol, isopropanol butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

This reaction is preferably carried out by using about 1 to 3 mole of the alkylating agent (III) and about 1 to 3 mole of the base relative to 1 mole of the compound (Ie), usually, at 0°C to the boiling point of a solvent for 5 to 40 hours, preferably room temperature to 100°C for about 10 to 20 hours.

### Method E

In case that R³ of the compound (I) is a substituted amino group (including a cyclic amino group), the compound (I) can be prepared by reacting a compound (Ib¹) represented by the formula (Ib¹): 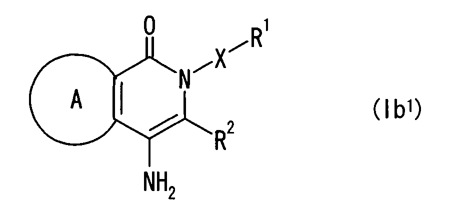 wherein the symbols are as defined above, or a salt thereof with an alkylating agent or an acyalting agent represented by the formula (V) (and (V')):

L¹-R⁵ (V)

(L¹-R^{5'} ) (V'))

wherein L¹ represents a leaving group; and R⁵ and R^{5'} represents a group corresponding to the substituent of the amino group of the above "optionally substituted amino group", respectively, or the formula (VI):

L²-R⁶-L³ (VI)

wherein L² and L³ represent a leaving group, respectively; and R⁶ represents a divalent group which can form a cyclic amino group represented by R³, or a salt thereof.

As the leaving group represented by L¹, L² and L³, the same group as the leaving group represented by the above L, etc. is used.

This reaction is alkylation or acylation and, generally, carried out in a solvent which does not affect the reaction in the presence of a base. Examples of the base to be used in this reaction include alkali metal hydrides such potassium hydride, sodium hydride, etc.; metal C₁₋₆ alkoxides such as lithium ethoxide, lithium tert-butoxide, sodium methoxide, sodium ethoxide, potassium tert-butoxide, etc.; inorganic bases such as lithium hydroxide, potassium hydroxide, sodium hydroxide, sodium carbonate, potassium carbonate, cesium carbonate, sodium bicarbonate, etc.; organic amines such as triethylamine, tri(n-propyl)amine, tri(n-butyl)amine, diisopropylethylamine, cyclohexyldimethylamine, pyridine, lutidine, γ-collidine, N,N-dimethylaniline, N-methylpiperidine, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; 2-tert-butylimino-2-diethylamino-1,3-dimethyl-perhydro-1,3,2-diazaphosphorin or a resin thereof; and the like.

As the solvent, a solvent which does not interfere with the reaction is appropriately selected. Examples of the solvent include alcohols such as methanol, ethanol, propanol, isopropanol, butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

When the compound (I) is obtained by reacting the compound (Ib¹) with the compound (V), the reaction can be carried out in one step or two steps. In case of the two step reaction, the compounds (V) and (V'), the base and the solvent to be used in each step may be the same or different.

This reaction is preferably carried out by using about 1 to 3 mole of the compound (V) or the compound (V') and about 1 to 3 mole of the base relative to 1 mole of the compound (Ib¹) or the compound (Ig), usually, at about 0°C to the boiling point of the solvent for 5 to 40 hours, preferably room temperature to about 100°C for about 10 to 20 hours.

Further, when the compound (I) is obtained by reacting the compound (Ib¹) with the compound (VI), the replacement reaction of L² and L³ can be carried out in one step or two steps. In case of the two step reaction, the base and the solvent to be used in each step may be the same or different.

This reaction is preferably carried out by using about 1 to 3 mole of the compound (VI) and about 2 to 4 mole of the base relative to 1 mole of the compound (Ib¹), usually, at about 0°C to the boiling point of the solvent for 5 to 40 hours, preferably room temperature to about 100°C for about 10 to 20 hours. In addition, in case of the two step reaction, the reaction of the second step is preferably carried out by using about 2 to 4 mole of the base relative to 1 mole of the compound (Ig'), usually, at about 0°C to the boiling point of the solvent for 5 to 40 hours, preferably room temperature to about 100°C for about 10 to 20 hours.

As a method similar to Method E, a compound which is the compound (I) wherein R³ is optionally substituted pyrrolyl can be prepared by reacting the compound (Ib¹) with a 2,5-dimethoxytetrahydrofuran derivative in the presence of an acid or a base according to a per se known method (e.g. the method disclosed in SYNTHETIC COMMUNICATION, 1991, 21(15-16), PP. 1567-1576) or a modification thereof.

Further, a compound which is the compound (I) wherein R³ is optionally substituted triazolyl can be prepared by reacting the compound (Ib¹) with hydrazines such as 1,2-bis[(dimethylamino)methylene hydrazine, etc. (Journal of American Chemical Society, 1995, 117(22), pp. 5951-5957, etc.) or a [1,3,4]oxadiazole derivative (Journal of Heterocyclic Chemistry, 1989, 26(1), pp. 225-230, etc.).

### Method F

In case that R³ of the compound (I) is an optionally substituted hydrocarbon group, an optionally substituted heterocyclic group, or an optionally substituted amino group, the compound (I) can be prepared by reacting a compound (VII) represented by the formula (VII): 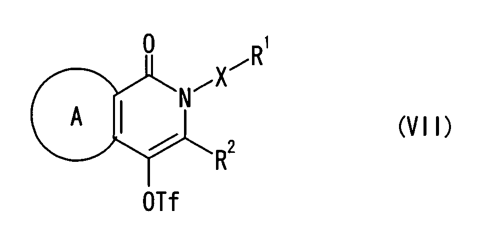 wherein OTf represents a trifluoromethanesulfonyloxy group; and the other symbols are as defined above, or a salt thereof with a compound (VIII) represented by the formula (VIII):

Q-R^{3c} (VIII)

wherein Q represents an atomic group capable of a cross coupling reaction (e.g. an atomic group bonding via boron, tin, magnesium, zinc, etc.); and R^{3c} represents an optionally substituted heterocyclic group or an optionally substituted hydrocarbon group, or a salt thereof, or an amino compound (VIII') represented by the formula (VIII'):

NHR⁵R^{5'} (VIII')

wherein R⁵ and R⁵' represent a group corresponding to the substituent which may be possessed by the amino group of the above "optionally substituted amino group", and R⁵ and R⁵' together with a nitrogen atom may form a cyclic amino group, or a salt thereof.

In this method, the compound (I) is prepared by subjecting the compound (VII) or a salt thereof and the compound (VIII) or a salt thereof or the amino compound (VIII') or a salt thereof to a cross coupling reaction (e.g. Suzuki cross coupling reaction, Heck reaction, Stille coupling reaction, Buchwald's amination, etc.).

This reaction is carried out in the presence of a base. Examples of the base include inorganic bases, for example, alkali metal hydrides such as sodium hydride, potassium hydride, sodium hydride, etc., alkali metal hydroxides such as lithium hydroxide, sodium hydroxide, potassium hydroxide, etc., alkaline earth metal hydroxides such as magnesium hydroxide, calcium hydroxide, etc. alkali metal carbonates such as sodium carbonate, potassium carbonate, etc., alkali metal bicarbonates such as sodium bicarbonate, potassium bicarbonate, etc.; metal C₁₋₆ alkoxides such as sodium methoxide, sodium ethoxide, sodium tert-butoxide, etc.; organic amines such as trimethylamine, triethylamine, diisopropylethylamine, pyridine, picoline, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; organic lithiums such as methyllithium, n-butyllithium, sec-butyllithium, tert-butyllithium, etc.; lithium amides such as lithium diisopropylamide, etc.; and the like.

Generally, this reaction is carried out in a solvent. As the solvent, a solvent which does not interfere with the reaction is appropriately selected. Examples of the solvent include alcohols such as methanol, ethanol, propanol, isopropanol, butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

Generally, this cross coupling reaction can be promoted by using a metal catalyst. In this reaction, metal complexes having various ligands are used as the metal catalyst. Examples of the catalyst include palladium compounds [e.g. palladium acetate, tetrakis(triphenylphosphine)palladium, bis(triphenylphosphine)palladium chloride, dichlorobis(triethylphosphine)palladium, tris(dibenzylideneacetone)dipalladium-2,2-bis(diphenylphosphino)-1,1'-binaphthyl, a complex of palladium (II) acetate and 1,1'-bis(diphenylphosphino)ferrocene, etc.], nickel compounds (e.g. tetrakis(triphenylphosphine)nickel, bis(triethylphosphine)nickel chloride, bis(triphenylphosphine)nickel chloride, etc.), rhodium compounds [e.g. tri(triphenylphosphine)rhodium chloride, etc.], cobalt compounds, platinum compounds, and the like. Inter alia, palladium and nickel compounds are preferable. The amount of the catalyst to be used is about 1 to 0.000001 mole, preferably about 0.1 to 0.0001 mole relative to 1 mole of the compound (VII).

This reaction is preferably carried out by using about 0.8 to 10 mole, preferably about 0.9 to 2 mole of the compound (VIII) or the compound (VIII') and about 1 to about 20 mole, preferably about 1 to about 5 mole of the base relative to 1 mole of the compound (VII).

The reaction temperature is about -10°C to about 250°C, preferably about 0°C to about 150°C.

The reaction time varies depending upon the kinds of the compound (VII), the compound (VIII) or the compound (VIII'), the metal catalyst, the base, the solvent, etc., usually, it is about 1 minute to about 200 hours, preferably about 5 minutes to about 100 hours.

### Method G

A compound (Ii) which is the compound (I) wherein R³ is a group represented by the formula -SOR⁷ or -SO₂R⁷ represented by the formula (Ii): 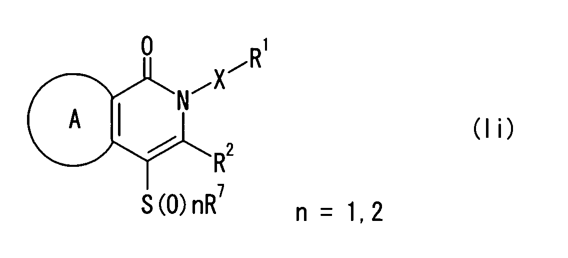 wherein R⁷ represents an optionally substituted hydrocarbon group or an optionally substituted heterocyclic group; n represents 1 or 2; and the other symbols are as defined above, can be obtained by the above Method E, or by oxidizing a compound (Ij) represented by the formula (Ij): 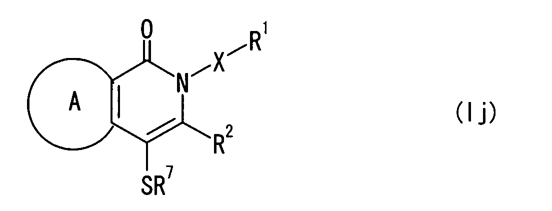 wherein the symbols are as defined above, which is obtained by the above Method D. This oxidation can be carried out by a per se known method or a modification thereof and, for example, a method using an oxidizing agent can be used. Examples of the oxidizing agent to be used include peroxides such as peracetic acid, 3-chloroperbenzoate, sodium metaperiodate, Oxone, etc. Usually, for sulfoxidation, one equivalent of an oxidizing agent is used and, for sulfonation, 2 to 5 equivalents of an oxidizing agent is used. Sodium metaperiodate is mainly used for sulfoxidation and can be used in an amount of 1 equivalent or more.

This reaction is advantageously carried out in a solvent and, as the solvent, usually, water, alcohols such as methanol, ethanol, propanol, etc., ethers such as tetrahydrofuran, dimethoxyethane, dioxane, etc., acetonitrile, amides such as N,N-dimethylformamide, etc., halogenated hydrocarbons such as dichloromethane, chloroform, chlorobenzene, etc., and a mixed solvent thereof, as well as other solvents which do not adversely affect the reaction are used.

The reaction is usually carried out in a temperature range of -20°C to 120°C (preferably 0°C to 50°C). The reaction time is usually, about 10 minutes to 48 hours, preferably 0.5 hour to about 24 hours.

### Method H

A compound (Ik) which is the compound (I) wherein R² is a carboxyl group represented by the formula (Ik): 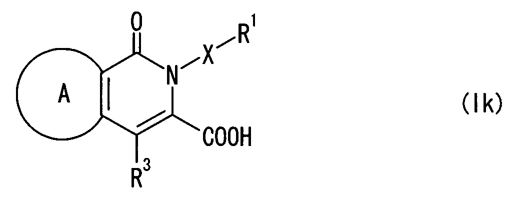 wherein the symbols are as defined above, can be obtained by hydrolyzing a compound (Im) represented by the formula (Im): 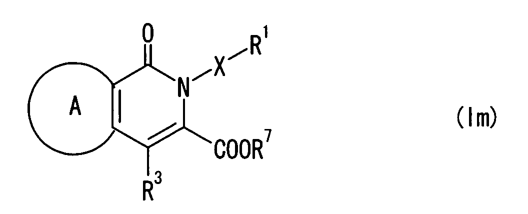 wherein R⁷ represent an optionally substituted lower (C₁₋₆) alkyl group (e.g. methyl, ethyl, propyl, butyl, tert-butyl, etc.); and the other symbols are as defined above, which is obtained by the above Methods A to E. This hydrolysis can be carried out by a per se known method or a modification thereof and, for example, a method with an acid, a method with a base, a method by reduction, a method with ultraviolet light, a method with tetrabutylammonium fluoride, a method with a palladium acetate, etc. can be used. The method with an acid is mainly used in case of the t-butyl ester, and preferable examples of the acid to be used include organic acids such as formic acid, trifluoroacetic acid, benzenesulfonic acid, p-toluenesulfonic acid, etc.; inorganic acids such as hydrochloric acid, hydrobromic acid, sulfuric acid, etc. The method with a base is usually used in case of the lower alkyl ester, and preferable examples of the base to be used include inorganic bases, for example, alkali metal hydroxides such as lithium hydroxide, sodium hydroxide, potassium hydroxide, etc.; alkaline earth metal hydroxides such as magnesium hydroxide, calcium hydroxide, etc.; alkali metal carbonates such as sodium carbonate, potassium carbonate, etc.; alkali metal bicarbonates such as sodium bicarbonate, potassium bicarbonate, etc.; alkali metal acetates such as sodium acetate, potassium acetate, etc.; alkaline earth metal phosphates such as calcium phosphate, magnesium phosphate, etc.; alkali metal hydrogen phosphates such as disodium hydrogen phosphate, dipotassium hydrogen phosphate, etc.; and aqueous ammonia. The method by reduction is used for deprotection of a carboxyl group protected with, for example, benzyloxymethyl, benzyl, p-nitrobenzyl, benzhydryl, etc. Preferable examples of the reduction to be used include reduction with zinc/acetic acid, catalytic reduction, etc. The method with ultraviolet light is used, for example, as a method for deprotection of a carboxylic group protected with o-nitrobenzyl. The method with tetrabutylammonium fluoride is used as a method for deprotection of a silyl ether type ester such as 2-trimethylsilylethyl, etc. and silyl esters to obtain a carboxyl group. The method with palladium acetate is used, for example, as a method for deprotection of an allyl ester to obtain a carboxyl group.

This reaction is advantageously carried out in a solvent and, as the solvent, usually, water, alcohols such as methanol, ethanol, propanol, etc., aprotic and protic solvents, for example, ethers such as tetrahydrofuran, dimethoxyethane, dioxane, etc., amides such as N,N-dimethylformamide, etc., sulfoxides such as dimethylsulfoxide, etc., and a mixed solvent thereof, as well as other solvents which do not adversely affect the reaction are used. A liquid acid or base can be used as the solvent. The reaction is usually carried out in a temperature range of -20°C to 120°C (preferably 0°C to 100°C). The reaction time is, usually, about 10 minutes to 48 hours, preferably 0.5 hour to about 24 hours.

In addition, various derivatives can be prepared by using the compound (Ik) thus obtained and modifying the carboxyl group of the compound (Ik) according to a per se known method or a modification thereof.

For example,
1) a compound (In) represented by the formula (In): 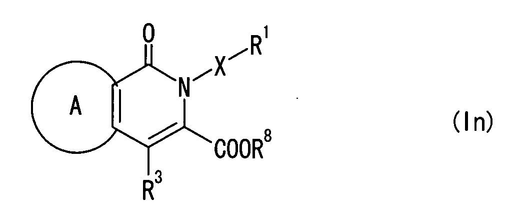 wherein R⁸ represented by an optionally substituted hydrocarbon group or an optionally substituted heterocyclic group; and the other symbols are as defined above can be prepared by esterification of the compound (Ik).
   This esterification can be carried out according to a per se known method or a modification thereof. For example, there are methods wherein the compound (Ik) is reacted with a compound represented by the formula R⁸-L⁵ [wherein L⁵ represents a leaving group (the same as defined with respect to the above L); and the other symbol is as defined above] in the presence of a base; the compound (Ik) is reacted with an alcohol represented by R⁸-OH in the presence of an acid catalyst; condensation is carried out by using a condensing agent [e.g. carbodiimides (DCC, WSC, DIC, etc.), phosphoric acid derivatives (e.g. cyano diethyl phosphate, diphenyl phosphate azide, BOP-C1, etc.), etc.]; Mitsunobu reaction is carried out using a reagent such as tripheyl phosphine and diethyl azodicarboxylate, etc.; a reactive derivative of the compound (Ik) (e.g. acid halide, activated ester, acid azide, etc.) is reacted with an alcohol represented by R⁸-OH in the presence of a base; and the like.
2) a compound (Ip) represented by the formula (Ip): 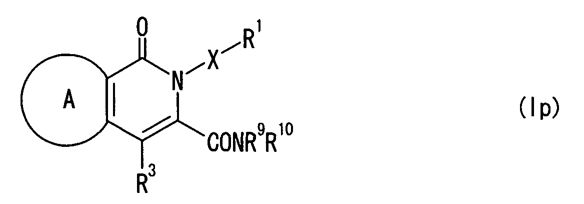 wherein R⁹ and R¹⁰ represent a hydrogen atom or a group corresponding to the substituent which may be possessed by the "optionally substituted carbamoyl group" represented by the above R², can be prepared by amidation of the compound (Ik).

This amidation can be carried out by a per se known method or a modification thereof. For example, the compound (Ik) and an amine are reacted with a condensing agent [e.g. carbodiimides (DCC, WSC, DIC, etc.), phosphoric acid derivatives (e.g. cyano diethyl phosphate, DPPA, BOP-Cl, etc.), etc.]; a reactive derivative of the compound (Ik) (e.g. acid halide, acid anhydride, activated ester, acid imidazolide, acid azide, etc.) is reacted with an amine; and the like.

### Method I

In case that the substituents of R¹, R², R³ and the ring A of the compound (I) have functional groups which can be converted into the substituents (e.g. carboxyl group, amino group, hydroxy group, carbonyl group, thiol group, ester group, sulfo group, halogen atom, etc.), various compounds can be prepared by converting the functional groups according to a per se known method or a modification thereof.

For example, in case of a carboxyl group, it can be converted by a reaction such as esterification, reduction, amidation, conversion into an amino group which may be protected, etc. as shown in the above Method G. In case of an amino group, it can be converted by, for example, a reaction such as amidation, sulfonylation, nitrosation, alkylation, arylation, imidation, etc. In case of a hydroxy group, it can be converted by a reaction such as esterification, carbamoylation, sulfonylation, alkylation, arylation, oxidation, halogenation, etc. In case of a carbonyl group, it can be converted by a reaction such as reduction, oxidation, imination (including oxime formation, hydrozone formation), (thio)ketal formation, alkylidene formation, thiocarbonylation, etc. In case of a thiol group, it can be converted by a reaction such as alkylation, oxidation, etc. In case of a sulfo group, it can be converted by a reaction such as sulfonamidation, reduction, etc. In case of a halogen atom, it can be converted by a reaction such as various nucleophilic substitution reactions, various coupling reactions etc.

The starting compounds (IXa), (IXb), (XX), (VII), (X) and (XII) used in the Method A to F can be prepared, for example, the following methods or a modification thereof.

### Method N

The compound (IXa) represented by the formula (IXa): 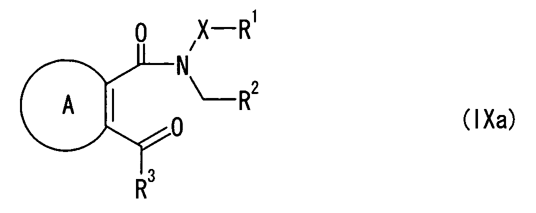 wherein the symbols are defined as above, or a salt thereof can be prepared by reacting a compound (XXII) represented by the formula (XXII): 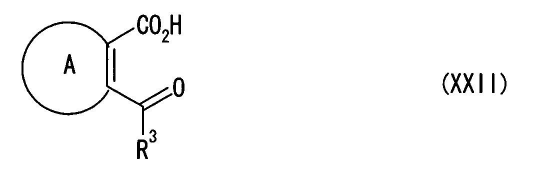 wherein the symbols are as defined above, or a salt or a reactive derivative (e.g. acid halide, acid anhydride, activated ester, ester, acid imidazolide, acid azide, etc.) thereof with a compound (XV) represented by the formula (XV): 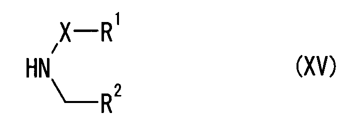 wherein the symbols are as defined above, or a salt thereof.

This reaction is amidation and the reactive derivative of the compound (XXII), reaction conditions, reaction solvent, reaction time, etc. are according to those illustrated with respect to Method H-3.

### Method I

The amide compound (IXb) represented by the formula (IXb): 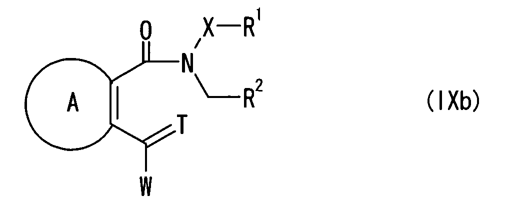 wherein the symbols are as defined above, or a salt thereof can be prepared by the following method. It can be prepared by reacting a compound (XIV) represented by the formula (XIV): 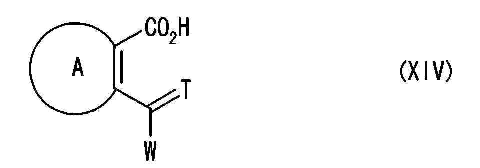 wherein the symbols are as defined above, or a salt or a reactive derivative (e.g. acid halide, acid anhydride, activated ester, ester, acid imidazolide, acid azide, etc.) thereof with a compound (XV) represented by the formula (XV) : 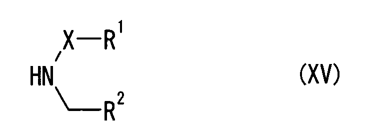 wherein the symbols are as defined above, or a salt thereof.

This reaction is amidation and the reactive derivative of the compound (XXII), reaction conditions, reaction solvent, reaction time, etc. are according to those illustrated with respect to Method H-3.

A compound (IXb²) which is the compound (IXb) wherein T is an oxygen atom and is used in the above Method I can also be prepared according to the following Method J.

### Method J

A compound (XVII) represented by the formula (XVII): 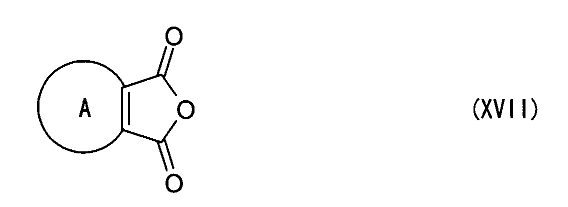 wherein the symbol is as defined above, or a salt thereof can be reacted with a compound (XV) represented by the formula (XV): 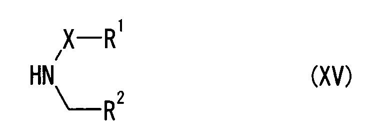 wherein the symbols are as defined above, or a salt thereof to prepare a compound (XVIII) represented by the formula (XVIII): 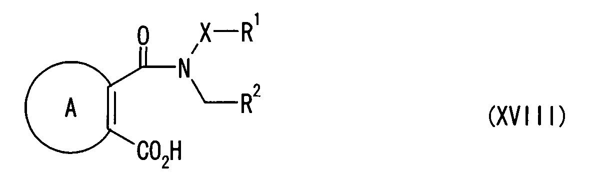 wherein the symbols are as defined above, or a salt thereof.

This reaction is carried out according to a conventional method in a solvent which does not affect the reaction.

Examples of the solvent which does not affect the reaction include halogenated hydrocarbons such as chloroform, dichloromethane, etc.; aromatic hydrocarbons such as benzene, toluene, etc.; ethers such as tetrahydrofuran, dioxane, diethyl ether, etc.; ethyl acetate; and the like. These solvents may be used by mixing them in an appropriate ratio.

The amount of the compound (XV) is about 1 to about 10 molar equivalents, preferably 1 to 3 molar equivalents relative to the compound (XVII).

The reaction temperature is usually in a temperature range of -30°C to 150°C, preferably 0°C to 100°C. The reaction time is, usually, about 10 minutes to 48 hours, preferably 0.5 hour to about 20 hours.

The thus obtained compound (XVIII) can be isolated and purified by a known separation and purification means such as concentration, concentration under reduced pressure, solvent extraction, crystallization, recrystallization, transfer dissolution, chromatography, and the like.

The compound (XVIII) thus obtained leads to the compound (IXb²) according to the reaction of the above Method H-1.

### Method K

The compound (XX) represented by the formula (XX): 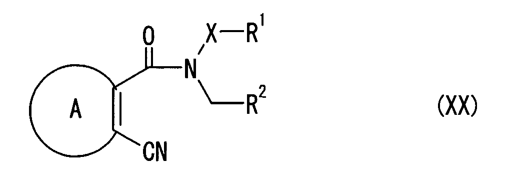 wherein the symbols are as defined above, or a salt thereof can be prepared, for example, by the following method. It can be prepared by reacting a compound (XIX) represented by the formula (XIX): 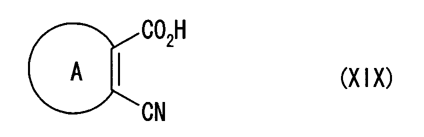 or a salt or a reactive derivative (e.g. acid halide, acid anhydride, activated ester, ester, acid imidazolide, acid azide, etc.) thereof with a compound (XV) represented by the formula (XV): 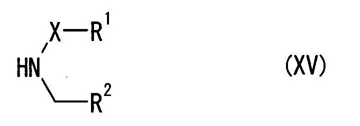 wherein the symbols are as defined above, or a salt thereof.

This reaction is an amidation and the reactive derivative of the compound (XIX), reaction conditions, reaction solvent, reaction time, etc. are according to those illustrated with respect to Method H-3.

### Method L

The isocoumarin compound (X) represented by the formula (X'): 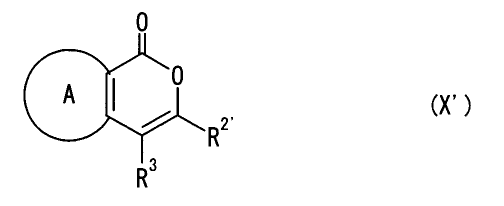 wherein R^{2'} represents an optionally esterified carboxyl group; and the other symbols are as defined above, or a salt thereof can be prepared, for example, by the following method. That is, the isocoumarin compound (X) wherein the 3-position is a carboxyl group can be prepared by reacting a compound (XXII) represented by the formula (XXII): 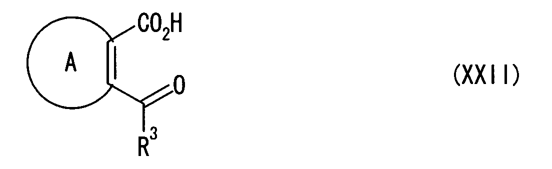 wherein the symbols are as defined above, or a salt thereof with a compound (XXIII) represented by the formula (XXIII): 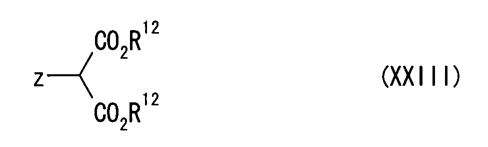 wherein Z represents a leaving group (the same as the above L); R¹² represents a lower (C₁₋₆)alkyl group (e.g. methyl, ethyl, propyl, butyl, tert-butyl, etc.), or a salt thereof in the presence of a base, followed by dehydration and decarboxylation under acidic conditions. Further, if desired, the carboxylic acid can be esterified to prepare the corresponding 3-ester compound.

### Method M

The compound (XII) represented by the formula (XII): 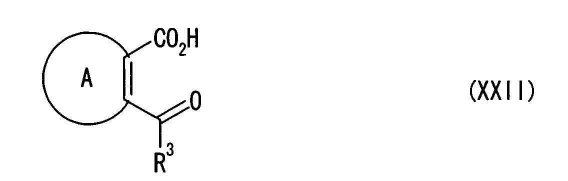 wherein the symbols are as defined above, or a salt thereof can be prepared by reacting a compound (X') represented by the formula (X'): 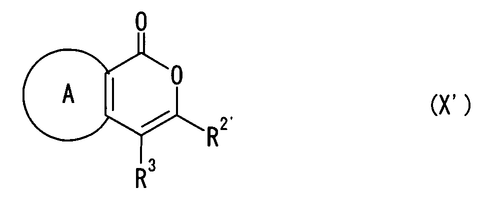 wherein R^{2'} represents an optionally esterified carboxyl group; and the other symbols are as defined above, or a salt thereof with ammonia, followed by dehydration under acidic conditions.

### Method O

The compound (VII) represented by the formula (VII): 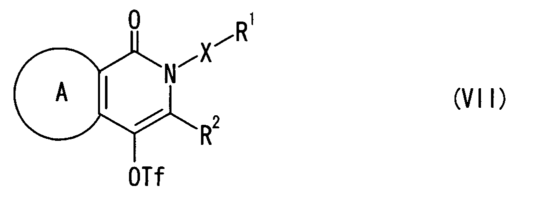 wherein the symbols are as defined above, or a salt thereof can be prepared by reacting the compound (II) represented by the formula (II): 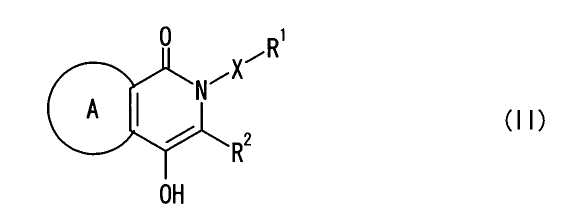 wherein the symbols are as defined above, or a salt thereof with a triflate formation reagent (e.g. trifluoromethanesulfonic acid anhydride, bis (trifluromethanesulfonyl) aniline, etc.) in the presence of a base.

Preferable examples of the base to be used in this reaction include inorganic bases, for example, alkali metal hydrides such as potassium hydride, sodium hydride, etc., alkali metal hydroxides such as lithium hydroxide, sodium hydroxide, potassium hydroxide, etc., alkaline earth metal hydroxides such as magnesium hydroxide, calcium hydroxide, etc., alkali metal carbonates such as sodium carbonate, potassium carbonate, etc., alkali metal bicarbonates such as sodium bicarbonate, potassium bicarbonate, etc.; metal C₁₋₆ alkoxides such as sodium methoxide, sodium ethoxide, sodium tert-butoxide, etc.; organic amines such as trimethylamine, diisopropylethylamine, pyridine, picoline, N-methylpyrrolidine, N-methylmorpholine, 1,5-azabicyclo[4.3.0]non-5-ene, 1,4-azabicyclo[2.2.2]octane, 1,8-azabicyclo[5.4.0]-7-undecene, etc.; organic lithiums such as methyl lithium, n-butyl lithium, sec-butyl lithium, tert-butyl lithium, etc.; and lithium amides such as lithium diisopropylamindes.

This reaction is generally carried out in a solvent. As the solvent, a solvent which does not interfere with the reaction is appropriately selected. Examples of the solvent include alcohols such as methanol, ethanol, propanol, isopropanol butanol, tert-butanol etc.; ethers such as dioxane, tetrahydrofuran, diethyl ether, tert-butyl methyl ether, diisopropyl ether, ethylene glycol-dimethyl ether, etc.; esters such as ethyl formate, ethyl acetate, n-butyl acetate, etc.; halogenated hydrocarbons such as dichloromethane, chloroform, carbon tetrachloride, trichlene, 1,2-dichloroethane, etc.; hydrocarbons such as n-hexane, benzene, toluene, etc.; amides such as formamide, N,N-dimethylformamide, N,N-dimethylacetamide, etc.; nitriles such as acetonitrile, propionitrile, etc.; as well as dimethylsulfoxide, sulfolane, hexamethylphosphoramide, water, and the like. These solvents can be used alone or as a mixed solvent thereof.

The starting compounds (XIV), (XV), (XVII), (XIX) and (XXII) used in the aforementioned Methods A to O are commercially available or can be prepared according to a per se known method or a modification thereof.

When a free compound is obtained in the above each reaction of the present invention, it may be converted into a salt according to a conventional method. On the other hand, when the compound is obtained as a salt, it can be converted into the free compound or another salt according to a conventional method.

In each reaction in the above process for preparing the compound (I) or each reaction of synthesizing a starting compound, when a starting compound has an amino group, a carboxyl group, or a hydroxy group as a substituent, a protecting group which is generally used in peptide chemistry may be introduced into these groups and, if necessary, a protecting group is removed after the reaction, thereby, the objective compound can be obtained.

As a protecting group for an amino group, for example, formyl, optionally substituted, C₁₋₆ alkyl-carbonyl (e.g. acetyl, ethylcarbonyl etc.), phenylcarbonyl, C₁₋₆ alkyloxycarbonyl (e.g. methoxycarbonyl, ethoxycarbonyl, tert-butoxycarbonyl (Boc) etc.), allyloxycarbonyl (Aloc), phenyloxycarbonyl, fluorenylmethyloxycarbonyl (Fmoc), C₇₋₁₀ aralkyl-carbonyl (e.g. benzylcarbonyl etc.), C₇₋₁₀ aralkyloxycarbonyl (e.g. benzyloxycarbonyl (Z) etc.), C₇₋₁₀ aralkyl (e.g. benzyl, etc.), trityl, phthaloyl or N,N-dimethylaminomethylene is used. As a substituent for them, a phenyl group, a halogen atom (e.g. fluorine, chlorine, bromine, iodide), C₁₋₆ alkyl-carbonyl (e.g. methylcarbonyl, ethylcarbonyl, butylcarbonyl etc.), and a nitro group are used, and the number of substituents is around 1 to 3.

As a protecting group for a carboxyl group, for example, optionally substituted, C₁₋₆ alkyl (e.g. methyl, ethyl, n-propyl, i-propyl, n-butyl, tert-butyl etc.), allyl, benzyl, phenyl, trityl or trialkylsilyl is used. As a substituent for them, a halogen atom (e.g. fluorine, chlorine, bromine, iodine), formyl, C₁₋₆ alkyl-carbonyl (e.g. acetyl, ethylcarbonyl, butylcarbonyl etc.), and a nitro group are used, and the number of substituents is about 1 to 3.

As a protecting group for a hydroxyl group, for example, optionally substituted, C₁₋₆ alkyl (e.g. methyl, ethyl, n-propyl, i-propyl, n-butyl, tert-butyl etc.), C₇₋₁₀ aralkyl (e.g. benzyl etc.), formyl, C₁₋₆ alkyl-carbonyl (e.g. acetyl, ethylcarbonyl etc.), benzoyl, C₇₋₁₀ aralkyl-carbonyl (e.g. benzylcarbonyl etc.), tetrahydropyranyl, furanyl or silyl is used. As a substituent for them, a halogen atom (e.g. fluorine, chlorine, bromine, iodine), C₁₋₆ alkyl (e.g. methyl, ethyl, n-propyl etc.), phenyl, C₇₋₁₀ aralkyl (e.g. benzyl), C₁₋₆ alkoxy (e.g. methoxy, ethoxy, n-propoxy etc.), and a nitro group are used, and the number of substituents is around 1 to 4.

In addition, as a method of removing a protecting group, the known per se method or a similar method is used, for example, a method of treating with an acid, a base, reduction, ultraviolet light, hydrazine, phenylhydrazine, sodium N-methyldithiocarbamate, tetrabutylammonium fluoride, or palladium acetate is used.

The compound (I) thus obtained can be isolated and purified by using a known separation and purification means such as extraction, concentration, neutralization, filtration, distillation, recrystallization, column chromatography, thin layer chromatography, preparative high performance liquid chromatography (HPLC), moderate pressure preparative liquid chromatograph (moderate pressure preparative LC), and the like.

The salt of the compound (I) can be prepared, for example, by addition of an inorganic acid or an organic acid in case that the compound (I) is a basic compound, or by addition of an organic base or an inorganic base in case that the compound (I) is an acidic compound according to a per se known means.

When there are optical isomers in the compound (I), each individual optical isomer and a mixture thereof are also included in the scope of the present invention. If desired, such isomers can be subjected to optical resolution according to a per se known means, or can be prepared individually.

Further, the compound (I) may be hydrated, and both hydrate and non-hydrate are included in the scope of the present invention. Furthermore, the compound (I) may be labeled with an isotope (e.g. ³H, ¹⁴C, ³⁵S, ¹²⁵I etc.).

Since a RFRP receptor function modulating agent containing the compound (I) or a salt thereof or a prodrug thereof (hereinafter, abbreviated as compound (I) of the present invention) has low toxicity and little side effect, it is useful as a safe medicament.

AN RFRP receptor is a receptor to which peptides having an RF amide structure (e.g. RFRPs such as RFRP-1, RFRP-2, RFRP-3, etc. described in WO00/29441) can bind, and examples include a G protein conjugated-type receptor protein OT7T022 (e.g. a human RFRP receptor having an amino acid sequence represented by SEQ ID No.: 1, a rat RFRP receptor having an amino acid sequence represented by SEQ ID No.:2), and the like.

Function modulating refers to both of activity of inhibiting the function of an RFRP receptor (e.g. RFRP receptor antagonizing activity, RFRP receptor antagonist activity), and activity of promoting the function of an RFRP receptor (e.g. RFRP receptor agonistic activity, RFRP receptor agonist activity). In the present invention, activity of inhibiting the function of an RFRP receptor, inter alia RFRP receptor antagonist activity is more preferable.

RFRP receptor function modulating activity, RFRP receptor agonist activity, and RFRP receptor antagonist activity can be measured using a method of screening a compound by changing binding property between RFRP and OT7T022 described in WO 00/29441.

Since the agent for modulating the function of an RFRP receptor of the present invention exhibits excellent RFRP receptor function modulating activity, particularly, RFRP receptor antagonizing activity (RFRP receptor antagonist activity) to a mammal (e.g. mouse, rat, hamster, rabbit, cat, dog, cow, sheep, monkey, human etc.), and is excellent in (oral) absorbing property, and (metabolism) stability, it is useful as an agent for preventing or treating RFRP-associated morbid state or a disease involved in RFRP.

Further, the agent for modulating the function of an RFRP receptor of the present invention is useful as an analgesic, an agent for promoting analgesic activity of another analgesic (e.g. morphine type anesthetic analgesic such as morphine, codeine, dihydrocodeine, ethylmorphine, oxycodone, heroin, etc.), or an agent for avoiding resistance due to another analgesic (e.g. morphine type anesthetic analgesic such as morphine, codeine, dihydrocodeine, ethylmorphine, oxycodone, heroin, etc.).

Further, an agent for modulating the function of an RFRP receptor of the present invention is useful as an agent for modulating the prolactin secretion, preferably also as an agent for suppressing the prolactin secretion, and is useful as an agent for preventing or treating, for example, hyperprolactinenia, pituitary gland tumor, diencephalons, emmeniopathy, stress, autoimmune disease, prolactinoma, infertility, impotence, amenorrhea, lactic leakage, acromegaly, Chiari-Frommel syndrome, Argonz-del Castilo syndrome, Forbes-Albright syndrome, breast cancer lymphoma, Sheehan's syndrome, or spermatogenesis abnormality.

Further, the agent for modulating the function of an RFRP receptor of the present invention is useful as an agent for preventing, treating or improving a muscular disease, adrenal gland function disorder, spasm, aggressive behavior, walking abnormality, body temperature elevation, decrease in the number of leukocyte, decrease in the number of platelets, increase in spontaneous behavior amount or decrease in a muscular force.

Further, the agent for modulating the function of an RFRP receptor of the present invention is useful as an agent for modulating the male hormone secretion, preferably as an agent for inhibiting the male hormone secretion(an agent for suppressing the male hormone secretion). Specifically, an agent for modulating the function of an RFRP receptor is useful as agent for preventing or treating, for example, male gonad function failure, male infertility accompanied with spermatogenesis function disorder, aplastic anemia, marrow fibrosis, renal anemia, pain alleviation of end female sexual organ cancer, breast cancer (e.g. unoperational breast cancer), mastopathy, mammary gland tumor, or gynecomastia.

Further, the agent for modulating the function of an RFRP receptor of the present invention is useful as an agent for suppressing the pancreatic glucagon secretion, a blood glucose lowering agent, a uropoiesis suppressing agent, or an agent for suppressing the deterioration of memory and learning abilities (an agent for suppressing the memory decrease), and is useful as an agent for preventing or treating diabetes, glucose tolerance disorder, ketosis, acidosis, diabetic neuropathy, nephropathy, diabetic retinopathy, pollakiuria, nocturnal enuresis, hyperlipemia, sexual function disorder, skin disease, arthritis, osteopenia, arteriosclerosis, thrombotic diseases, maldigestion, or memory and learning disabilities.

Further, the agent for modulating the function of an RFRP receptor of the present invention is useful, for example, as a bladder constriction inhibiting agent, and is useful as an agent for preventing or treating urine incontinence, lower urinary tract disease, urge micturition due to excessively active bladder, pollakiuria, or hypotonic bladder accompanied with excessively active bladder.

In particular, the agent for modulating the function of an RFRP receptor of the present invention is useful as an analgesic, or as an agent for preventing or treating memory learning disorder.

When the compound (I) of the present invention is applied to each of the aforementioned diseases, it is possible to conveniently use a drug or a treating method which is conventionally used in those diseases jointly.

Further, when the compound (I) of the present invention is applied to each of the aforementioned diseases, it is also possible to use a biological preparation (e.g. antibody, vaccine preparation), or it is also possible to apply as a joint treating method by combining with genetic therapy.

The compound (I) of the present invention can be orally or parenterally administered as it is, or by blending a pharmacologically acceptable carrier.

Examples of a dosage form of the agent for modulating the function of an RFRP receptor of the present invention, when orally administered, include tablets (including sugar-coated tablets, film-coated tablets), pills, granules, powders, capsules (including soft capsules, microcapsules), syrups, emulsions, and suspensions, and examples of a dosage form when administered parenterally include injectables, infusions, drops, and suppositories. In addition, it is effective to formulate into sustained-release preparations by combining with a suitable base (e.g. a copolymer of butyric acid, a polymer of glycolic acid, a copolymer of butyric acid-glycolic acid, a mixture of a polymer of butyric acid and a polymer of glycolic acid, polyglycerol fatty acid ester etc.).

A content of the compound (I) of the present invention in the preparation of the present invention varies depending on the form of a preparation, and is usually about 0.01 to 100% by weight, preferably about 2 to 85% by weight, further preferably about 5 to 70% by weight relative to a whole preparation.

As a method of preparing the compound (I) of the present invention into the aforementioned dosage form, a known preparation method which is generally used in the art can be applied. In addition, when prepared into the aforementioned dosage form, the dosage form can be prepared, if necessary, by conveniently blending an appropriate amount of excipients, binders, disintegrating agents, lubricants, sweeteners, surfactants, suspending agents or emulsifying agents which are conventionally used in the pharmaceutical art when prepared into the dosage form.

For example, when the compound (I) of the present invention is prepared into tablets, they can be prepared so that they contain excipients, binders, disintegrating agents, lubricants or the like and, when prepared into pills or granules, they can be prepared so that they contain excipients, binders, disintegrating agents or the like. In addition, when prepared into powders or capsules, they can be prepared so that they contain excipients and, when prepared into syrups, they can be prepared so that they contain sweeteners and, when prepared into emulsions or suspensions, they can be prepared so that they contain suspending agents, surfactants, emulsifying agents or the like.

Examples of excipients include lactose, white sugar, glucose, starch, sucrose, microcrystalline cellulose, powdered glycyrrhiza, mannitol, sodium bicarbonate, calcium phosphate, and calcium sulfate.

Examples of binders include 5 to 10 weight% starch paste, 10 to 20 weight% gum arabic solution or gelatin solution, 1 to 5 weight% tragacanth solution, carboxymethylcellulose solution, sodium alginate solution, and glycerin.

Examples of disintegrating agents include starch, and calcium carbonate.

Examples of lubricants include magnesium stearate, stearic acid, calcium stearate, and purified talc.

Examples of sweeteners include glucose, fructose, inverted sugar, sorbitol, xylitol, glycerin, and simple syrup.

Examples of surfactants include sodium laurylsulfate Polysorbate 80, sorbitan monofatty acid ester, and polyoxyl stearate 40.

Examples of suspending agents include gum arabic, sodium alginate, sodium carboxymethylcellulose, methylcellulose, and bentonite.

Examples of emulsifying agents include gum arabic, tragacanth, gelatin, and Polysorbate 80.

Further, when the compound (I) of the present invention is prepared into the aforementioned dosage form, optionally, an appropriate amount of colorants, preservatives, flavors, corrigents, stabilizers, and thickeners which are conventionally used in the purification art can be added.

The agent for modulating the function of an RFRP receptor of the present invention is safe, and has low toxicity, therefore, it can be safely used. The dose per day varies depending on the state and weight of a patient, kind of compound, and administration route. For example, when orally administered to a patient for the purpose of analgesic effect, the dose per adult (weight about 60 kg a day) is, as expressed as an effective ingredient (present compound (I)), about 1 to 1000 mg, preferably about 3 to 300 mg, further preferably about 10 to 200 mg, and the dose can be administered once, or by dividing into two to three times.

When the compound (I) of the present invention is parenterally administered, usually, it is administered in the form of a liquid (e.g. injectables). A single dose thereof varies depending on administration subject, subject organ, symptom, and administration method. For example, in the form of injectables, it is advantageous to administer, by intravenous injection, usually about 0.01 to about 100 mg, preferably about 0.01 to about 50 mg, more preferably about 0.01 to about 20 mg per weight 1 kg. Examples of injectables include, in addition to intravenous injectables, subcutaneous injectables, intradermal injectables, intramuscular injectables, and drop injectables, and examples of long acting preparations include iontophoresis transdermal agents. Such the injectables are prepared by the known per se method, that is, by dissolving, suspending or emulsifying the compound (I) of the present invention in an aqueous solution or an oily solution. Examples of the aqueous solution for injection include isotonics (e.g. D-sorbitol, D-mannitol, sodium chloride etc.) containing a physiological saline, glucose and other additives, and the aqueous solution may be used together with appropriate dissolution aides such as alcohol (e.g. ethanol), polyalcohol (e.g. propylene glycol, polyethylene glycol), or nonionic surfactant (e.g. Polysorbate 80, HCO-50). Examples of the oily solution include sesame oil, and soybean oil, and it may be used together with benzyl benzoate, or benzyl alcohol as a dissolution aide. In addition, buffers (e.g. phosphate buffer, sodium acetate buffer), soothing agents (e.g. benzalkonium chloride, procaine hydrochloride etc.), stabilizers (e.g. human serum albumin, polyethylene glycol etc.), and preservatives (e.g. benzyl alcohol, phenol etc.) may be blended. Prepared injectables are usually filled into an ampoule.

Examples of a drug which can be used together with the compound (I) of the present invention (hereinafter, abbreviated as joint use drug in some cases) include another diabetes treating agent, a diabetic complication treating agent, a hyperlipemia treating agent, a hypotensive agent, an anti-obesity agent, a diuretic, a chemical therapy agent, an immunological therapy agent, an immunological regulating drug, an anti-inflammatory, an anti-thrombus agent, an osteoporosis treating agent, an antibacterial agent, an anti-fungus agent, an anti-protozoan agent, an antibiotic, an antitussive/expectorant, a sedative, an anesthetic, an anti-ulcer drug, a tranquilizer, an anti-psychosis drug, an anti-tumor drug, a muscular relaxant, an anti-epilepsy drug, an antidepressant, an anti-allergy drug, a cardiotonic, an anti-arrhythmia drug, a vasodilator, a vasoconstrictor, a narcotic antagonist, a vitamin drug, a vitamin derivative, an anti-asthma drug, an anti-dementia drug, pollakiuria/urine incontinence treating drug, a dysuria treating drug, an atopic dermatitis treating drug, an allergic rhinitis treating drug, a vasopressor, an endotoxin antagonist or antibiotic, a signal transmission inhibiting drug, an inflammatory mediator action inhibiting drug, an inflammatory mediator action inhibiting antibody, an anti-inflammatory mediator action inhibiting drug, and an anti-inflammatory mediator action inhibiting antibody. Specific examples are as follows:

Examples of other diabetes treating agents include an insulin preparation (e.g. an animal insulin preparation extracted from cow or pig pancreas; a human insulin preparation synthesized by genetic engineering using *Escherichia coli* or yeast; zinc insulin, zinc protamineinsulin; a fragment or a derivative of insulin (e.g. INS-1 etc., an oral insulin preparation etc.), an insulin sensitivity potentiating agent (e.g. pioglitazone or a salt thereof (preferably hydrochloride), troglitazone, rosiglitazone or a salt thereof (preferably maleate), Reglixane (JTT-501), Netoglitazone (MCC-555), YM-440, GI-262570, KRP-297, FK-614, CS-011, (γE)-γ-[[[4-[(5-methyl-2-phenyl-4-oxazolyl)methoxy]phenyl]methoxy]imino]benzenebutanoic acid, a compound described in WO 99/58510 (e.g. (E)-4-[4-(5-methyl-2-phenyl-4-oxazolylmethoxy)benzyloxyimino]-4-phenylbutyric acid, a compound described in WO 01/38325, Tesaglitazar (AZ-242), Ragaglitazar (NN-622), BMS-298585, ONO-5816, BM-13-1258, LM-4156, MBX-102, LY-519818, MX-6054, LY-510929, Balaglitazone (NN-2344), T-131 or a salt thereof, THR-0921), an α-glucosidase inhibitor (e.g. voglibose, acarbose, miglitole, emiglitate etc.), a biguanide agent (e.g. phenformin, metformin, buformin etc.), an insulin secretion promoting agent [sulfonylurea agent (e.g. tolbutamide, glibenclamide, gliclazid, chlorpropamide, tolazamide, acetohexamide, glyclopyramide, glimepiride etc.), repaglinide, senaglinide, mitiglinide or a calcium salt hydrate thereof, nateglinide etc.], a GLP-1 receptor agonist [e.g. GLP-1, GLP-1MR agent, NN-2211, AC-2993 (exendin-4), BIM-51077, Aib (8,35)hGLP-1(7,37)NH₂, CJC-1131 etc.], dipeptidylpeptidase IV inhibitor (e.g. NVP-DPP-278, PT-100, P32/98, P93/01, NVP-DPP-728, LAF237, TS-021 etc.), a β3 agonist (e.g. CL-316243, SR-58611-A, UL-TG-307, AJ-9677, AZ40140 etc.), an aniline agonist (e.g. pramlintide etc.), a phosphotyrosine phosphatase inhibitor (e.g. vanadic acid etc.), a glyconeogenesis inhibitor (e.g. glycogen phosphorylase inhibitor, glucose-6-phosphatase inhibitor, glucagon inhibitor etc.), a SGLT (sodium-glucose cotransporter) inhibitor (e.g. T-1095 etc.), a 11β-hydroxysteroid dehydrogenase inhibitor (e.g. BVT-3498 etc.), adiponectin or an agonist thereof, an IKK inhibitor (e.g. AS-2868 etc.), a leptin resistance improving drug, a somatostatin receptor agonist (a compound described in WO 01/25228, WO 03/42204, a compound described in WO 98/44921, WO 98/45285, WO 99/22735 etc.), and a glucokinase activating drug (e.g. Ro-28-1675).

Examples of the diabetic complication treating agent include an aldose reductase inhibitor (e.g. Torestat, epalrestat, zenarestat, zopolrestat, fidarestat (SNK-860), minalrestat (ARI-509), CT-112 etc.), a neurotrophic factor and a drug for increasing it (e.g. NGF, NT-3, BDNF, a neurotrophin production/secretion promoting agent described in WO 01/14372 (e.g. 4-(4-chlorophenyl)-2-(2-methyl-1-imidazolyl)-5-[3-(2-methylphenoxy)propyl]oxazole etc.) etc.), a protein kinase C (PKC) inhibitor (e.g. LY-333531 etc.), an AGE inhibitor (e.g. ALT-945, pimagedine, piratoxathine, N-phenasylthiazolium bromide (ALT-766), EXO-226, ALT-711, Pyridorin, pyridoxamine etc.), an active oxygen scavenger (e.g. thioctic acid etc.), a brain vasodilator (e.g. tiapride etc.), a somatostatin receptor agonist (BIM23190), a apoptosis signal regulating kinase-1 (ASK-1) inhibitor.

Examples of the hyperlipemia treating agent include a statin compound which is a cholesterol synthesis inhibiting agent (e.g. pravastatin, simvastatin, lovastatin, atorvastatin, fluvastatin, cerivastatin or a salt thereof (e.g. sodium salt etc.) etc.), a squalene synthesizing enzyme inhibitor (e.g. a compound described in WO 97/10224, for example, N-[[(3R,5S)-1-(3-acetoxy-2,2-dimethylpropyl)-7-chloro-5-(2,3-dimethoxyphenyl)-2-oxo-1,2,3,5-tetrahydro-4,1-benzoxazepin-3-yl]acetyl]piperidine-4-acetic acid etc.), a fibrate compound (e.g. bezafibrate, clofibrate, simfibrate, clinofibrate etc.), and an antioxidant (e.g. lipoic acid, producol).

Examples of the hypotensive agent include an angiotensin converting enzyme inhibitor (e.g. captopril, enalapril, derapril etc.), an angiotensin II antagonist (e.g. losartan, candesartan cilexetil, eprosartan, valsartan, telmisartan, irbesartan, tasosartan, 1-[[2'-(2,5-dihydro-5-oxo-4H-1,2,4-oxadiazol-3-yl)biphenyl-4-yl]methyl]-2-ethoxy-1H-benzimidazole-7-carboxylic acid etc.), a calcium antagonist (e.g. manidipine, nifedipine, amlodipine, efonidipine, nicardipine etc.), and clonidine.

Examples of the anti-obesity agent include a neutral anti-obesity drug (e.g. dexfenfluramine, fenfluramine, phentermine, sibutramine, amfepramone, dexamphetamine, mazindol, phenylpropanolamine, clobenzorex; MCH receptor antagonist (e.g. SB-568849; SNAP-7941; a compound included in WO 01/82925 and WO 01/87834 etc.); a neuropeptide Y antagonist (e.g. CP-422935 etc.); a cannabinoid receptor antagonist (e.g. SR-141716, SR-147778 etc.); a ghrelin antagonist; a 11β-hydroxysteroid dehydrogenase inhibitor (e.g. BVT-3498 etc.) etc.), a pancreatic lipase inhibitor (e.g. orlistat, ATL-962 etc.), a β3 agonist (e.g. CL-316243, SR-58611-A, UL-TG-307, AJ-9677, AZ40140 etc.), a peptidic appetite inhibitor (e.g. leptin, CNTF (CORPUS ciliare neurotrophic factor) etc.), a cholecystokinin agonist (e.g. rinchitript, FPL-15849 etc.), and a eating inhibitor (e.g. P-57 etc.).

Examples of the diuretic include a xanthine derivative (e.g. sodium salicylate theobromine, calcium salicylate theobromine etc.), a thiazide preparation (e.g. ethiazide, cyclopentiazide, trichlormethiazide, hydrochlorothiazide, hydroflumethiazide, benzylhydrochlorothiazide, penflutizide, polythiazide, methyclothiazide etc.), an anti-aldosterone preparation (e.g. spironolactone, triamteren etc.), a decarboxylase inhibitor (e.g. acetazolamide etc.), a chlorobenzenesulfonamide preparation (e.g. chlortalidone, mefruside, indapamide etc.), azosemide, isosorbide, ethacrynic acid, piretanide, bumetanide, and furosemide.

Examples of the chemical therapy agent include an alkylating agent (e.g. cyclophosphamide, ifosfamide etc.), a metabolism antagonist (e.g. methotrexate, 5-fluorouracil etc.), an anti-cancer antibiotic (e.g. mitomycin, adriamycin etc.), a plant-derived anti-cancer agent (e.g. vincristine, vindesin, taxol etc.), cisplatin, carboplatin, and etoposide. Inter alia, Furtulon or NeoFurtulon which is a 5-fluorouracil derivative is preferable.

Examples of the immunological therapy agent include a microorganism or bacterium component (e.g. muramyl dipeptide derivative, Picibanil etc.), a polysaccharide having immunity potentiating activity (e.g. lentinan, sizofiran, Krestin etc.), cytokine obtained by a genetic engineering method (e.g. interferon, interleukin (IL) etc.), and a colony stimulating factor (e.g. granulocyte colony stimulating factor, erythropoietin etc.) and, inter alia, interleukins such as IL-1, IL-2, and IL-12 are preferable.

Examples of the anti-inflammatory include non-steroidal anti-inflammatory such as aspirin, acetoaminophen, and indometacin.

Examples of the anti-thrombus agent include heparin (e.g. heparin sodium, heparin calcium, dalteparin sodium etc.), warfarin (warfarin potassium etc.), an anti-thrombin drug (e.g. aragatroban etc.), a thrombolytic drug (e.g. urokinase, tisokinase, alteplase, nateplase, monteplase, pamiteplase etc.), a platelet aggregation inhibitor (ticlopidine hydrochloride, cilostazol, ethyl eicosapentoate, beraprost sodium, sarpogrelate hydrochloride etc.).

Examples of the osteoporosis treating agent include alfacalcidol, calcitriol, elcatonin, calcitonin salmon, estriol, ipriflavone, pamidronate disodium, alendronate sodium hydrate, and incadronate disodium.

Examples of the vitamin drug include vitamin B₁, and vitamin B₁₂.

Examples of the anti-dementia agent include tacrine, donepezil, rivastigmine, and galantamine.

Examples of the pollakiuria•urine incontinence treating drug include flavoxate hydrochloride, oxybutynin hydrochloride, and propiverine hydrochloride.

Examples of the dysuria treating drug include an acetylcholine esterase inhibitor (e.g. distigmine).

Further, drugs which are recognized to have cachexia improving activity in an animal model or a clinical test, that is, a cyclooxygenase inhibitor (e.g. indometacin etc.) [Cancer Research, vol. 49, p5935-5939, 1989] a progesterone derivative (e.g. megestrol acetate) [Journal of Clinical Oncology, vol. 12, p213-225, 1994], a glycosteroid (e.g. dexamethasone etc.), a metochropramide drug, tetrahydrocannabinol drug (the references are the same as those described above), a lipid metabolism improving agent (e.g. eicosapentaenoic acid etc.) [British Journal of Cancer, vol. 68, p314-318, 1993], a growth hormone, IGF-1, or TNF-α which is a factor inducing cachexia, LIF, IL-6, and an antibody to oncostatin M can be used together with the compound (I) of the present invention.

Further, a glycosylation inhibitor (e.g. ALT-711 etc.), a nerve regeneration stimulator (e.g. Y-128, VX853, prosaptide etc.), an antidepressant (e.g. desipramine, amitriptyline, imipramin), an anti-epilepsy drug (e.g. lamotrigine, Trileptal, Keppra, Zonegran, Pregabalin, Harkoseride, carbamazepine), an anti-arrhythmia drug (e.g. mexiletine), an acetylcholine receptor ligand (e.g. ABT-594), an endothelin receptor antagonist (e.g. ABT-627), a monoamine uptake inhibitor (e.g. tramadol), a morphine analgesic (e.g. morphine), a GABA receptor agonist (e.g. gabapentin, gabapentin MR agent), an α2 receptor agonist (e.g. clonidine), a local analgesic (e.g. capsaicin), an anti-anxiety drug (e.g. benzothiazepine), a phosphodiesterase inhibitor (e.g. sindenafil), and a dopamine receptor agonist (e.g. apomorphine) can be also used together with the compound (I) of the present invention.

By combining the compound (I) of the present invention and the joint use drug, the following excellent effects can be obtained:
(1) A dose thereof can be decreased as compared with the case where the compound (I) of the present invention or the joint use drug is administered alone.
(2) A drug to be used with the compound (I) of the present invention jointly can be selected depending on symptom of a patient (slight, severe).
(3) By selecting the joint use drug having different mechanism of action from that of the compound (I) of the present invention, a treating period can be set long.
(4) By selecting the joint use drug having different mechanism of action from that of the compound (I) of the present invention, durability of the treating effect can be realized.
(5) By using the compound (I) of the present invention and the joint use drug jointly, the synergistic effect is obtained

Hereinafter, joint use of the compound (I) of the present invention and the joint use drug will be referred to as "joint use agent of the present invention".

Upon use of the joint use agent of the present invention, an administration time of the compound (I) of the present invention and the joint use drug is not limited, but the compound (I) of the present invention and the joint use drug may be administered to an administration subject simultaneously, or at different times. A dose of the joint use drug may be according to a dose which is clinically used, and can be appropriately selected depending on administration subject, administration route, disease, and combination.

An administration form of the joint use agent of the present invention is not particularly limited as long as the compound (I) of the present invention and the joint use drug are combined at administration. Examples of such administration form include (1) simultaneous formulation of the compound (I) of the present invention and the joint use drug into a preparation, and administration of the resulting single preparation, (2) simultaneous administration of two kinds of preparations obtained by separately preparing the compound (I) of the present invention and the joint use drug into a preparation through the same administration route, (3) administration of two kinds of preparations obtained by separately formulating the compound (I) of the present invention and the joint use drug into a preparation through the same administration route at different times, (4) simultaneous administration of two kinds of preparations obtained by separately formulating the compound (I) of the present invention and the joint use drug through different administration routes, and (5) administration of two kinds of preparations obtained by separately formulating the compound (I) of the present invention and the joint use drug through different administration routes at different times (e.g. administration in an order of the compound (I) of the present invention; the joint use drug, or administration in a reverse order).

The joint use agent of the present invention has low toxicity and, for example, the compound (I) of the present invention or (and) the joint use drug are mixed with a pharmacologically acceptable carrier according to the known per se method to prepare a pharmaceutical composition such as tablets (including sugar-coated tablets, film-coated tablets), powders, granules, capsules (including soft capsules), liquids, injectables, suppositories and sustained-release preparations, which can be safely administered orally or parenterally (e.g. local, rectal, intravenous administration etc.). An injectable can be administered intravenously, intramuscularly, or subcutaneously, or can be administered into organs, or can be directly administered to a lesion.

Examples of a pharmacologically acceptable carrier which may be used in preparing the joint use agent of the present invention include the same carriers as those for the aforementioned pharmacologically acceptable carrier which may be used in preparing a drug of the present invention. In addition, if necessary, additives such as antiseptics, antioxidants, colorants, sweeteners, adsorbing agents, and wetting agents which may be used in preparing a drug of the present invention can be appropriately used at an appropriate amount.

A ratio of blending the compound (I) of the present invention and the joint use drug in the joint use agent of the present invention can be appropriately selected depending on an administration subject, an administration route, and a disease.

For example, a content of the compound (I) of the present invention in the joint use agent of the present invention is different depending on a form of a preparation, and is usually about 0.01 to 100% by weight, preferably about 0.1 to 50% by weight, further preferably about 0.5 to 20% by weight relative to a whole preparation.

A content of the joint use drug in the joint use agent of the present invention is different depending on a form of a preparation and is usually about 0.01 to 90% by weight, preferably about 0.1 to 50% by weight, further preferably about 0.5 to 20% by weight relative to a whole preparation.

A content of an additive such as a carrier in the joint use agent of the present invention varies depending on a form of a preparation, and is usually about 1 to 99.99% by weight, preferably about 10 to 90% by weight relative to a whole preparation.

In addition, when the compound (I) of the present invention and the joint use drug are separately formulated into a preparation, the same content may be used.

These preparations can be usually prepared by the known per se method which is generally used in a pharmacy step.

For example, the compound (I) of the present invention or the joint use drug together with dispersants (e.g. Tween 80 (manufactured by Atlas Powder, USA), HCO 60 (manufactured by Nikko Chemicals Co., Ltd.), polyethylene glycol, caboxymethylcellulose, sodium alginate, hydroxypropylmethylcellulose, dextrin etc.), stabilizers (e.g. ascorbic acid, sodium pyrosulfite etc.), surfactants (e.g. Polysorbate 80, macrogol etc.), solubilizers (e.g. glycerin, ethanol etc.), buffers (e.g. phosphoric acid and an alkali metal salt thereof, citric acid and an alkali metal salt thereof etc.), isotonics (e.g. sodium chloride, potassium chloride, mannitol, sorbitol, glucose etc.), pH adjusting agents (e.g. hydrochloric acid, sodium hydroxide etc.), preservatives (e.g. ethyl paraoxybenzoate, benzoic acid, methyl paraben, propyl paraben, benzyl alcohol etc.), dissolving agents (e.g. concentrated glycerin, meglumine etc.), dissolution aides (e.g. propylene glycol, white sugar etc.), and soothing agents (e.g. glucose, benzyl alcohol etc.) is prepared into an aqueous injectable, or is dissolved, suspended or emulsified in a vegetable oil such as an olive oil, a sesame oil, a cottonseed oil, and a corn oil, or in a dissolution aide such as propylene glycol to prepare an oily injectable, which may be used as an injectable.

Alternatively, according to a per se known method, by adding, for example, an excipient (e.g. lactose, white sugar, starch etc.), a disintegrating agent (e.g. starch, calcium carbonate etc.), a binder (e.g. starch, gum arabic, carboxymethylcellulose, polyvinylpyrrolidone, hydroxypropylcellulose etc.) or a lubricant (e.g. talc, magnesium stearate, polyethylene glycol 6000) to the compound (I) of the present invention or the joint use drug to compression-mold them and, if necessary, performing coating by the known per se method for the purpose of taste masking, enteric solubility or durability, or an oral preparation can be obtained. As a coating agent used in coating, for example, hydroxypropylmethylcellulose, ethylcellulose, hydroxymethylcellulose, hydroxypropylcellulose, polyoxyethylene glycol, Tween 80, Pluronic F68, cellulose acetate phthalate, hydroxypropylmethylcellulose phthalate, hydroxymethylcellulose acetate succinate, Eudragit (manufactured by Rohm, Germany, a methacrylic acid•acrylic acid copolymer) and a pigment (e.g. bengal, titanium dioxide etc.) are used. An oral preparation may be any of a rapid-releasing preparation and a sustained-release preparation.

Further, according to a per se known method, by mixing the compound (I) of the present invention or the joint use drug with an oily base, an aqueous base or an aqueous gel base, an oily or aqueous solid, semisolid or liquid suppository can be obtained. Examples of the oily base include glyceride of higher fatty acid [e.g. cacao butter, witepsol (manufactured by Dynamite Novel, Germany)], middle fatty acid [e.g. miglyol (manufactured by Dynamite Novel, Germany) etc.], and a vegetable oil (e.g. sesame oil, soybean oil, cottonseed oil etc.). Examples of the aqueous base include polyethylene glycol, and propylene glycol. Examples of the aqueous gel base include natural gums, a cellulose derivative, a vinyl polymer, and an acrylic acid polymer.

Examples of the sustained-release preparation include sustained-release microcapsules. The sustained-release microcapsule is prepared by the known per se method such as the method shown in the following [2].

It is preferable that the compound (I) of the present invention is molded into an oral preparation such as a solid preparation (e.g. powders, granules, tablets, capsules), or is molded into a rectal preparation such as suppositories. An oral preparation is particularly preferable.

The joint use drug can be formulated into the aforementioned dosage form depending on a kind of a drug.
[1] Injectables of the compound (I) of the present invention or the joint use drug and preparation thereof, [2] sustained-release preparations or rapid-releasing preparations of the compound (I) of the present invention or the joint use drug, and preparation thereof, and [3] sublingual tablets, buccal or oral rapid disintegrating agents of the compound (I) of the present invention or the joint use drug, and preparation thereof will be specifically shown below.

### [1] Injectables and preparation thereof

An injectable in which the compound (I) of the present invention or the joint use drug is dissolved in water is preferable. The injectable may contain benzoate or/and salicylate.

The injectable is obtained by dissolving both of the compound (I) of the present invention or the joint drug and, optionally, benzoate or/and salicylate in water.

Examples of a salt of benzoic acid and salicylic acid include an alkali metal salt such as sodium, and potassium, an alkaline earth metal salt such as calcium, and magnesium, an ammonium salt, a meglumine salt, and an organic acid salt such as trometamol.

A concentration of the compound (I) of the present invention or the joint use drug in an injectable is about 0.5 to 50 w/v%, preferably about 3 to 20 w/v%. A concentration of benzoate or/and salicylate is about 0.5 to 50 w/v%, preferably about 3 to 20 w/v%.

In addition, an additive which is generally used in injectables, such as a stabilizing agent (e.g. ascorbic acid, sodium pyrosulfite etc.), a surfactant (e.g. Polysorbate 80, macrogol etc.), a solubilizer (e.g. glycerin, ethanol etc.), a buffer (e.g. phosphoric acid and an alkali metal salt thereof, citric acid and an alkali metal salt thereof), tonicity agent (e.g. sodium chloride, potassium chloride etc.), a dispersant (e.g. hydroxypropylmethylcellulose, dextrin), a pH adjusting agent (e.g. hydrochloric acid, sodium hydroxide etc.), a preservative (e.g. ethyl paraoxybenzoate, benzoic acid etc.), a dissolving agent (e.g. concentrated glycerin, meglumine etc.), a dissolution aide (e.g. propylene glycol, white sugar etc.), and a soothing agent (e.g. glucose, benzyl alcohol etc.) can be appropriately blended into the present injectable. These additives are generally blended at a ratio which is usually used in injectables.

It is suitable that injectables are adjusted to a pH of 2 to 12, preferably a pH of 2.5 to 8.0 by adding a pH adjusting agent.

Injectables are obtained by dissolving both of the compound (I) of the present invention or the joint use drug and, optionally, benzoate or/and salicylate and, if necessary, the aforementioned additive in water. Dissolution of them may be performed in any order, and can be appropriately performed as in the previous process for preparing injectables.

Suitably, an aqueous solution for injection is warmed, and can be supplied as an injectable by filtration sterilization or high pressure heating sterilization as in the conventional injectable.

Suitably, an aqueous solution for injectable is subjected to high pressure heating sterilization for 5 to 30 minutes, for example, under conditions of 100 to 121°C.

Further, an injectable may be formulated into a preparation to which antibacterial property of a solution is imparted so that it can be used as a multiple divided administration preparation.

### [2] Sustained-release preparations or rapid-releasing preparations and preparation thereof

Sustained-release preparations in which a core containing the compound (I) of the present invention or the joint use drug is optionally covered with a covering agent such as a water-insoluble substance or a wetting polymer are preferable. For example, once a day administration-type oral sustained-release preparations are preferable.

Examples of the water-insoluble substance used in a covering agent include cellulose ethers such as ethylcellulose, and butylcellulose, cellulose esters such as cellulose acetate, and cellulose propionate, polyvinyl esters such as polyvinyl acetate, and polyvinyl butyrate, acrylic acid-based polymer such as an acrylic acid/methacrylic acid copolymer, a methyl methacrylate copolymer, an ethoxyethyl methacrylate/cinnamoethyl methacrylate/aminoalkyl methacrylate copolymer, polyacrylic acid, polymethacrylic acid, a methacrylic acid alkylamide copolymer, poly(methyl methacrylate), polymethacrylate, polymethacrylamide, an aminoalkyl methacrylate copolymer, poly(methacrylic acid anhydride), a glycidyl methacrylate copolymer, inter alia, Eudragit (Rohm•Firma) such as Eudragit RS-100, RL-100, RS-30D, RL-30D, RL-PO, RS-PO (an ethyl acrylate•methyl methacrylate•trimethyl methacrylate chloride•ethyl ammonium copolymer), and Eudragit NE-30D (a methyl methacrylate•ethyl acrylate copolymer), a hydrogenated oil such as hydrogenated castor oil (e.g. Lovely wax (Freund Industry) etc.), waxes such as carnauba wax, fatty acid glycerin ester, and paraffin, and polyglycerin fatty acid ester.

As a wettable polymer, a polymer having an acidic dissociating group and exhibiting pH dependent swelling is preferable, and a polymer having an acidic dissociating group, which is slightly swollen in an acidic region such as in stomach, and in which swelling becomes great in a neutral region such as in small intestine and large intestine is preferable.

Examples of such the polymer having an acidic dissociating group and exhibiting pH dependent swelling include crosslinking-type polyacrylic acid polymer such as Carbomer 934P, 940, 941, 974P, 980, 1342 etc., polycarbophil, and calcium polycarbophil (all manufactured by BF Goodrich), and Hiviswako 103, 104, 105, and 304 (all manufactured by Wako Pure Chemical Industries, Ltd.).

A covering agent used in sustained-release preparations may further contain a hydrophilic substance.

Examples of the hydrophilic substance include polysaccharides optionally having a sulfate group such as pullulan, dextrin, alginic acid alkali metal salt, polysaccharides having a hydroxyalkyl group or a carboxyalkyl group such as hydroxypropylcellulose, hydroxypropylmethylcellulose, and sodium carboxymethylcellulose, methylcellulose, polyvinylpyrrolidone, polyvinyl alcohol, and polyethylene glycol.

The content of the water-insoluble substance in a covering agent of sustained-release preparations is about 30 to about 90% (w/w), preferably about 35 to about 80% (w/w), further preferably about 40 to 75% (w/w), and a content of the wettable polymer is about 3 to about 30% (w/w), preferably about 3 to about 15% (w/w). A covering agent may further contain a hydrophilic substance and, in that case, a content of the hydrophilic substance in a covering agent is about 50% (w/w) or lower, preferably about 5 to about 40% (w/w), further preferably about 5 to about 35% (w/w). Herein, the % (w/w) indicates weight% relative to a covering agent composition obtained by removing a solvent (e.g. water, lower alcohol such as methanol, ethanol etc.) from a covering agent solution.

A sustained-release preparation is manufactured by preparing a core containing a drug and, then, covering a core with a covering agent solution obtained by heating-dissolving a water-insoluble substance or a wettable polymer, or dissolving or dispersing in a solvent, as exemplified below.

### I. Preparation of core containing drug

A form of a core containing a drug to be covered with a covering agent (hereinafter, simply referred to as core in some cases) is not particularly limited, but a core is formed into a particulate shape such as a granule or a fine particle.

When a core is a granule or a fine particle, an average particle diameter thereof is preferably about 150 to 2,000 µm, further preferably about 500 to about 1,400 µm.

Preparation of a core can be performed by a conventional manufacturing method. For example, a core is prepared by mixing a drug with an appropriate excipient, binder, disintegrating agent, lubricant, and stabilizing agent, and forming a particle by a wet extrusion granulating method or a fluidized layer granulating method.

A drug content of a core is about 0.5 to about 95% (w/w), preferably about 5.0 to about 80% (w/w), further preferably about 30 to about 70% (w/w).

As an excipient contained in a core, sugars such as white sugar, lactose, mannitol, and glucose, starch, crystalline cellulose, calcium phosphate, and corn starch are used. Inter alia, crystalline cellulose, and corn starch are preferable.

As the binder, for example, polyvinyl alcohol, hydroxypropylcellulose, polyethylene glycol, polyvinylpyrrolidone, Pluronic F68, gum arabic, gelatin, and starch are used. As the disintegrating agent, for example, calcium carboxymethylcellulose (ECG505), sodium croscarmelose (Ac-Di-Sol), crosslinking-type polyvinylpyrrolidone (crospovidone), and low-substituted hydroxypropylcellulose (L-HPC) are used. Inter alia, hydroxypropylcellulose, polyvinylpyrrolidone, and low-substituted hydroxypropylcellulose are preferable. As the lubricant, or the aggregation preventing agent, for example, talc, and magnesium stearate and an inorganic salt thereof are used and, as the lubricating agent, polyethylene glycol is used. As the stabilizing agent, an acid such as citric acid, succinic acid, fumaric acid, and maleic acid is used.

A core may be prepared by a rolling granulating method, a pan coating method, a fluidized layer coating method or a melt granulating method in which a small amount of a drug or a mixture thereof and an excipient or a lubricant is added while a binder dissolved in a suitable solvent such as water, and a lower alcohol (e.g. methanol, ethanol etc.) is sprayed on an inert carrier particle as a center of a core, in addition to the aforementioned manufacturing method. As the inert carrier particle, a carrier prepared with white sugar, lactose, starch, crystalline cellulose, or waxes can be used, and a particle having an average particle diameter of about 100 µm to about 1,500 µm is preferable.

In order to separate a drug contained in a core, and a covering agent, a surface of a core may be covered with a protecting agent. As the protecting agent, for example, the aforementioned hydrophilic substance or water-insoluble substance is used. Preferably, as the protecting agent, polyethylene glycol, a polysaccharide having a hydroxyalkyl group or a carboxyalkyl group is used. More preferably, hydroxypropylmethylcellulose, and hydroxypropylcellulose are used. The protecting agent may contain an acid such as tartaric acid, citric acid, succinic acid, fumaric acid, and maleic acid as a stabilizing agent, or a lubricant such as talc. When the protecting agent is used, a covering amount thereof is about 1 to about 15% (w/w), preferably about 1 to about 10% (w/w), further preferably about 2 to about 8% (w/w) relative to a core.

The protecting agent can be covered by a conventional coating method and, specifically, can be covered by spray-coating the protecting agent on a core, for example, by a fluidized layer coating method or a pan coating method.

### II. Covering of core with covering agent

Sustained-release preparations are prepared by covering a core obtained in the aforementioned I with a covering solution obtained by heating-dissolving the aforementioned water-insoluble substance and pH dependent wettable polymer, and hydrophilic substance, or dissolving or dispersing them in a solvent.

Examples of a method of covering a core with a covering solution include a spray coating method.

A compositional ratio of a water-insoluble substance, a wettable polymer or a hydrophilic substance in a covering agent solution is appropriately selected so that contents of respective components in a cover become the aforementioned contents, respectively.

A covering amount of a covering agent is about 1 to about 90% (w/w), preferably about 5 to about 50% (w/w), further preferably about 5 to 35% (w/w) relative to a core (not containing a covering amount of a protecting agent).

As a solvent for a covering agent solution, water or an organic solvent alone, or a mixed solution of both of them may be used. Upon use of a mixed solution, a ratio of mixing water and an organic solvent (water/organic solvent: weight ratio) can be changed in a range of 1 to 100%, preferably 1 to about 30%. The organic solvent is not particularly limited as far as it dissolves a water-insoluble substance, but for example, lower alcohol such as methyl alcohol, ethyl alcohol, isopropyl alcohol, and n-butyl alcohol, lower alkanone such as acetone, acetonitrile, chloroform, and methylene chloride are used. Among them, a lower alcohol is preferable, and ethyl alcohol, and isopropyl alcohol are particularly preferable. Water, and a mixed solution of water and an organic solvent are preferably used as a solvent for a covering agent. Thereupon, if necessary, in order to stabilize a covering agent solution, an acid such as tartaric acid, citric acid, succinic acid, fumaric acid, and maleic acid may be added to the covering agent solution.

Operation when covered by spray coating can be performed by a conventional coating method and, specifically, can be performed by spray coating a covering solution on a core, for example, by a fluidized layer coating method, or a pan coating method. Thereupon, if necessary, talc, titanium oxide, magnesium stearate, calcium stearate, and light silicic acid anhydride as a lubricant, and glycerin fatty acid ester, hydrogenated castor oil, triethyl citrate, cetyl alcohol, and stearyl alcohol as a plasticizer may be added.

After covering with a covering agent, if necessary, an antistatic agent such as talc may be mixed therein.

A rapid-releasing preparation may be liquid (solutions, suspensions, emulsions etc.) or solid (particles, pills, tablets etc.). As a rapid-releasing preparation, an oral administration agent, and a parenteral administration agent such as injectable are used, and an oral preparation agent is preferable.

A rapid-releasing preparation may usually contain a carrier, an additive and an excipient (hereinafter, abbreviated as excipient in some cases) which are conventional in the pharmacy field, in addition to a drug which is an active ingredient. An excipient agent used is not particularly limited as long as it is an excipient which is conventionally used as a preparation excipient. Examples of an excipient for an oral solid preparation include lactose, starch, corn starch, crystalline cellulose (Avicel PH101 manufactured by Asahi Chemical Industry Co., Ltd.), powdered sugar, granulated sugar, mannitol, light silicic anhydride, magnesium carbonate, calcium carbonate, and L-cysteine, preferably corn starch and mannitol. These excipients can be used alone, or by combining two or more kinds of them. The content of an excipient is, for example, about 4.5 to about 99.4 w/w%, preferably about 20 to about 98.5 w/w%, further preferably about 30 to about 97 w/w% relative to the total amount of rapid-releasing preparation.

The content of a drug in a rapid-releasing preparation can be appropriately selected from a range of about 0.5 to about 95%, preferably about 1 to about 60% relative to the total amount of rapid-releasing preparation.

When a rapid-releasing preparation is an oral solid preparation, it usually contains a disintegrating agent in addition to the aforementioned components. As such the disintegrating agent, for example, calcium carboxymethylcellulose (ECG-505 manufactured by Gotokuyakuhin), sodium croscarmelose (e.g. Acsisol manufactured by Asahi Chemical Industry Co., Ltd.), crospovidone (e.g. Colidone CL manufactured by BASF), low-substituted hydroxypropylcellulose (manufactured by Shin-Etsu Chemical Co., Ltd.), carboxymethylstarch (manufactured by Matsutani Chemical Industry Co., Ltd.), sodium carboxymethylstarch (Extract Protub manufactured by Kimura Sangyo), and partially gelatinized starch (PCS manufactured by Asahi Chemical Industry Co., Ltd.) are used. For example, a disintegrating agent which disintegrates a granule by contacting with water to absorb water or be swollen, or making a channel between an active ingredient and an excipient constituting a core, can be used. These disintegrating agents can be used alone, or by combining two or more kinds of them. An amount of a disintegrating agent to be blended is appropriately selected depending on a kind and a blending amount of a drug to be used, and preparation design of releasability, and is, for example, about 0.05 to about 30 w/w%, preferably about 0.5 to about 15 w/w% relative to a total amount of a rapid-releasing preparation.

When a rapid-releasing preparation is an oral solid preparation, the preparation may further contain, optionally, an additive which is conventional in a solid preparation, in addition to the aforementioned composition. As such the additive, for example, a binder (e.g. sucrose, gelatin, gum arabic powder, methyl cellulose, hydroxypropylcellulose, hdyroxypropylmethylcellulose, carboxymethylcellulose, polyvinylpyrrolidone, pullulan, dextrin etc.), a lubricant (e.g. polyethylene glycol, magnesium stearate, talc, light silicic anhydride (e.g. Aerosil (Nippon Aerosil)), a surfactant (e.g. anionic surfactant such as sodium alkylsulfate, nonionic surfactant such as polyoxyethylene fatty acid ester and polyoxyethylene sorbitan fatty acid ester, polyoxyethylene castor oil derivative etc.), a colorant (e.g. tar-based pigment, caramel, bengal, titanium oxide, riboflavins) and, if necessary, a corrigent (e.g. sweetener, flavor etc.), an adsorbing agent, an antiseptic, a wetting agent, and an antistatic agent are used. In addition, an organic acid such as tartaric acid, citric acid, succinic acid, and fumaric acid as a stabilizing agent may be added.

As the binder, hydroxypropylcellulose, polyethylene glycol and polyvinylpyrrolidone are preferably used.

A rapid-releasing preparation can be prepared by mixing the aforementioned respective components and, if necessary, further kneading the mixture, and molding this, based on the conventional technique for manufacturing a preparation. The mixing is performed by a generally used method, for example, by mixing and kneading. Specifically, for example, when a rapid-releasing preparation is formed into a particle shape, by the same procedure as the aforementioned process for preparing a core of the sustained-release preparation, the rapid-releasing preparation can be prepared by mixing components using a vertical granulator, a universal kneader (manufactured by Hatatekkosho), or a fluidized layer granulator FD-5S (manufactured by Powlex) and, thereafter, performing granulation by a wet extrusion granulating method or a fluidized layer granulating method.

The thus obtained rapid-releasing preparation and sustained-release preparation as they are, or after convenient separate formulation into a preparation with a pharmacy excipient by a conventional method, may be formulated into preparations which are administered simultaneously, or which are administered at an arbitrary interval by combination, or both may be used as they are, or both may be conveniently formulated into one oral preparation (e.g. granules, fine particles, tablets, capsules etc.) with a pharmacy excipient. Both preparations may be made into granules or fine particles, and they may be filled into the same capsule to obtain an oral preparation.

### [3] Sublingual tablets, buccals or oral rapid disintegrating preparations and preparation thereof

Sublingual tablets, buccal preparations, and oral rapid disintegrating preparations may be a solid preparation such as tablets, or may be oral mucosal applying tablets (films).

As the sublingual tablet, the buccal or the oral rapid disintegrating preparation, preparations containing the compound (I) of the present invention or the joint use drug, and an excipient are preferable. Alternatively, an auxiliary agent such as a lubricant, tonicity agent, a hydrophilic carrier, a water-dispersible polymer, and a stabilizing agent may be included. Alternatively, in order to facilitate absorption and enhance bioavailability, β-cyclodextrin or a β-cyclodextrin derivative (e.g. hydroxypropyl-β-cyclodextrin etc.) may be included.

Examples of the excipient include lactose, white sugar, D-mannitol, starch, crystalline cellulose, and light silicic anhydride. Examples of the lubricant include magnesium stearate, calcium stearate, talc, and colloidal silica and, particularly, magnesium stearate and colloidal silica are preferable. Examples of the isotonic include sodium chloride, glucose, fructose, mannitol, sorbitol, lactose, saccharose, glycerin, and urea and, particularly, mannitol is preferable. Examples of the hydrophilic carrier include crystalline cellulose, ethylcellulose, crosslinking polyvinylpyrrolidone, light silicic anhydride, silicic acid, dicalcium phosphate, and a swelling hydrophilic carrier such as calcium carbonate and, particularly, crystalline cellulose (e.g. microcrystalline cellulose etc.) is preferable. Examples of the water-dispersible polymer include a gum (e.g. tragacanth gum, acacia gum, guar gum), alginate (e.g. sodium alginate), cellulose derivative (e.g. methylcellulose, carboxymethylcellulose, hydroxymethylcellulose, hydroxypropylcellulose, hydroxypropylmethylcellulose), gelatin, water-soluble starch, polyacrylic acid (e.g. carbomer), polymethacrylic acid, polyvinyl alcohol, polyethylene glycol, polyvinylpyrrolidone, polycarbophil, ascorbic acid, and palmitate, and hydroxypropylmethylcellulose, polyacrylic acid, alginate, gelatin, carboxymethylcellulose, polyvinylpyrrolidone, and polyethylene glycol are preferable. Particularly, hydroxypropylmethylcelullose is preferable. Examples of the stabilizing agent include cysteine, thiosorbitol, tartaric acid, citric acid, sodium carbonate, ascorbic acid, glycine, and sodium sulfite and, particularly, citric acid and ascorbic acid are preferable.

A sublingual tablet, a buccal or an oral rapid disintegrating preparation can be prepared by mixing the compound (I) of the present invention or the joint use drug and an excipient by the known per se method. Further, if desired, the aforementioned auxiliary agent such as a lubricant, tonicity agent, a hydrophilic carrier, a water-dispersible polymer, a stabilizing agent, a colorant, a sweetener, and an antiseptic may be mixed therein. After the aforementioned components are mixed simultaneously or at different times, a sublingual tablet, a buccal tablet or an oral rapid disintegrating tablet is obtained by molding by compression under pressure. In order to obtain a suitable hardness, the tablet may be prepared by wetting or wet-swelling the material, if necessary, using a solvent such as water and an alcohol before or after a stage of compression molding and, after molding, drying this.

When molded into a mucosal applying tablet (film), the compound (I) of the present invention or the joint use drug and the aforementioned water-dispersible polymer (preferably hydroxypropylcellulose, hydroxypropylmethylcellulose) and excipient are dissolved in a solvent such as water, and the resulting solution is cast to obtain a film. Further, an additive such as a plasticizer, a stabilizer, an antioxidant, a preservative, a colorant, a buffer and a sweetener may be added. In order to impart suitable elasticity to a film, glycols such as polyethylene glycol and propylene glycol may be contained, or in order to enhance adherability of a film to a mucosal lining in an oral cavity, a biological adhesive polymer (e.g. polycarbophil, carbopol) may be contained. Casting is performed by pouring a solution on a non-adhesive surface, spreading this to a uniform thickness (preferably around 10 to 1000 micron) with a coating equipment such as a doctor blade, and drying the solution to form a film. The thus formed film may be dried at room temperature or under warming, and cut into a desired surface area.

Examples of a preferable oral rapid disintegrating preparation include a solid rapid diffusing agent composed of a net-like entity of the compound (I) of the present invention or the joint use drug, and a water-soluble or water-diffusing carrier which is inert to the compound (I) of the present invention or the joint use drug. The net-like entity is obtained by sublimating a solvent from a solid composition composed of a solution in which the compound (I) of the present invention or the joint use drug is dissolved in a suitable solvent.

It is preferable that a composition of the oral rapid disintegrating preparation contains a matrix forming agent and a secondary component in addition to the compound (I) of the present invention or the joint use drug.

The matrix forming agent includes gelatins, dextrins, as well as animal proteins or plant proteins such as soybean, wheat and psyllium seed proteins; gummy substances such as gum arabic, guar gum, agar and xanthane; polysaccharides; alginates; carboxymethylcelluloses; carrageenans; dextrans; pectins; synthetic polymers such as polyvinylpyrrolidone; substances derived from gelatin-gum arabic complex. Further, the matrix forming agent includes sugars such as mannitol, dextrose, lactose, galactose and trehalose; cyclic sugars such as cyclodextrin; inorganic salts such as sodium phosphate, sodium chloride and aluminum silicate; amino acids of a carbon number of 2 to 12 such as glycine, L-alanine, L-aspartic acid, L-glutamic acid, L-hydroxyproline, L-isoleucine, L-leucine, and L-phenylalanine.

One or more kinds of the matrix forming agents can be introduced into a solution or a suspension before solidification thereof. Such the matrix forming agent may be present in addition to a surfactant, or may be present without a surfactant. The matrix forming agent can assist to maintain the diffused state of the compound (I) of the present invention or the joint use drug as it is in a solution or a suspension, in addition to formation of a matrix.

A composition may contain a secondary component such as a preservative, an antioxidant, a surfactant, a viscosity increasing agent, a colorant, a pH adjusting agent, a flavor, a sweetener and a taste masking agent. Examples of a suitable colorant include red, black and yellow iron oxides, as well as FD & C dyes such as FD & C Blue No. 2 and FD & C Red No. 40 of Ellis And Ebelar. Examples of a suitable flavor include mint, raspberry glycyrrhiza, orange, lemon, grapefruit, caramel, vanilla, cherry and grape flavor, and a combination thereof. Examples of a suitable pH adjusting agent include citric acid, tartaric acid, phosphoric acid, hydrochloric acid and maleic acid. Examples of a suitable sweetener include aspartame, acesulphame K and taumatin. Examples of a suitable taste masking agent include sodium bicarbonate, ion exchange resin, cyclodextrin inclusion compound, adsorbing substance and microcapsulated apomorphine.

A preparation contains the compound (I) of the present invention or the joint use drug usually at about 0.1 to about 50% by weight, preferably at about 0.1 to about 30% by weight, and a preparation (the aforementioned sublingual tablet, buccal) which can dissolve 90% or more of the compound (I) of the present invention or the joint use drug (in water) for about 1 minute to about 60 minutes, preferably about 1 minute to about 15 minutes, more preferably about 2 minutes to about 5 minutes, and an oral rapid disintegrating preparation which is disintegrated in 1 to 60 seconds, preferably 1 to 30 seconds, further preferably 1 to 10 seconds after administered into an oral cavity, are preferable.

A content of the excipient relative to a whole preparation is about 10 to about 99% by weight, preferably about 30 to about 90% by weight. A content of β-cyclodextrin or a β-cyclodextrin derivative relative to a whole preparation is 0 to about 30% by weight. A content of a lubricant to a whole preparation is about 0.01 to about 10% by weight, preferably about 1 to about 5% by weight. A content of tonicity agent to a whole preparation is about 0.1 to about 90% by weight, preferably about 10 to about 70% by weight. A content of a hydrophilic carrier to a whole preparation is about 0.1 to about 50% by weight, preferably about 10 to about 30% by weight. A content of a water-dispersible polymer to a whole preparation is about 0.1 to about 30% by weight, preferably about 10 to about 25% by weight. A content of a stabilizing agent relative to a whole preparation is about 0.1 to about 10% by weight, preferably about 1 to about 5% by weight. The aforementioned preparation may further contain an additive such as a colorant, a sweetener, and an antiseptic, if necessary.

A dose of the joint use of the present invention is different depending on a kind of the compound (I) of the present invention, an age, a weight, symptom, a dosage form, an administration method, and an administration term and, for example, about 0.01 to about 1000 mg/kg, preferably about 0.01 to about 100 mg/kg, more preferably about 0.1 to about 100 mg/kg, particularly about 0.1 to about 50 mg/kg, inter alia, about 1.5 to about 30 mg/kg per day in terms of the compound of the present invention and the joint use drug is intravenously administered per a diabetic patient (adult, weight about 60 kg) once to a few times a day. Of course, since a dose varies under the various conditions as described above, an amount smaller than the aforementioned dose is sufficient in some cases, and an amount exceeding the aforementioned range must be administered in some cases.

Any amount of the joint use drug may be set in such as range that side effect does not become problematic. A one day dose as the joint use drug is different depending on an extent of symptom, an age, a sex, a weight, and a difference in sensitivity of an administration subject, a term, and an interval of administration, nature, compounding, a kind, and a kind of an active ingredient of a drug preparation, and is not particularly limited. An amount of a drug is usually about 0.001 to 2000 mg, preferably about 0.01 to 500 mg, further preferably about 0.1 to 100 mg per 1 kg of a weight of a mammal by oral administration, and this is usually administered by dividing into once to four times a day.

Upon administration of the joint use agent of the present invention, the compound (I) of the present invention and the joint use drug may be administered at the same term, or after administration of the joint use drug, the compound (I) of the present invention may be administered, or after administration of the compound (I) of the present invention, the joint use drug may be administered. When they are administered at an interval, the interval is different depending on an active ingredient to be administered, a dosage form, and an administration method, and for example, when the joint use drug is administered first, there is a method of administering the compound (I) of the present invention in 1 minute to 3 days, preferably 10 minutes to 1 day, more preferably 15 minutes to 1 hour after administration of the joint use drug. When the compound (I) of the present invention is administered first, there is a method of administering the joint use drug in 1 minute to 1 day, preferably 10 minutes to 6 hours, more preferably 15 minutes to 1 hour after administration of the compound (I) of the present invention.

As a preferable administering method, for example, about 0.001 to 200 mg/kg of the joint use drug which has been formulated into an oral administration preparation is orally administered and, after about 15 minutes, about 0.005 to 100 mg/kg of the compound (I) of the present invention which has been formulated into an oral administration preparation is orally administered as one dose.

### Examples

The present invention will be explained in detail by way of the following Reference Examples, Examples, Preparation Examples and Test Examples, but these examples are merely actual examples, and do not limit the present invention, and a variation is possible in such a range that it is not apart from the scope of the present invention.

Elution in column chromatography of Reference Examples, and Examples was performed under observation with TLC (Thin Layer Chromatography). In TLC observation, 60F₂₅₄ manufactured by Merck, or NH manufactured by Fuji Silicia Chemical was adopted as a TLC plate, a solvent used as an elution solvent in column chromatography was adopted as a developing solvent, and a UV detector was adopted as a detecting method. As the column silica gel, Kiesel Gel 60 (70 to 230 mesh) or Kiesel Gel 60 (230 to 400 mesh) manufactured by Merck was used. NMR spectra were measured with Varian Gemini 200-type, Varian Mercury 300-type or Brucca DPX-300-type spectrometer using tetramethylsilane as an internal or external standard, and a chemical shift is shown in a δ value, and a coupling constant is shown in Hz. IR spectra were measured with Shimadzu FTIR-8200-type spectrometer.

In a Reference Examples, and Examples, HPLC was measured under the following conditions, and purity and the like were determined.
Measuring equipment: Shimadzu Corporation LC-10Avp system (when otherwise is indicated) or Ajirent 1100 system
Column: CAPSEL PAK C18UG120 S-3 µm, 2.0×50 mm
Solvent: A solution; 0.1% trifluoroacetic acid containing water,
   B solution; 0.1% trifluoroacetic acid containing acetonitrile
Gradient cycle: (A method): 0.00 minute (A solution/B solution = 90/10), 2.00 minutes (A solution/B solution = 5/95), 2.75 minutes (A solution/B solution = 5/95), 2.76 minutes (A solution/B solution = 90/10), 3.45 minutes (A solution/B solution = 90/10), or (B method): 0.00 minute (A solution/B solution = 90/10), 4.00 minutes (A solution/B solution = 5/95), 5.50 minutes (A solution/B solution = 5/95), 5.51 minutes (A solution/B solution = 90/10), 8.00 minutes (A solution/B solution = 90/10)
Injection amount: 10 µl, flow rate: 0.5 ml/min, detecting method: UV 220 nm

In Reference Examples, and Examples, mass spectra (MS) were measured under the following conditions.
Measurement equipment: Micromass Platform II, Waters ZQ, Waters ZMD, or JEOL. Ltd. JMS-AX505W
Ionization method: Atmospheric Pressure Chemical Ionization (APCI), Electron Spray Ionization (ESI), or Fast Atom Bombardment (FAB)

In purification of compounds in Reference Examples, and Examples, in addition to column chromatography, the following preparative HPLC equipment or intermediate pressure preparative LC equipment was used.

### 1) Preparative HPLC equipment: Gilson High Throughput purifying system

Column: YMC Combiprep ODS-A S-5 µm, 50×20 mm
Solvent: A solution; 0.1% trifluoroacetic acid containing water
   B solution; 0.1% trifluoroacetic acid containing acetonitrile
Gradient cycle: 0.00 minute (A solution/B solution = 90/10), 1.20 minutes (A solution/B solution = 90/10), 4.75 minutes (A solution/B solution = 0/100), 7.30 minutes (A solution/B solution = 0/100), 7.40 minutes (A solution/B solution = 90/10), 7.50 minutes (A solution/B solution = 90/10)
Flow rate: 25 ml/min, detecting method: UV 220 nm

### 2) Moderate pressure preparative LC equipment: Molitex High Throughput purifying system (PURIF 8) column: Yamazen HI-FLASH™ COLUMN (silica gel: 40 µm, 60 Å), 26×100 mm or 20×65 mm

Flow rate: 20 ml/min
Detecting method: UV 254 nm

In a mixed solvent, a numerical indicated in ( ) is a volume mixing ratio of each solvent. And, % in a solution represents a g number in 100 ml of a solution.

In addition, symbols in Reference Examples, and Examples have the following meanings.
S: singlet
d: doublet
t: triplet
q: quartet
quint: quintet
dd: double doublet
m: multiplet
br: broad
brs: broad singlet
J: coupling constant
CDCl₃: heavy chloroform
DMSO-d₆ :heavy dimethyl sulfoxide
¹H-NMR: proton nuclear magnetic resonance
WSC: water-soluble carbodiimide
THF: tetrahydrofuran
DMF: dimethylformamide
DMSO: dimethyl sulfoxide
DNA: deoxyribonucleic acid

In case that nucleotides and amino acids are represented by symbols herein, they are those of IUPAC-IBU Commission on Biochemical Nomenclature or conventional ones in this art field. Examples thereof are shown below. Further, in case that there are optical isomers of amino acids, they are L-isomers unless otherwise stated.
cDNA: complementary deoxyribonucleic acid
A: adenine
T: thymine
G: guanine
C: cytosine
Gly: glycine
Ala: alanine
Val: valine
Leu: leucine
Ile: isoleucine
Ser: serine
Thr: threonine
Cys: cysteine
Met: methionine
Glu: glutamic acid
Asp: aspartic acid
Lys: lysine
Arg: arginine
His: histidine
Phe: phenylalanine
Tyr: tyrosine
Trp: tryptophan
Pro: proline
Asn: asparagine
Gln: glutamine

SEQ ID Nos. in Sequence Listing in the present specification represent the following sequences.

### [SEQ ID No.: 1]

An amino acid sequence of a human-type RFRP receptor (OT7T022) is shown.

### [SEQ ID No.: 2]

An amino acid sequence of a rat-type RFRP receptor is shown.

### [SEQ ID No.: 3]

An amino acid sequence of human RFRP is shown.

### Reference Example 1

### Ethyl 6-fluoro-4-hydroxy-2-neopentyl-1-oxo-1,2-dihydro-3-isoquinolinecarboxylate and ethyl 7-fluoro-4-hydroxy-2-neopentyl-1-oxo-1,2-dihydro-3-isoquinolinecarboxylate

According to the method described in Example 1 (1) of WO02/62764, the title compounds were obtained.

### Reference Example 2

### Ethyl 4-butoxy-6-fluoro-2-neopentyl-1-oxo-1,2-dihydro-3-isoquinolinecarboxylate

According to the method described in Example 1 (2) of WO02/62764, the title compound was obtained.

### Reference Example 3

### Ethyl 4-butoxy-7-fluoro-2-neopentyl-1-oxo-1,2-dihydro-3-isoquinolinecarboxylate

According to the method described in Example 2 (1) of WO02/62764, the title compound was obtained.

### Reference Example 4

### Ethyl 6,7-dichloro-4-hydroxy-2-neopentyl-1-oxo-1,2-dihydro-3-isoquinolinecarboxylate

According to the method described in Example 27 (1) of WO02/62764, the title compound was obtained.

### Reference Example 5

### tert-Butyl 6,7-dichloro-2-(3-ethoxy-3-oxopropyl)-4-hydroxy-1-oxo-1,2-dihydro-3-isoquinolinecarboxylate

According to the method described in Example 35 (1) of WO02/62764, the title compound was obtained.

### Reference Example 6

### Ethyl 4-hydroxy-2-neopentyl-1-oxo-1,2-dihydro-3-isoquinolinecarboxylate

According to the method described in Example 46 (1) of WO02/62764, the title compound was obtained.

### Reference Example 7

### Ethyl 4-hydroxy-7-methyl-2-neopentyl-1-oxo-1,2-dihydro-3-isoquinolinecarboxylate

According to the method described in Example 50 (1) of WO02/62764, the title compound was obtained.

### Reference Example 8

### Ethyl 4-hydroxy-6-methyl-2-neopentyl-1-oxo-1,2-dihydro-3-isoquinolinecarboxylate

According to the method described in Example 51 (1) of WO02/62764, the title compounds were obtained.

### Reference Example 9

### Ethyl 4-hydroxy-2-neopentyl-1-oxo-7-trifluoromethyl-1,2-dihydro-3-isoquinolinecarboxylate

According to the method described in Example 52 (1) of WO02/62764, the title compound was obtained.

### Reference Example 10

### Ethyl 4-hydroxy-2-neopentyl-1-oxo-6-trifluoromethyl-1,2-dihydro-3-isoquinolinecarboxylate

According to the method described in Example 53 (1) of WO02/62764, the title compound was obtained.

### Reference Example 11

### Ethyl 4-hydroxy-2-neopentyl-1-oxo-1,2-dihydrobenzo[g]isoquinoline-3-carboxylate

According to the method described in Example 56 (1) of WO02/62764, the title compound was obtained.

### Reference Example 12

### tert-Butyl 4-butoxy-6-fluoro-2-neopentyl-1-oxo-1,2-dihydro-3-isoquinolinecarboxylate

According to the method described in Example 57 (2) of WO02/62764, the title compound was obtained.

### Reference Example 13

### Ethyl 6-benzyloxy-4-hydroxy-2-neopentyl-1-oxo-1,2-dihydro-3-isoquinolinecarboxylate

According to the method described in Example 58 (1) of WO02/62764, the title compound was obtained.

### Reference Example 14

### Ethyl 7-benzyloxy-4-hydroxy-2-neopentyl-1-oxo-1,2-dihydro-3-isoquinolinecarboxylate

According to the method described in Example 63 (1) of WO02/62764, the title compound was obtained.

### Reference Example 15

### Ethyl 5,6-dimethoxy-4-hydroxy-2-neopentyl-1-oxo-1,2-dihydro-3-isoquinolinecarboxylate

According to the method described in Example 69 (2) of WO02/62764, the title compound was obtained.

### Reference Example 16

### Ethyl 6,7-dimethoxy-4-hydroxy-2-neopentyl-1-oxo-1,2-dihydro-3-isoquinolinecarboxylate

According to the method described in Example 70 (2) of WO02/62764, the title compound was obtained.

### Reference Example 17

### Ethyl 4-hydroxy-6-neopentyl-7-oxo-6,7-dihydrothieno[2,3-c]pyridine-5-carboxylate

According to the method described in Example 71 (2) of WO02/62764, the title compound was obtained.

### Reference Example 18

### Ethyl 4-hydroxy-6-neopentyl-7-oxo-6,7-dihydrothieno[3,2-c]pyridine-5-carboxylate

According to the method described in Example 72 (2) of WO02/62764, the title compound was obtained.

### Reference Example 19

### Ethyl 6-bromo-4-hydroxy-2-neopentyl-1-oxo-1,2-dihydro-3-isoquinolinecarboxylate

According to the method described in Example 83 (1) of WO02/62764, the title compound was obtained.

### Reference Example 20

### Ethyl 7-fluoro-4-hydroxy-2-isobutyl-1-oxo-1,2-dihydro-3-isoquinolinecarboxylate and ethyl 6-fluoro-4-hydroxy-2-isobutyl-1-oxo-1,2-dihydro-3-isoquinolinecarboxylate

According to the method described in Example 150 (1) of WO02/62764, the title compounds were obtained.

### Reference Example 21

### Ethyl 2-cyclopropylmethyl-7-fluoro-4-hydroxy-1-oxo-1,2-dihydro-3-isoquinolinecarboxylate and 2-cyclopropylmethyl-6-fluoro-4-hydroxy-1-oxo-1,2-dihydro-3-isoquinolinecarboxylate

According to the method described in Example 149 (1) of WO02/62764, the title compounds were obtained.

### Reference Example 22

### tert-Butyl 7-benzyloxy-4-hydroxy-2-isobutyl-1-oxo-1,2-dihydro-3-isoquinolinecarboxylate and tert-butyl 6-benzyloxy-4-hydroxy-2-isobutyl-1-oxo-1,2-dihydro-3-isoquinolinecarboxylate

According to the method described in Example 161 (1) of WO02/62764, the title compounds were obtained.

### Example 1

### tert-Butyl 7-fluoro-4-hydroxy-2-neopentyl-1-oxo-1,2-dihydro-3-isoquinolinecarboxylate

To a suspension of 4-fluorophthalic anhydride (6.59 g) in methanol (100 mL) was added a 28% sodium methylate solution (15 mL) and the mixture was stirred at room temperature for 1 hour. The reaction mixture was poured into 1 N hydrochloric acid (150 mL) and the mixture was extracted with ethyl acetate. The extract was washed with brine, dried over anhydrous magnesium sulfate, and concentrated under reduced pressure. The residue was dissolved in acetonitrile (100 mL). To the solution were added tert-butyl 2-(neopentylamino)acetate (11.66 g), 1-ethyl-3-(3-dimethylaminopropyl)carbodiimide hydrochloride (14.3 g) and 1-hydroxybenzotriazole (7.87 g), and the mixture was stirred at room temperature for 15 hours. The reaction mixture was poured into water, and the mixture was washed with ethyl acetate. The extract was washed with brine, dried over anhydrous magnesium sulfate, and concentrated under reduced pressure. The residue was dissolved in tetrahydrofuran (200 mL), and to the solution was added potassium tert-butoxide (6.48 g) at 0°C. The mixture was stirred at room temperature for 1 hour. The reaction mixture was poured into 1 N hydrochloric acid (150 mL), and the mixture was extracted with ethyl acetate. The extract was washed with brine, dried over anhydrous magnesium sulfate, and concentrated under reduced pressure. The residue was purified by silica gel column chromatography, and the component eluted in preference was concentrated to obtain the title compound (2.0 g) as colorless crystals.
¹H-NMR(CDCl₃) δ : 0.86 (9H, s), 1. 65 (9H, s), 4.55 (2H, br), 7.45 (1H, m), 8.04-20 (2H, m), 10.88 (1H, s).

### Example 2

### tert-Butyl 6-fluoro-4-hydroxy-2-neopentyl-1-oxo-1,2-dihydro-3-isoquinolinecarboxylate

In the purification by silica gel column chromatography in Example 1, the component eluted afterward was concentrated to obtain the title compound (3.85 g) as colorless crystals.
¹H-NMR(CDCl₃) δ : 0.86 (9H, s), 1.65 (9H, s), 4.57 (2H, br), 7.34 (1H, dt, J=8.8, 2.5 Hz), 7.74 (1H, dd, J=9.2, 2.5 Hz), 8.46 (1H, dd, J=8.8, 5.5 Hz), 10.67 (1H, s).

### Example 3

### tert-Butyl 6,7-dichloro-4-hydroxy-2-neopentyl-1-oxo-1,2-dihydro-3-isoquinolinecarboxylate

A solution of 4,5-dichlorophthlic anhydride (5.0 g), tert-butyl 2-(neopentylamino)acetate (4.64 g) and triethylamine (4.66 g) in tetrahydrofuran (100 mL) was stirred at room temperature for 15 hours. The reaction mixture was poured into water, and the mixture was extracted with ethyl acetate. The extract was washed with aqueous 10% citric acid solution and brine, dried over anhydrous magnesium sulfate, and concentrated under reduced pressure. The residue was dissolved in acetone (50 mL) and N,N-dimethylformamide (5 mL), and to the solution were added potassium carbonate (3,18 g) and methyl iodide (6.4 g). The mixture was stirred at room temperature for 15 hours. The reaction mixture was poured into water, and the mixture was extracted with ethyl acetate. The extract was washed with brine, dried over anhydrous magnesium sulfate, and concentrated under reduced pressure. The residue was dissolved in tetrahydrofuran (100 mL) and to the solution was added potassium tert-butoxide (2.58 g) at 0°C. The mixture was stirred at room temperature for 1 hour. The reaction mixture was poured into an aqueous 10% citric acid solution (150 mL), and the crystals precipitated were collected by filtration. The crystals were washed with water and methanol to obtain the title compound (6.4 g).
¹H-NMR(CDCl₃) δ :0.85 (9H, s), 1.65 (9H, s), 4.45 (2H, br), 8.20 (1H, s), 8.51 (1H, s), 10.71 (1H, s).

### Example 4

### tert-Butyl 4-butoxy-6,7-dichloro-2-neopentyl-1-oxo-1,2-dihydro-3-isoquinolinecarboxylate

To a solution of tert-butyl 6,7-dichloro-4-hydroxy-2-neopentyl-1-oxo-1,2-dihydro-3-isoquinolinecarboxylate (399 mg), 1-butanol (180 mg) and triphenylphosphine (390 mg) in tetrahydrofuran (20 mL) was added diethyl diazocarboxylate (260 mg), and the mixture was stirred at room temperature for 15 hours. The reaction mixture was concentrated under reduced pressure, and the residue was purified by silica gel chromatography to obtain the title compound (310 mg) as colorless crystals.
¹H-NMR(CDCl₃) δ : 0.95 (9H, s), 1.02 (3H, t, J=7.3 Hz), 1.55 (2H, m), 1.62 (9H, s), 1.81 (2H, m), 3.95 (2H, t, J=6.7 Hz), 4.00 (2H, br), 7.80 (1H, s), 8.49 (1H, s).

### Example 5

### tert-Butyl 6,7-dichloro-4-methoxy-2-neopentyl-1-oxo-1,2-dihydro-3-isoquinolinecarboxylate

To a solution of tert-butyl 6,7-dichloro-4-hydroxy-2-neopentyl-1-oxo-1,2-dihydro-3-isoquinolinecarboxylate (399 mg) and potassium carbonate (138 mg) in DMF (20 mL) was added methyl iodide (420 mg), and the mixture was stirred at room temperature for 15 hours. The reaction mixture was poured into water and the mixture was extracted with ether. The extract was washed in turn with aqueous 1 N sodium hydroxide solution, water and brine, dried over anhydrous magnesium sulfate, and concentrated under reduced pressure. The residue was purified by silica gel chromatography to obtain the title compound (300 mg) as colorless crystals.
¹H-NMR(CDCl₃) δ : 0.96 (9H, s), 1.64 (9H, s), 3.87 (3H, s), 4.03 (2H, br), 7.84 (1H, s), 8.50 (1H, s).

### Example 6

### tert-Butyl 4-benzyloxy-6,7-dichloro-2-neopentyl-1-oxo-1,2-dihydro-3-isoquinolinecarboxylate

To a solution of tert-butyl 6,7-dichloro-4-hydroxy-2-neopentyl-1-oxo-1,2-dihydro-3-isoquinolinecarboxylate (300 mg), benzyl alcohol (97 mg) and triphenylphosphine (157 mg) in tetrahydrofuran (4 mL) was added diethyl azodicarboxylate (157 mg), and the mixture was stirred at room temperature for 15 hours. The reaction mixture was concentrated under reduced pressure, and the residue was purified by silica gel chromatography to obtain the title compound (90 mg) as colorless crystals.
¹H-NMR(CDCl₃) δ : 0.98 (9H, s), 1.56 (9H, s), 4.04 (2H, br), 5.04 (2H, 2H), 7.32-7.49 (m, 5H), 7.75 (1H, s), 8.50 (1H, s).

### Example 7

### tert-Butyl 6,7-dichloro-2-neopentyl-1-oxo-4-trifluoromethanesulfonyloxy-1,2-dihydro-3-isoquinolinecarboxylate

To a solution of tert-butyl 6,7-dichloro-4-hydroxy-2-neopentyl-1-oxo-1,2-dihydro-3-isoquinolinecarboxylate (2.5 g) and pyridine (2.5 g) in methylene chloride (90 mL) was added trifluoromethanesulfonic anhydride (1.9 g) with stirring at -78°C, and the mixture was stirred to 5 minutes. After stirring at 0°C for additional 1 hour, the reaction mixture was poured into a saturated aqueous solution of sodium bicarbonate, and the mixture was extracted with ethyl acetate. The extract was washed in turn with 1 N hydrochloric acid, a saturated aqueous solution of sodium bicarbonate, water and brine, dried over anhydrous magnesium sulfate, and concentrated under reduced pressure. The residue was recrystallized from hexane/ethyl acetate to obtain the title compound (2.5 g) as white crystals.
¹H-NMR(CDCl₃) δ : 0.92 (9H, s), 1.64 (9H, s), 4.17 (2H, br), 7.85 (1H, s), 8.50 (1H, s).

### Example 8

### tert-Butyl 6,7-dichloro-2-neopentyl-1-oxo-4-phenyl-1,2-dihydro-3-isoquinolinecarboxylate

A mixture of tert-butyl 6,7-dichloro-2-neopentyl-1-oxo-4-trifluoromethanesulfonyloxy-1,2-dihydro-3-isoquinolinecarboxylate (430 mg), phenyl boronic acid (118 mg), tetrakis(triphenylphosphine)palladium (94 mg), dimethoxyethane (12 mL) and aqueous 2 N sodium carbonate solution (3 mL) placed in a round bottom flask equipped with a Dimroth condenser was degassed, replaced with argon, and stirred at 100°C for 15 hours. The reaction mixture was cooled, and filtrated with Celite, and the filtrate was extracted with ethyl acetate. The extract was washed with water and brine, dried over anhydrous magnesium sulfate, and concentrated under reduced pressure. The residue was purified by silica gel column chromatography to obtain the title compound (100 mg) as colorless crystals.
¹H-NMR(CDCl₃) δ : 0.99 (9H, s), 1.15 (9H, s), 4.11 (2H, br), 7.13 (1H, s), 7.25-7.35 (2H, m), 7.42-7.49 (3H, m), 8.55 (1H, s).

### Example 9

### tert-Butyl 6,7-dichloro-4-(2-ethoxy-2-oxoethoxy)-2-neopentyl-1-oxo-1,2-dihydro-3-isoquinolinecarboxylate

A mixture of tert-butyl 6,7-dichloro-4-hydroxy-2-neopentyl-1-oxo-1,2-dihydroisoquinoline-3-carboxylate (4.00 g, 10.0 mmol), potassium carbonate (2.07 g, 15.0 mmol), ethyl bromoacetate (1.7 mL, 15.0 mmol) and N,N-dimethylformamide (30 mL) was stirred at room temperature for 3 hours. The reaction mixture was poured into water, and the mixture was extracted with ethyl acetate. The extract was washed with saturated brine, dried over anhydrous magnesium sulfate, and concentrated under reduced pressure. The residue was purified by silica gel chromatography to obtain the title compound (4.51 g, yield 95%) as a colorless oily material.
¹H-NMR (CDCl₃) δ: 0.95 (9H, s), 1.36 (3H, t, J = 7.1 Hz), 1.65 (9H, s), 4.03 (2H, br), 4.34 (2H, q, J = 7.1 Hz), 4.59 (2H, s), 8.02 (1H, s), 8.49 (1H, s).

### Example 10

### tert-Butyl 7-bromo-4-hydroxy-2-neopentyl-1-oxo-1,2-dihydroisoquinoline-3-carboxylate

To a solution of 4-bromophthalic anhydride (22.70 g, 100 mmol) in tetrahydrofuran (200 mL) was added ethyl 2-(neopentylamino)acetate (20.79 g, 120 mmol), and the mixture was stirred at room temperature for 1 hour. The reaction mixture was poured into water, made acidic with 1 N hydrochloric acid, and extracted with ethyl acetate. The extract was washed with saturated brine, and dried over anhydrous magnesium sulfate, and the solvent was distilled off under reduced pressure. The residue was dissolved in N,N-dimethylformamide (200 mL), to the solution were added potassium carbonate (13.82 g, 100 mmol) and methyl iodide (9.6 mL, 120 mmol), and the mixture was stirred at room temperature for 2 hours. The reaction mixture was poured into water, and the mixture was extracted with ethyl acetate. The extract was washed with brine, dried over anhydrous magnesium sulfate, and concentrated under reduced pressure. The residue was dissolved in ethanol (200 mL), to the solution was added a 20% solution of sodium ethoxide in ethanol (68.10 g, 200 mmol), and the mixture was stirred at room temperature for 1 hour. The reaction mixture was poured into 1 N hydrochloric acid (150 mL), and the mixture was extracted with ethyl acetate. The extract was washed with brine, dried over anhydrous magnesium sulfate, and concentrated under reduced pressure. The residue was purified by silica gel column chromatography, and the component eluted in preference was concentrated to obtain the title compound (12.07 g, 31.6%) as colorless crystals.
m.p. 103.5-104°C.
Elemental analysis for C₁₇H₂₀NO₄Br
Calcd: C, 53.42; H, 5.27; N, 3.66.
Found: C, 53.66; H, 5.27; N, 3.66.
¹H-NMR(CDCl₃) δ : 0.84 (9H, s), 1.47 (3H, t, J=7.2 Hz), 4.48 (2H, q, J=7.2 Hz), 4.54 (2H, br), 7.85 (1H, dd, J=2.0, 8.6 Hz), 8.01 (1H, d, J=8.6 Hz), 8.60 (1H, d, J=2.0 Hz), 10.80 (1H, s).

### Example 11

### Ethyl 8-hydroxy-6-neopentyl-5-oxo-5,6-dihydro[1,3]dioxolo[4,5-g]isoquinoline-3-carboxylate

(1) A solution of 6-bromo-1,3-benzodioxole-5-carbaldehyde (22.90 g, 100 mmol), methyl orthoformate (109 mL, 1000 mmol) and p-toluenesulfonic acid monohydrate (0.95 g, 5 mmol) in methanol (30 mL) was heated under reflux for 1 hour. The reaction mixture was poured into water, and the mixture was extracted with ethyl acetate. The extract was washed with saturated brine, and dried over anhydrous magnesium sulfate, and the solvent was distilled off under reduced pressure. The residue and N,N,N',N'-tetramethylethylenediamine (14.5 mL, 96 mmol) were dissolved in tetrahydrofuran (200 mL), and the solution was added dropwise to a solution of n-butyl lithium in hexane (60 mL, 96 mmol) at -78°C over 1 hour. After completion of the addition, the mixture was stirred at -78°C for 1 hour under a CO₂ atmosphere. The resulting mixture was poured into 1 N hydrochloric acid, followed by stirring at room temperature. The reaction mixture was poured into water and the mixture was extracted with ethyl acetate. The extract was washed with brine, dried over anhydrous magnesium sulfate, and concentrated under reduced pressure. The residue was dissolved in N,N-dimethylformamide (100 mL), to the solution were added potassium carbonate (6.91 g, 50 mmol) and ethyl iodide (4.8 mL, 60 mmol), and the mixture was stirred at room temperature for 3 hours. The reaction mixture was poured into water and the mixture was extracted with ethyl acetate. The extract was washed with brine, dried over anhydrous magnesium sulfate, and concentrated under reduced pressure. The residue was purified by silica gel chromatography to obtain crystals of ethyl 6-formyl-1,3-benzodioxole-5-carboxylate (2.12 g, 12%).
   ¹H-NMR(CDCl₃) δ : 1.42 (3H, t, J=7.2 Hz), 4.41 (2H, q, J=7.2 Hz), 6.12 (2H, s), 7.39 (1H, s), 7.43 (1H, s), 10.55 (1H, s).
(2) Ethyl 6-formyl-1,3-benzodioxole-5-carboxylate (2.22 g, 10 mmol), sodium dihydrogen phosphate (1.44 g, 12 mmol) and a solution of 2-methyl-2-butene (4.7 mL, 44 mmol) in tert-butanol (20 mL), tetrahydrofuran (20 mL) and water (10 mL) were stirred at room temperature for 10 minutes. To the resulting mixture was added sodium chlorite (3.07 g, 34 mmol), and the mixture was stirred at room temperature for 2 hours. The reaction mixture was poured into water, and the mixture was extracted with ethyl acetate. The extract was washed with brine, dried over anhydrous magnesium sulfate, and concentrated under reduced pressure to obtain 6-(ethoxycarbonyl)-1,3-benzodioxol-5-carboxylic acid (2.11 g, 88%) as an oily material. 6-(Ethoxycarbonyl)-1,3-benzodioxol-5-carboxylic acid (2.11 g, 8.9 mmol) was dissolved in tetrahydrofuran (20 mL), to the solution were added oxalyl chloride (1.1 mL, 12 mmol) and N,N-dimethylformamide (2 drops), and the mixture was stirred at room temperature for 1 hour. The solvent was distilled off under reduced pressure, and the residue was dissolved in tetrahydrofuran (20 mL). The resulting solution was slowly added dropwise to a solution of ethyl 2-(neopentylamino)acetate (2.69 g, 15 mmol) in N,N-dimethylacetamide (20 mL). After completion of the addition, the mixture was stirred at room temperature. The reaction mixture was poured into water and the mixture was extracted with ethyl acetate. The extract was washed with brine, dried over anhydrous magnesium sulfate, and concentrated under reduced pressure. The residue was dissolved in ethanol (50 mL), and the solution was added to a 20% solution of sodium ethoxide in ethanol (6.81 g, 20 mmol). The mixture was stirred at room temperature for 1 hour. The reaction mixture was poured into 1 N hydrochloric acid (20 ml), and the mixture was extracted with ethyl acetate. The extract was washed with brine, dried over anhydrous magnesium sulfate, and concentrated under reduced pressure. The residue was purified by silica gel chromatography to obtain the title compound (3.11 g, 89%) as a colorless oily material.
   ¹H-NMR(CDCl₃) δ : 0.84 (9H, s), 1.46 (3H, t, J=7.2 Hz), 4.46 (2H, q, J=7.2 Hz), 4.52 (2H, br), 6.14 (2H, s), 7.48 (1H, s), 7.81 (1H, s), 10.92 (1H, s).

### Example 12

### tert-Butyl 2-(1-adamantylmethyl)-6,7-dichloro-4-hydroxy-1-oxo-1,2-dihydro-3-isoquinolinecarboxylate

According to the same manner as that described in Example 3, the title compound (390 mg) as pale yellow crystals was obtained from 4,5-dichlorophthalic anhydride (680 mg) and tert-butyl 2-[(1-adamentylmethyl)amino]acetate (880 mg).
¹H-NMR(CDCl₃) δ:1.25-1.70 (21H, m), 1.86-1.92 (3H, m), 4.08 (1H, br), 4.74 (1H, 8.21 (1H, s), 8.51 (1H, s), 10.66 (1H, s) .

### Example 13

### tert-Butyl 6,7-dichloro-2-neopentyl-1-oxo-4-[2-(1-pyrrolidinyl)ethoxy]-1,2-dihydro-3-isoquinolinecarboxylate oxalate

To a solution of tert-butyl 6,7-dichloro-4-hydroxy-2-neopentyl-1-oxo-1,2-dihydro-3-isoquinolinecarboxylate obtained in Example 3 (200 mg) and 1-(2-chloroethyl)pyrrolidine hydrochloride (102 mg) in DMF (6.0 ml) was added potassium carbonate (346 mg), and the mixture was stirred at 70°C for 15 hours. The reaction mixture was poured into water, and the mixture was extracted with ethyl acetate. The extract was washed in turn with water and brine, dried over anhydrous magnesium sulfate, and concentrated under reduced pressure. The residue was purified by silica gel column chromatography to obtain tert-butyl 6,7-dichloro-2-neopentyl-1-oxo-4-[2-(1-pyrrolidinyl)ethoxy]-1,2-dihydro-3-isoquinolinecarboxylate as an oily material. To a solution of the oily material in ethyl acetate (5.0 mL) was added a solution of oxalic acid (25 mg) in 2-propanol (3.0 ml), and the mixture was stirred at room temperature for 5 minutes. The crystals precipitated were collected by filtration, and washed with ethyl acetate and 2-propanol to obtain the title compound (120 mg) as white crystals.
Free compound: ¹H-NMR(CDCl₃) δ : 0.95 (9H, s), 1.62 (9H, s), 1.82-1.95 (2H, m), 2.58-2.70 (2H, m), 2.89 (2H, t, J=5.4 Hz), 4.01 (2H, br), 4.07 (2H, t, J=5.4 Hz), 8.20 (1H, s), 8.48 (1H, s).

### Example 14

### tert-Butyl 6,7-dichloro-4-[3-(dimethylamino)propoxy]-2-neopentyl-1-oxo-1,2-dihydro-3-isoquinolinecarboxylate oxalate

According to the same manner as that described in Example 13, the title compound (70 mg) as white crystals was obtained from tert-butyl 6,7-dichloro-4-hydroxy-2-neopentyl-1-oxo-1,2-dihydro-3-isoquinolinecarboxylate obtained in Example 3 (200 mg) and 3-dimethylaminopropyl chloride hydrochloride (95 mg).
Free compound: ¹H-NMR(CDCl₃) δ : 0.95 (9H, s), 1.63 (9H, s), 1.92-2.02 (2H, m), 2.31 (6H, s), 2.52-2.58 (2H, m), 3.95-4.08 (4H, m), 8.15 (1H, s), 8.48 (1H, s).

### Example 15

### tert-Butyl 2-(1-adamantylmethyl)-6,7-dichloro-4-methoxy-1-oxo-1,2-dihydro-3-isoquinolinecarboxylate

According to the same manner as that described in Example 5, the title compound (90 mg) as colorless crystals was obtained from tert-butyl 2-(1-adamantylmethyl)-6,7-dichloro-4-hydroxy-1-oxo-1,2-dihydro-3-isoquinolinecarboxylate obtained in Example 12 (300 mg).
¹H-NMR(CDCl₃) δ : 1.50-1.80 (21H, m), 1.89-2.00 (3H, m), 3.78-4.00 (5H, m), 7.84 (1H, s), 8.50 (1H, s).

### Example 16

### N-(tert-Butyl)-6,7-dichloro-4-methoxy-2-neopentyl-1-oxo-1,2-dihydro-3-isoquinolinecarboxaminde

(1) A mixture of tert-butyl 6,7-dichloro-4-methoxy-2-neopentyl-1-oxo-1,2-dihydro-3-isoquinolinecarboxylate obtained in Example 5 (360 mg) and trifluoroacetic acid (6.0 ml) was stirred at room temperature for 15 hours. The reaction mixture was concentrated under reduced pressure, and the residue was recrystallized from diethyl ether to obtain 6,7-dichloro-4-methoxy-2-neopentyl-1-oxo-1,2-dihydro-3-isoquinolinecarboxylic acid (40 mg) as white crystals.
   ¹H-NMR(DMSO-d₆) δ : 0.89 (9H, s), 3.83 (3H, s), 4.01 (2H, br), 8.01 (1H, s), 8.37 (1H, s).
(2) To a solution of 6,7-dichloro-4-methoxy-2-neopentyl-1-oxo-1,2-dihydro-3-isoquinolinecarboxylic acid (190 mg) and DMF (20 mg) in THF (4.0 mL) was added oxalyl chloride (43 mg) at 0°C, and the mixture was stirred at room temperature for 2 hours. The reaction mixture was concentrated under reduced pressure, the residue was dissolved in methylene chloride (3.0 ml), and to the solution were added tert-butylamine (39 mg) and pyridine (126 mg). The mixture was stirred at room temperature for 15 hours, the reaction mixture was poured into water, and the mixture was extracted with ethyl acetate. The extract was washed in turn with an aqueous 1 N hydrochloric acid solution, water and brine, dried over anhydrous magnesium sulfate, and concentrated under reduced pressure. The residue was purified by silica gel column chromatography to obtain the title compound (90 mg) as colorless crystals.
   ¹H-NMR(CDC1₃) δ : 0.93 (9H, s), 1.51 (9H, s), 3.78 (3H, s), 4.20 (2H, bs), 6.43 (1H, bs), 7.77 (1H, s), 8.40 (1H, s).

### Example 17

### tert-Butyl 4-hydroxy-2-neopentyl-1-oxo-1,2-dihydro-3-isoquinolinecarboxylate

According to the same manner as that described in Example 3, the title compound (90 mg) as colorless crystals was obtained from phthlic anhydride (740 mg) and tert-butyl 2-(neopneylamino)acetate (1.00 g).
¹H-NMR(CDCl₃) δ : 0.87 (9H, s), 1.65 (9H, s), 4.48 (2H, br), 7.59-7.75 (2H, m), 8.13 (1H, d, J=8.0 Hz), 8.45 (1H, d, J=7.8 Hz).

### Example 18

### tert-Butyl 6,7-dichloro-4-[(2-(dimethylamino)ethoxy)]-2-neopentyl-1-oxo-1,2-dihydro-3-isoquinolinecarboxylate oxalate

According to the same manner as that described in Example 13, the title compound (96 mg) as white crystals was obtained from tert-butyl 6,7-dichloro-4-hydroxy-2-neopentyl-1-oxo-1,2-dihydro-3-isoquinolinecarboxylate obtained in Example 3 (200 mg) and 2-dimethylaminoethylchloride hydrochloride (86 mg).
Free compound: ¹H-NMR(CDCl₃) δ: 0.95 (9H, s), 1.62 (9H, s), 2.38 (6H, s), 2.70-2.74 (2H, m), 4.01 (2H, br), 4.02-4.07 (2H, m), 8.19 (1H, s), 8.48 (1H, s).

### Example 19

### N-{2-[Benzyl(methyl)amino]ethyl}-6,7-dichloro-4-methoxy-2-neopentyl-1-oxo-1,2-dihydro-3-isoquinolinecarboxamide hydrochloride

To a solution of 6,7-dichloro-4-methoxy-2-neopentyl-1-oxo-1,2-dihydro-3-isoquinolinecarboxylic acid obtained in Example 16 (1) (170 mg), 1-hydroxybenzotriazole (76 mg), 1-ethyl-3-(3-dimethylaminopropyl)carbodiimide hydrochloride (107 mg) and triethylamine (95 mg) in DMF (5.0 mL) was added N-benzyl-N-methyl-1,2-ethanediamine (92 mg), and the mixture was stirred at room temperature for 15 hours. The reaction mixture was poured into water, and the mixture was extracted with ethyl acetate. The extract was washed in turn with water and brine, dried over anhydrous magnesium sulfate, and concentrated under reduced pressure. The residue was purified by silica gel column chromatography to obtain N-{2-[2-benzyl(methyl)amino]ethyl}-6,7-dichloro-4-methoxy-2-neopentyl-1-oxo-1,2-dihydro-3-isoquinolinecarboxamide as a colorless oily material. To a solution of the oily material in methylene chloride (3.0 mL) was added ethereal 1 N hydrochloric acid (0,5 mL) and the mixture was stirred at room temperature for 5 minutes. The reaction mixture was concentrated under reduced pressure, and the resulting residue was crystallized by using ethyl acetate and hexane to obtain the title compound (23 mg) as pale yellow crystals.
¹H-NMR(DMSO-d₆) δ : 0.86 (9H, s), 2.78 (3H, d, J=4.4 Hz), 3.12-3.30 (2H, m), 3.52-3.83 (5H, m), 3.92 (2H, s), 4.30-4.52 (2H, m), 7.43-7.60 (5H, m), 8.01 (1H, s), 8.37 (1H, s), 9.20 (1H, brs), 10.00 (1H, brs).

### Example 20

### N-{3-[Benzyl(methyl)amino]propyl}-6,7-dichloro-4-methoxy-2-neopentyl-1-oxo-1,2-dihydro-3-isoquinolinecarboxamide hydrochloride

According to the same manner as that described in Example 19, the title compound (36 mg) as pale yellow crystals was obtained from 6,7-dichloro-4-methoxy-2-neopentyl-1-oxo-1,2-dihydro-3-isoquinolinecarboxylic acid obtained in Example 16 (1) (170 mg) and N-benzyl-N-methyl-1,3-propanediamine (100 mg).
¹H-NMR(DMSO-d₆) δ:0.87 (9H, s), 1.90-2.08 (2H, m), 2.69 (3H, d, J=4.6 Hz), 2.93-3.35 (4H, m), 3.69 (3H, s), 3.94 (2H, s), 4.20-4.46 (2H, m), 7.43-7.60 (5H, m), 7.99 (1H, s), 8.37 (1H, s), 9.02 (1H, bs), 10.00 (1H, brs).

### Example 21

### 6,7-Dichloro-N-[3-(dimethylamino)propyl]-4-methoxy-2-neopentyl-1-oxo-1,2-dihydro-3-isoquinolinecarboxamide hydrochloride

According to the same manner as that described in Example 19, the title compound (26 mg) as pale yellow crystals was obtained from 6,7-dichloro-4-methoxy-2-neopentyl-1-oxo-1,2-dihydro-3-isoquinolinecarboxylic acid obtained in Example 16 (1) (220 mg) and N,N-dimethyl-1,3-propanediamine (75 mg).
¹H-NMR(DMSO-d₆) δ : 0.88 (9H, s), 1.86-2.00 (2H, m), 2.78 (6H, s), 3.07-3.17 (2H, m), 3.22-3.45 (2H, m), 3.74 (3H, s), 3.96 (2H, br), 8.01 (1H, s), 8.38 (1H, s), 9.04 (1H, brs), 9.96 (1H, brs).

### Example 22

### 6,7-Dichloro-4-methoxy-2-neopentyl-1-oxo-N-[3-(1-pyrrolidinyl)propyl]-1,2-dihydro-3-isoquinolinecarboxamide hydrochloride

According to the same manner as that described in Example 19, the title compound (40 mg) as pale yellow crystals was obtained from 6,7-dichloro-4-methoxy-2-neopentyl-1-oxo-1,2-dihydro-3-isoquinolinecarboxylic acid obtained in Example 16 (1) (220 mg) and 1-(3-aminopropyl)pyrrolidine (94 mg).
¹H-NMR(DMSO-d₆) δ : 0.89 (9H, s), 1.85-2.08 (6H, m), 2.93-3.07 (2H, m), 3.14-3.26 (2H, m), 3.30-3.42 (2H, m), 3.50-3.62 (2H, m), 3.74 (3H, s), 3.96 (2H, br), 8.01 (1H, s), 8.38 (1H, s), 9.04 (1H, brs), 10.03 (1H, brs).

### Example 23

### tert-Butyl 4-amino-2-neopentyl-1-oxo-1,2-dihydro-3-isoquinolinecarboxylate

To a solution of 2-cyanobenzoic acid (1.00 g), 1-hydroxybenzotriazole (1.10 g), 1-ethyl-3-(3-dimethylaminopropyl)carbodiimide hydrochloride (1.60 g) and triethylamine (1.00 g) in DMF (10 mL)/acetonitrile (20 mL) was added tert-butyl 2-(neopentylamino)acetate (1.40 g), and the mixture was stirred at room temperature for 20 hours. The reaction mixture was poured into water and the mixture was extracted with ethyl acetate. The extract was washed in turn with water and brine, dried over anhydrous magnesium sulfate, and concentrated under reduced pressure. The residue was purified by silica gel column chromatography to obtain a colorless oily material. To a solution of the oily material in THF (20 mL) was added potassium tert-butoxide (115 mg) at 0°C, and the mixture was stirred at room temperature for 1 hour. The reaction mixture was poured into an aqueous 10% citric acid solution and the mixture was extracted with ethyl acetate. The extract was washed in turn with water and brine, dried over anhydrous magnesium sulfate, and concentrated under reduced pressure. The residue was purified by silica gel column chromatography, and crystallized by using ethyl acetate and hexane to obtain the title compound (50 mg) as pale yellow crystals.
¹H-NMR(CDCl₃) δ : 0.86 (9H, s), 1.61 (9H, s), 4.40 (2H, br), 5.07 (2H, brs), 7.59-7.90 (3H, m), 8.52 (1H, d, J=7.9 Hz).

### Example 24

### tert-Butyl 6,7-dichloro-4-(1H-indol-2-ylmethoxy)-2-neopentyl-1-oxo-1,2-dihydro-3-isoquinolinecarboxylate

According to the same manner as that described in Example 4, the title compound (75 mg) as pale yellow crystals was obtained from tert-butyl 6,7-dichloro-4-hydroxy-2-neopentyl-1-oxo-1,2-dihydro-3-isoquinolinecarboxylate obtained in Example 3 (375 mg) and indole-3-carbinol (152 mg).
¹H-NMR(CDCl₃) δ : 0.98 (9H, s), 1.62 (9H, s), 4.06 (2H, br), 5.22 (2H, brs), 6.57 (1H, s), 7.10-7.25 (2H, m), 7.39 (1H, d, J=8.1 Hz), 7.62 (1H, d, J=7.7 Hz), 7.85 (1H, s), 8.50 (1H, s), 8.82 (1H, brs).

### Example 25

### N-(1-Azabicyclo[2.2.2]oct-3-yl)-6,7-dichloro-4-methoxy-2-neopentyl-1-oxo-1,2-dihydro-3-isoquinolinecarboxamide hydrochloride

According to the same manner as that described in Example 19, the title compound (35 mg) as pale yellow crystals was obtained from 6,7-dichloro-4-methoxy-2-neopentyl-1-oxo-1,2-dihydro-3-isoquinolinecarboxylic acid obtained in Example 16 (1) (210 mg) and 3-aminoquinuclidine dihydrochloride (141 mg).
¹H-NMR(DMSO-d₆) δ : 0.89 (9H, s), 1.70-2.20 (4H, m), 2.90-3.02 (2H, m), 3.12-3.30 (3H, m), 3.66-3.78 (4H, m), 3.82-4.12 (2H, m), 4.38 (2H, br), 8.04 (1H, s), 8.39 (1H, s), 9.32 (1H, d, J=6.9 Hz), 9.61 (1H, brs).

### Example 26

### Methyl 6,7-dichloro-4-hydroxy-2-neopentyl-1-oxo-1,2-dihydro-3-isoquinolinecarboxylate

A solution of 4,5-dichlorophthalic anhydride (27.1 g), methyl 2-(neopentylamino)acetate (23.6 g) and triethylamine (25,3 g) in tetrahydrofuran (120 mL) was stirred at room temperature for 3 days. The reaction mixture was concentrated under reduced pressure, and poured into an aqueous 1 N hydrochloric acid solution, and the mixture was extracted with ethyl acetate. The extract was washed with brine, and concentrated under reduced pressure. The residue was crystallized by using ethyl acetate and hexane to obtain orange crystals. To a solution of the crystals in DMF (100 mL) were added potassium carbonate (15.8 g) and methyl iodide (10.8 g), and the mixture was stirred at room temperature for 15 hours. The reaction mixture was poured into water, and the mixture was extracted with ethyl acetate. The extract was washed in turn with water and brine, dried over anhydrous magnesium sulfate, and concentrated under reduced pressure. The residue was crystallized by using ethyl acetate and hexane to obtain pale orange crystals. To a solution of the crystals in THF (200 mL) was a 28% solution of sodium methoxide/methanol (10,0 g) at 0°C, and the mixture was stirred at room temperature for 30 minutes. The reaction mixture was poured into an aqueous 1 N hydrochloric acid solution, and the mixture was extracted with ethyl acetate. The extract was washed in turn with water and brine, dried over anhydrous magnesium sulfate, and concentrated under reduced pressure. The residue was crystallized by using ethyl acetate and hexane to obtain the title compound (5.47 g) as white crystals.
¹H-NMR(CDCl₃) δ : 0.83 (9H, s), 4.02 (3H, s), 4.70 (2H, br), 8.23 (1H, s), 8.53 (1H, s), 10.65 (1H, s).

### Example 27

### tert-Butyl 6,7-dichloro-4-(1H-indol-3-ylmethoxy)-2-neopentyl-1-oxo-1,2-dihydro-3-isoquinolinecarboxylate

According to the same manner as that described in Example 4, the title compound (60 mg) as yellow crystals was obtained from tert-butyl 6,7-dichloro-4-hydroxy-2-neopentyl-1-oxo-1,2-dihydro-3-isoquinolinecarboxylate obtained in Example 3 (350 mg) and indole-3-carbinol (154 mg).
¹H-NMR(CDCl₃) δ : 1.07 (9H, s), 1.49 (9H, s), 3.15 (1H, d, J=14.0 Hz), 3.56 (1H, d, J=15.5 Hz), 3.82-3.92 (2H, m), 6.73 (1H, d, J=2.3 Hz), 6.97-7.13 (3H, m), 7.35 (1H, d, J=7.2 Hz), 7.62 (1H, s), 7.87 (1H, brs), 8.01 (1H, s).

### Example 28

### Methyl 6,7-dichloro-4-methoxy-2-neopentyl-1-oxo-1,2-dihydro-3-isoquinolinecarboxylate

According to the same manner as that described in Example 5, the title compound (250 mg) as colorless crystals was obtained from methyl 6,7-dichloro-4-hydroxy-2-neopentyl-1-oxo-1,2-dihydro-3-isoquinolinecarboxylate obtained in Example 26 (2.00 g) and methyl iodide (1.59 g). ¹H-NMR(CDCl₃) δ : 0.92 (9H, s), 3.87 (3H, s), 3.98 (3H, s), 4.06 (2H, br), 7.86 (1H, s), 8.52 (1H, s).

### Preparation Example 1

An RFRP receptor function modulating agent containing the compound represented by the formula (I) of the present invention or a salt thereof as an active ingredient can be prepared, for example, by the following formulation.

### 1. Capsule

| | | |
|---|---|---|
| (1) | Compound obtained in Example 1 | 40 mg |
| (2) | Lactose | 70 mg |
| (3) | Microcrystalline cellulose | 9 mg |
| (4) | Magnesium stearate | 1 mg |
| | one capsule | 120 mg |

(1), (2) and (3), and 1/2 of (4) are kneaded, and this is granulated. To this is added the remaining (4), and a whole is encapsulated into a gelatin capsule.

### 2. Tablet

| | | |
|---|---|---|
| (1) | Compound obtained in Example 1 | 40 mg |
| (2) | Lactose | 58 mg |
| (3) | Corn starch | 18 mg |
| (4) | Microcrystalline cellulose | 3.5 mg |
| (5) | Magnesium stearate | 0.5 mg |
| | one tablet | 120 mg |

(1), (2), (3), 2/3 of (4), and 1/2 of (5) are kneaded, and this is granulated. The remaining (4) and (5) are added to this granule, and this is pressure-molded into a tablet.

### Preparation Example 2

After 50 mg of the compound obtained in Example 1 is dissolved in 50 ml of Japanese Pharmacopoeia distilled water for injection, Japanese Pharmacopoeia distilled water for injection is added to 100 ml. This solution is filtered under the sterilization condition, then, each 1 ml of this solution is taken, filled into a vial for injection under the sterilization condition, and this is lyophilized, and sealed.

### Test Example 1

### Assessment of binding inhibiting activity of test compound using human-type OT7T022-expressing CHO cell

### (1) Preparation of iodine-labeled entity of human-type RFRP-3 (Y-RFRP-3)

Twenty µm of a peptide (Y-RFRP-3) (sequence: Tyr-Val-Pro-Asn-Leu-Pro-Gln-Arg-Phe-amide) (0.1 mM) in which a Tyr residue had been added to a N-terminus of hRFRP-3-8 (sequence: Val-Pro-Asn-Leu-Pro-Gln-Arg-Phe-amide) having binding inhibiting activity equivalent to that of endogenous human-type RFRP-3 (hRFRP-3-28) for human-type OT7T022-expressing CHO cell and 10 µL of distilled water were mixed, 20 µL of a lactoperoxidase solution (Sigma, prepared using 0.1 M HEPES-NaOH, pH 7.0 in 10 µg/mL), 10 µL of Idoine-125 (Amersham, IMS-30, 74 MBq), and 20 µL of 0.005% hydrogen peroxide (Wako Pure Chemical Industries, Ltd.) were successively mixed therein, the mixture was allowed to stand at room temperature for 10 minutes, 600 µL of 0.1% TFA-water was added, this was separated by reverse phase HPLC, a peak of a labeled entity was collected, an equivalent amount of a buffer for a binding test (50 mM TrisHCl (pH 7.5), 0.1% BSA, 5 mM EDTA, 0.5 mM PMSF, 20 µg/mL leupeptin, 0.1 µg/mL pepstatin A, 4 µ/mL E-64) was added, and this was immediately stored on ice. A part thereof was 1/100 diluted, radioactivity was measured with a γ-counter, and the remaining authentic product was distributed, which was stored at -30°C.

### (2) Assessment of binding inhibiting activity

To a 96-well microplate were added 1 µg of a membrane fraction, a compound and Y-RFRP-3 labeled with ¹²⁵I, which had been diluted with a reaction buffer (50 mM Tris-HCl, 5 mM EDTA, 0.1% BSA, 0.5 mM PMSF, 20 µg/ml leupeptin, 0.1 µg/ml pepstatin A, 4 µg/ml E-64, 10 mM MgCl₂, pH 7.5), to 100pM to react them at room temperature for 1.5 hours. For measuring non-specific binding, non-labeled Y-RFRP-3 was further added to 100 pM. Then, a membrane fraction was transferred to a unifilter GF/C (Perkin Elmer) by filtering the reaction solution using a cell harvester (Perkin Elmer), and this was washed with a cooled 50 mM Tris buffer (pH 7.5) five times. The filter was dried, Microsinti (Packard) was added to the filter, and radioactivity was measured with Topcount (Packard).

A binding inhibiting rate (IC₅₀ value) of a test compound is shown in [Table 1].

**[Table 1]**

| Test compound | IC₅₀ value |
|---|---|
| Reference Example 12 | <1 µM |
| Example 1 | <1 µM |
| Example 3 | <1 µM |
| Example 10 | <1 µM |

From this, it is seen that the compound (I) of the present invention has the excellent RFRP receptor binding activity.

### Test Example 2

### Test of antagonist activity of compound in cAMP production inhibiting test system using human-type OT7T022-expressing CHO cell

Antagonist activity of a test compound was measured in an intracellular cAMP production inhibiting test system of a CHO cell expressing human-type OT7T022. In the cAMP production inhibiting test, as an assay buffer, a Hanks' balanced salt solution (Gibco) to which 20 mM HEPES pH 7.4, 0.1% bovine serum albumin, and 0.2 mM 3-isobutyl-1-methylxanthine (Sigma) had been added, was used. A sample compound was adjusted with an assay buffer so that the final concentration became 10⁻⁵M, 10⁻⁶M, 10⁻⁷M, 10⁻⁸M, or 10⁻¹⁰M. An agonist: human-type RFRP-3-8 (Val-Pro-Asn-Leu-Pro-Gln-Arg-Phe-amide) was diluted with an assay buffer obtained by adding 4 µM (final concentration 2 µM) forscoline to 40 nM (final concentration 20 nM). A human-type OT7T022-expressing CHO cell was passaged on a 96-well plate at 4×10⁴/well, and cultured at 37°C, 5% CO₂ and 95% air for one day. The plate which had been cultured for one day was washed with an assay buffer (150 µl) two times and, after 30 minutes, this was cultured for 30 minutes at 37°C and 100% air. After washed with an assay buffer (150 µl) two times, 50 µl of a sample compound solution and, then, 50 µl of an agonist + forskolin solution were added, the mixture was stirred well and, after 30 minutes, this was cultured for 30 minutes at 37°C and 100% air. An intracellular cAMP amount was measured according to a protocol of the present kit using cAMP-Screen™ system (ABI).

The antagonist activity of the test compound is shown in [Table 2].

**[Table 2]**

| Test compound | IC₅₀ value |
|---|---|
| Example 1 | <5 µM |
| Example 3 | <5 µM |

From this, it is seen that the compound (I) of the present invention has the excellent RFRP receptor antagonism.

### Test Example 3

### Assessment of binding inhibiting activity of test compound using rat-type OT7T022-expressing CHO cell

### (1) Preparation of rat-type OT7T022-expressing CHO cell membrane fraction

A flask in which a rat-type OT7T022-expressing CHO cell had been cultured was washed with 5 mM EDTA/PBS, cells were peeled with 5 mM EDTA/PBS, and cells were recovered by centrifugation, suspended in 25 mL of a buffer for preparing a membrane fraction (50 mM Tris-HCl, pH 7.5, 5 mM EDTA, 0.5 mM PMSF (manufactured by Wako Pure Chemical Industries, Ltd.), 20 µg/ µL leupeptin (manufactured by Peptide Laboratory), 0.1 µg/mL pepstatin A (manufactured by Peptide Laboratory), 4 µg/mL E-64 (manufactured by Peptide Laboratory)), and homogenized on an ice using Polytron (12,000 rpm, 15 seconds × 3 times). This was centrifuged in a high speed cooling centrifuge at 4°C and 1,000 g for 10 minutes, and the supernatant was recovered. 25 mL of a buffer for preparing a membrane fraction was added to precipitates, and the supernatant was recovered by the same procedure. These supernatants were combined, subjected to a cell strainer, dispensed into tubes for a supercentrifuge, and centrifuged at 4°C and 100,000 g for 1 hour. The pellet was suspended in a small amount of a buffer for preparing a membrane fraction, this was suspended using a Teflon (registered trade mark) homogenizer, a protein amount was measured using a part, and the remaining was dispensed, and stored at -80°C.

### (2) Test of binding inhibition of a sample compound for rat-type OT7T022-expressing CHO cell membrane fraction

Using an assay buffer (50 mM Tris-HCl, pH 7.5, 5 mM EDTA, 0.5 mM PMSF, 20 µg/mL leupeptin, 0.1 µg/mL pepstatinA, 4 µg/mL E-64, 0.1% bovine serum albumin, 10 mM MgCl₂), a membrane fraction of a rat-type OT7T022-expressing CHO cell was diluted to the final concentration of 0.75 µg/well, and iodine-labeled Y-RFRP-3 was diluted to the final concentration of 100 pM. Regarding a sample compound, a 10⁻²M or 10⁻³M stock solution was diluted with an assay buffer so that the final concentration became 10⁻⁵M, 10⁻⁶M, 10⁻⁷M, 10⁻⁸M, 10⁻⁹M, 10⁻¹⁰M or 10⁻¹¹M. For non-specific binding, hRFRP-3-8 having the final concentration of 10⁻⁵M was prepared. Using a polypropylene 96-well plate, a prepared sample solution, a non-specific binding solution, and 50 µl of an assay buffer for total binding were dispensed, 25 µl of an iodine-labeled entity diluent was added, the mixture was stirred, 25 µl of a rat-type OT7T022-expressing CHO cell membrane fraction solution was dispensed, and this was stirred, and incubated at room temperature for 1.5 hours. This was transferred to a unifilter (Perkin Elmer) pre-wetted with a washing buffer (50 mM Tris-HCl, pH 7.5) using a harvester for a 96-well plate (Packard), and this was washed with a washing buffer six times, and sufficiently dried. 50 µl of Microscinti O (Packard) was dispensed, radioactivity was measured with Top Count (Packard), and data were analyzed in a triplicate manner. Binding inhibiting activity (IC₅₀ value) of a test compound for rat-type OT7T022 is shown in [Table 3].

**[Table 3]**

| Test compound | IC₅₀ value |
|---|---|
| Example 1 | <1 µM |
| Example 3 | <1 µM |

From this, it is seen that the compound (I) of the present invention has the excellent antagonism also for a rat-type RFRP receptor.

### Test Example 4

### Blood glucose concentration increasing activity of RFRP

As RFRP, human RFRP-1 (37 amino acids) consisting of an amino acid sequence of 56^{th} (Ser) to 92^{nd} (Phe) of an amino acid sequence represented by SEQ ID No.: 3. Hereinafter, this peptide is abbreviated as RFRP-1.

In order to study influence of peripheral administration of RFRP-1 on a blood glucose concentration, operation for collecting blood under free moving was performed. A mature Wistar male rat (weight 310 to 350 g at operation) was anesthetized with 50 mg/kg of pentobarbital by intraperitoneal administration. The rat was fixed on an anatomic pad in a supine position, and a left jugular vein was exposed. A polyethylene tube SP35 (internal diameter 0.5 mm, external diameter 0.9 mm, Natsume Seisakusho) was cut into a length of about 30 cm, and this was filled with a 200 unit/ml heparin-containing physiological saline, inserted into a jugular vein by about 4.5 cm, and fixed therein. Another end of a tube was exposed at a neck part (back side) through a part under a skin on a back side.

Overnight after operation, 300 µl of blood was collected using a tuberculin injection syringe having a dose of 1 ml and a 25 gauge injection needle (both Telmo) before administration of RFRP-1. In order to prevent blood coagulation, 3 µl of a 300 KIU/ml aprotinin solution containing 3 mg/ml EDTA had been placed into an injection syringe in advance. An Otsuka physiological saline or RFRP-1 (Peptide Laboratory) (17, 80, 170 nmol) dissolved in 1 mL of a physiological saline was intravenously administered through a tube at 1 mL/Kg. After 0, 5, 15, 30 and 60 minutes from initiation of intravenous administration, each 300 µL of blood was collected from a jugular vein. The collected blood was centrifuged (13,000 rpm, 5 minutes) using a minor amount high speed cooling centrifuge (MR-150, Tommy Seiko), and the supernatant (plasma) was recovered. A blood glucose concentration was measured using Fuji Drichem 3500 (FUJIFILM). As shown in Fig. 1, an RFRP-1 10 nmol/kg administration group showed significant (p<0.05, n = 4) activity of increasing a blood glucose concentration 5 minutes and 15 minutes after intravenous administration as compared with a physiological saline administration group.

### Test Example 5

### Pancreatic glucagon secretion promoting activity of RFRP

In order to study mechanism of blood glucose concentration increasing activity of RFRP-1, influence of RFRP-1 on a blood concentration of glucagon and insulin which are known as a hormone imparting a variation to a blood glucose concentration was studied. A mature Wistar male rat (weight 310 to 350 g at operation) was operated for collecting blood under free moving. Overnight after operation, 300 µl of a blood was collected using a tuberculin injection syringe having a dose of 1 ml and a 25 gauge injection needle (both Telmo) before administration of RFRP-1. In order to prevent blood coagulation, 3 µl of a 300 KIU/ml aprotinin solution containing 3 mg/ml EDTA had been placed into an injection syringe in advance. An Otsuka physiological saline or RFRP-1 dissolved in a physiological saline (80 nmol/mL) was intravenously administered through a tube at 1 mL/Kg. After 1, 3, 5 and 15 minutes from initiation of intravenous administration, each 300 µL of blood was collected from a jugular vein. The collected blood was centrifuged (13,000 rpm, 5 minutes) using a minor amount high speed cooling centrifuge (MR-150, Tommy Seiko), and the supernatant (plasma) was recovered. A blood glucagon concentration was measured using a glucagons kit ("First" (Daiichi Radioisotope Laboratory), and a blood insulin concentration was measured using rat insulin [¹²⁵I], an assay system (Amersham Biosciences). As shown in Fig. 2, in an RFRP-1 administration group, significant (p<0.01) increase in a blood glucagon concentration was recognized two minutes after administration as compared with a physiological saline administration group and, also 5 minutes after administration, significant (p<0.01) increase was continued. On the other hand, a variation due to administration of RFRP-1 was not recognized in a blood insulin concentration (Fig. 3). From these results, and from the fact that, in an RFRP-1 administration group, increase in a blood glucose concentration is seen after increase in a blood glucagon concentration, it was thought that activity of increasing a blood glucose concentration due to intravenous administration of RFRP-1 is caused by glucagon secretion of stimulation by RFRP-1.

### Test Example 6

### Amnesia promoting activity of RFRP

Since an RFRP nerve is projected on amygdaloid complex, in order to study involvement of RFRP in amygdaloid complex-dependent memory•learning ability, influence under cued fear conditioning due to ventricle administration of RFRP-1 was studied. A mature Wistar male rat (weight 280 to 320 g at operation) was anesthetized with 50 mg/kg of pentobarbital by intraperitoneal administration, and a rat was fixed in a brain localization apparatus. A tooth cutting bar was set lower by 3.3 mm from an interoralline. A cranial bone was exposed, and a hole was opened in a bone using a dental drill in order to embed a guide cannulae AG-12 (internal diameter 0.4 mm, external diameter 0.5 mm, Eicom) in a ventricle. In addition, an anchor screw was embedded in four places at a periphery thereof. A stainless guide cannulae AG-12 was inserted so that its tip was positioned at an upper part of a side ventricle. A localization coordinate was set to be AP: -0.8 mm, L: 1.5 mm, H: 4.5 mm from a Blegma according to Atlas of Paxinos and Watson (1986). A guide cannulae was fixed at a cranial bone with an instant adhesive and a dental cement, and an anchor screw. A stainless dummy cannulae AD-12 (external diameter 0.35 mm, Eicon) was inserted into a guide cannulae, and fixed with a capnight (Eicom). After operation, a rat was raised in a separate cage. A recovery term was set to be one week after operation, during which sufficient handling was performed.

In cued fear conditioning, first, as a training session, a rat was placed into a shock chamber, and was acclimated for 2 minutes and, immediately after impartation of sound stimulation for 30 seconds, a cycle giving electric stimulation 2.5 mA for 2 seconds and giving rest for 28 seconds was repeated five times (total 5 minutes). After test, the rat was released in a chamber for 2 minutes, and returned to an original cage. Then, as a test session, 24 hours (1 day) and 48 hours (2 day) after the aforementioned training, the rat was placed into the same chamber as that at training, sound stimulation for 30 seconds was given at the same timing as that of training five times, the rat was placed into a chamber, and behavior for 5 minutes was observed. Behavior analysis was used using analysis software Freeze Frame (Actimetric). When behavior of a variation rate of 15 or lower was observed by sound stimulation, it was defined as freezing. RFRP-1 (3 nmol) and a physiological saline (Otsuka Seiyaku) were administered into a ventricle before and after training, and before a test. The number of experimental animals was 12 in each group. As condition of the present test, a route when a test animal is brought to a test chamber was changed every time, and the animal was made to wait in a room other than a room in which a test was performed. As shown in Fig. 4, in an RFRP-1 administration group, a rate of freezing was remarkably reduced on 2 day as compared with a physiological saline administration group (physiological saline administration group; 46.5%, RFRP administration group; 35.5%). From these results, it was seen that RFRP-1 shows amnesia promoting activity.

### Industrial Applicability

Since the compound (I) of the present invention or a salt thereof or a prodrug thereof has the excellent RFRP receptor function modulating activity, and exhibits the excellent oral absorbing property, it is used as a safe and effective drug, and is used as an analgesic, an agent for promoting analgesic activity of morphine, an agent for avoiding resistance due to morphine, an agent for modulating prolactin secretion, an agent for inhibiting pancreatic glucagon secretion, a blood glucose lowering agent, a urine production inhibiting agent, or a bladder constriction inhibiting agent.

## Claims

1. An agent for modulating the function of an RFRP receptor, which comprises a compound represented by the formula: 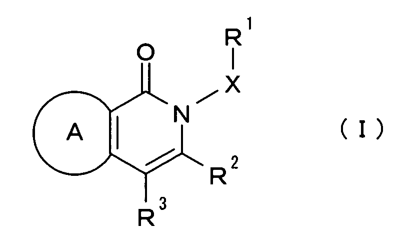 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof.

2. The agent according to claim 1, wherein R³ is an optionally substituted hydroxy group.

3. The agent according to claim 1, which comprises a compound represented by the formula: 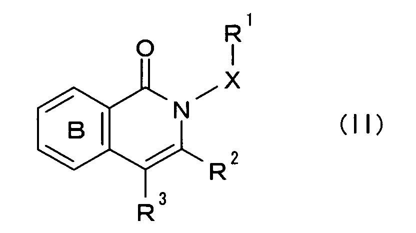 wherein a ring B represents an optionally substituted benzene ring; and the other symbols are as defined in claim 1, or a salt thereof, or a prodrug thereof.

4. The agent according to claim 1, which comprises a compound represented by the formula: 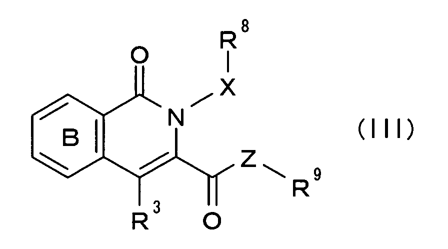 wherein a ring B represents an optionally substituted benzene ring; Z represents a bond, an optionally substituted alkylene group, O, S or NR¹⁰ (R¹⁰ represents a hydrogen atom, an optionally substituted hydrocarbon group, or an optionally substituted heterocyclic group); R⁸ and R⁹ each represents an optionally substituted branched hydrocarbon group; and the other symbols are as defined in claim 1, or a salt thereof, or a prodrug thereof.

5. The agent according to claim 1, which comprises a compound represented by the formula: 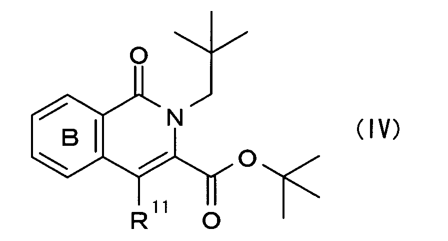 wherein a ring B represents an optionally substituted benzene ring; and R¹¹ represents an optionally substituted hydroxy group, or a salt thereof, or a prodrug thereof.

6. The agent according to claim 1, which is an analgesic, an agent for promoting analgesic activity of another analgesic drug, or an agent for avoiding resistance due to another analgesic drug.

7. The agent according to claim 1, which is an agent for modulating the prolactin secretion.

8. The agent according to claim 1, which is an agent for preventing or treating hyperprolactinemia, pituitary gland tumor, diencephalons tumor, emmeniopathy, stress, autoimmune disease, prolactinoma, infertility, impotence, amenorrhea, galactic leakage, acromegaly, Chiari-Frommel syndrome, Argonz-del Castilo syndrome, Forbes-Albright syndrome, breast cancer lymphoma, Sheehan's syndrome or spermatogenesis abnormality.

9. The agent according to claim 1, which is a pancreatic glucagon secretion inhibiting agent, a blood glucose lowering agent or a urine production inhibiting agent.

10. The agent according to claim 1, which is an agent for preventing or treating diabetes, glucose tolerance disorder, ketosis, acidosis, diabetic neuropathy, diabetic nephropathy, diabetic retinopathy, pollakiuria, nocturnal enarusis, hyperlipemia, sexual function disorder, skin disease, arthritis, osteopenia, arteriosclerosis, thrombotic disease, maldigestion or memory and learning disabilities.

11. The agent according to claim 1, which is a bladder constriction inhibiting agent.

12. The agent according to claim 1, which is an agent for preventing or treating urine incontinence, lower uropathy, urge micturition due to excessive active bladder, or hypotonic bladder accompanied with excessive active bladder.

13. A compound represented by the formula: 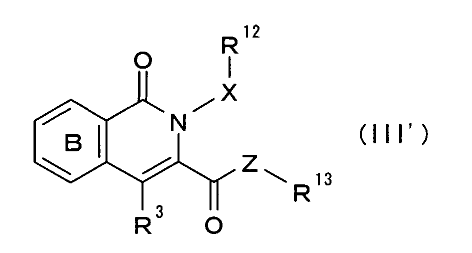 wherein a ring B represents an optionally substituted benzene ring; X represents a bond, O, NR⁴ (R⁴ represents a hydrogen atom, an optionally substituted hydrocarbon group, and an optionally substituted heterocyclic group), or an optionally substituted alkylene group; Z represents a bond, an optionally substituted alkylene group, O, S or NR¹⁰ (R¹⁰ represents a hydrogen atom, an optionally substituted hydrocarbon group, or an optionally substituted heterocyclic group); R¹² and R¹³ each represents an optionally substituted C₃ or higher hydrocarbon group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, provided that tert-butyl 6-fluoro-4-hydroxy-2-neopentyl-1-oxo-1,2-dihydro-3-isoquinolinecarboxylate, tert-butyl 4-butoxy-6-fluoro-2-neopentyl-1-oxo-1,2-dihydro-3-isoquinolinecarboxylate, tert-butyl 7-benzyloxy-4-hydroxy-2-isobutyl-1-oxo-1,2-dihydro-3-isoquinolinecarboxylate and tert-butyl 6-benzyloxy-4-hydroxy-2-isobutyl-1-oxo-1,2-dihydro-3-isoquinolinecarboxylate are excluded.

14. The compound according to claim 13, wherein X is a methylene group.

15. The compound according to claim 13, wherein Z is an oxygen atom.

16. The compound according to claim 13, wherein R¹² is a tert-butyl group.

17. The compound according to claim 13, wherein R¹³ is a tert-butyl group.

18. The compound according to claim 13, wherein R³ is an optionally substituted hydroxy group.

19. The compound according to claim 13, which is represented by the formula: 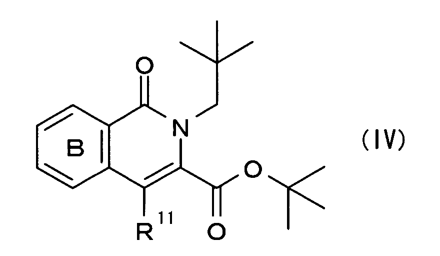 wherein a ring B represents an optionally substituted benzene ring; and R¹¹ represents an optionally substituted hydroxy group.

20. (i) Ethyl 7-bromo-4-hydroxy-2-neopentyl-1-oxo-1,2-dihydroisoquinoline-3-carboxylate, (ii) ethyl 8-hydroxy-6-neopentyl-5-oxo-5,6-dihydro[1,3]dioxolo[4,5-g]isoquinoline-7-carboxylate, (iii) N-{2-[benzyl(methyl)amino]ethyl}-6,7-dichloro-4-methoxy-2-neopentyl-1-oxo-1,2-dihydro-3-isoquinolinecarboxamide, (iv) methyl 6,7-dichloro-4-hydroxy-2-neopentyl-1-oxo-1,2-dihydro-3-isoquinolinecarboxylate, (v) methyl 6,7-dichloro-4-methoxy-2-neopentyl-1-oxo-1,2-dihydro-3-isoquinolinecarboxylate, or a salt thereof.

21. A prodrug of the compound according to claim 13 or 20.

22. A drug comprising the compound according to claim 13 or 20 or a prodrug thereof.

23. The drug according to claim 22, which is an agent for preventing or treating RFRP-relating disease states or diseases involving RFRP.

24. A method of modulating the function of an RFRP receptor, which comprises administering an effective amount of a compound represented by the formula: 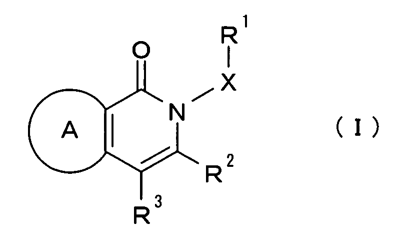 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof to a mammal.

25. Use of a compound represented by the formula: 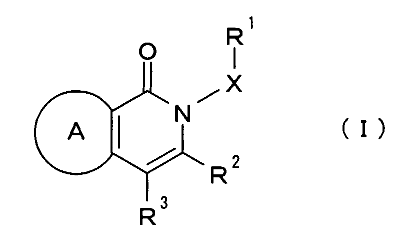 wherein a ring A represents an optionally substituted aromatic ring; X represents a bond, O, NR⁴ (R⁴ represents a hydrogen atom, an optionally substituted hydrocarbon group, or an optionally substituted heterocyclic group), or an optionally substituted alkylene group; R¹ represents an optionally substituted hydrocarbon group, or an optionally substituted heterocyclic group; R² represents a group represented by the formula -COYR⁵ (Y represents a bond, an optionally substituted alkylene group, O, S or NR⁶ (R⁶ represents a hydrogen atom, an optionally substituted hydrocarbon group, or an optionally substituted heterocyclic group), and R⁵ represents an optionally substituted hydrocarbon group, or an optionally substituted heterocyclic group), an optionally substituted hydrocarbon group, or an optionally substituted heterocyclic group; and R³ represents an optionally substituted hydrocarbon group, an optionally substituted heterocyclic group, an optionally substituted hydroxy group, an optionally substituted amino group, or a group represented by the formula -S(O)ₙR⁷ (R⁷ represents an optionally substituted hydrocarbon group, or an optionally substituted heterocyclic group, and n is an integer of 0 to 2), or a salt thereof, or a prodrug thereof for preparing an agent for modulating the function of an RFRP receptor.


SEQUENCE LISTING
<110> Takeda Chemical Industries, Ltd.
<120> RFRP Receptor Antagonist
<130> 3163WOOP
<150> JP 2003-72709
<151> 2003-03-17
<160> 3
<210> 1
<211> 430
<212> PRT
<213> Homo sapiens
<220>
<400> 1
Met Glu Gly Glu Pro Ser Gln Pro Pro Asn Ser Ser Trp Pro Leu Ser
1               5                   10                  15
Gln Asn Gly Thr Asn Thr Glu Ala Thr Pro Ala Thr Asn Leu Thr Phe
            20                  25                  30
Ser Ser Tyr Tyr Gln His Thr Ser Pro Val Ala Ala Met Phe Ile Val
        35                  40                  45
Ala Tyr Ala Leu Ile Phe Leu Leu Cys Met Val Gly Asn Thr Leu Val
    50                  55                  60
Cys Phe Ile Val Leu Lys Asn Arg His Met His Thr Val Thr Asn Met
65                  70                  75                  80
Phe Ile Leu Asn Leu Ala Val Ser Asp Leu Leu Val Gly Ile Phe Cys
                85                  90                  95
Met Pro Thr Thr Leu Val Asp Asn Leu Ile Thr Gly Trp Pro Phe Asp
            100                 105                 110
Asn Ala Thr Cys Lys Met Ser Gly Leu Val Gln Gly Met Ser Val Ser
        115                 120                 125
Ala Ser Val Phe Thr Leu Val Ala Ile Ala Val Glu Arg Phe Arg Cys
    130                 135                 140
Ile Val His Pro Phe Arg Glu Lys Leu Thr Leu Arg Lys Ala Leu Val
145                 150                 155                 160
Thr Ile Ala Val Ile Trp Ala Leu Ala Leu Leu Ile Met Cys Pro Ser
                165                 170                 175
Ala Val Thr Leu Thr Val Thr Arg Glu Glu His His Phe Met Val Asp
            180                 185                 190
Ala Arg Asn Arg Ser Tyr Pro Leu Tyr Ser Cys Trp Glu Ala Trp Pro
        195                 200                 205
Glu Lys Gly Met Arg Arg Val Tyr Thr Thr Val Leu Phe Ser His Ile
    210                 215                 220
Tyr Leu Ala Pro Leu Ala Leu Ile Val Val Met Tyr Ala Arg Ile Ala
225                 230                 235                 240
Arg Lys Leu Cys Gln Ala Pro Gly Pro Ala Pro Gly Gly Glu Glu Ala
                245                 250                 255
Ala Asp Pro Arg Ala Ser Arg Arg Arg Ala Arg Val Val His Met Leu
            260                 265                 270
Val Met Val Ala Leu Phe Phe Thr Leu Ser Trp Leu Pro Leu Trp Ala
        275                 280                 285
Leu Leu Leu Leu Ile Asp Tyr Gly Gln Leu Ser Ala Pro Gln Leu His
    290                 295                 300
Leu Val Thr Val Tyr Ala Phe Pro Phe Ala His Trp Leu Ala Phe Phe
305                 310                 315                 320
Asn Ser Ser Ala Asn Pro Ile Ile Tyr Gly Tyr Phe Asn Glu Asn Phe
                325                 330                 335
Arg Arg Gly Phe Gln Ala Ala Phe Arg Ala Arg Leu Cys Pro Arg Pro
            340                 345                 350
Ser Gly Ser His Lys Glu Ala Tyr Ser Glu Arg Pro Gly Gly Leu Leu
        355                 360                 365
His Arg Arg Val Phe Val Val Val Arg Pro Ser Asp Ser Gly Leu Pro
    370                 375                 380
Ser Glu Ser Gly Pro Ser Ser Gly Ala Pro Arg Pro Gly Arg Leu Pro
385                 390                 395                 400
Leu Arg Asn Gly Arg Val Ala His His Gly Leu Pro Arg Glu Gly Pro
                405                 410                 415
Gly Cys Ser His Leu Pro Leu Thr Ile Pro Ala Trp Asp Ile
            420                 425                 430
<210> 2

<211> 432
<212> PRT
<213> Rattus sp
<400> 2
Met Glu Ala Glu Pro Ser Gln Pro Pro Asn Gly Ser Trp Pro Leu Gly
                5                   10                  15
Gln Asn Gly Ser Asp Val Glu Thr Ser Met Ala Thr Ser Leu Thr Phe
            20                  25                  30
Ser Ser Tyr Tyr Gln His Ser Ser Pro Val Ala Ala Met Phe Ile Ala
        35                  40                  45
Ala Tyr Val Leu Ile Phe Leu Leu Cys Met Val Gly Asn Thr Leu Val
    50                  55                  60
Cys Phe Ile Val Leu Lys Asn Arg His Met Arg Thr Val Thr Asn Met
65                  70                  75                  80
Phe Ile Leu Asn Leu Ala Val Ser Asp Leu Leu Val Gly Ile Phe Cys
                85                  90                  95
Met Pro Thr Thr Leu Val Asp Asn Leu Ile Thr Gly Trp Pro Phe Asp
            100                 105                 110
Asn Ala Thr Cys Lys Met Ser Gly Leu Val Gln Gly Met Ser Val Ser
        115                 120                 125
Ala Ser Val Phe Thr Leu Val Ala Ile Ala Val Glu Arg Phe Arg Cys
    130                 135                 140
Ile Val His Pro Phe Arg Glu Lys Leu Thr Leu Arg Lys Ala Leu Phe
145                 150                 155                 160
Thr Ile Ala Val Ile Trp Ala Leu Ala Leu Leu Ile Met Cys Pro Ser
                165                 170                 175
Ala Val Thr Leu Thr Val Thr Arg Glu Glu His His Phe Met Leu Asp
            180                 185                 190
Ala Arg Asn Arg Ser Tyr Pro Leu Tyr Ser Cys Trp Glu Ala Trp Pro
        195                 200                 205
Glu Lys Gly Met Arg Lys Val Tyr Thr Ala Val Leu Phe Ala His Ile
    210                 215                 220
Tyr Leu Val Pro Leu Ala Leu Ile Val Val Met Tyr Val Arg Ile Ala
225                 230                 235                 240
Arg Lys Leu Cys Gln Ala Pro Gly Pro Ala Arg Asp Thr Glu Glu Ala
                245                 250                 255
Val Ala Glu Gly Gly Arg Thr Ser Arg Arg Arg Ala Arg Val Val His
            260                 265                 270
Met Leu Val Met Val Ala Leu Phe Phe Thr Leu Ser Trp Leu Pro Leu
        275                 280                 285
Trp Val Leu Leu Leu Leu Ile Asp Tyr Gly Glu Leu Ser Glu Leu Gln
    290                 295                 300
Leu His Leu Leu Ser Val Tyr Ala Phe Pro Leu Ala His Trp Leu Ala
305                 310                 315                 320
Phe Phe His Ser Ser Ala Asn Pro Ile Ile Tyr Gly Tyr Phe Asn Glu
                325                 330                 335
Asn Phe Arg Arg Gly Phe Gln Ala Ala Phe Arg Ala Gln Leu Cys Trp
            340                 345                 350
Pro Pro Trp Ala Ala His Lys Gln Ala Tyr Ser Glu Arg Pro Asn Arg
        355                 360                 365
Leu Leu Arg Arg Arg Val Val Val Asp Val Gln Pro Ser Asp Ser Gly
    370                 375                 380
Leu Pro Ser Glu Ser Gly Pro Ser Ser Gly Val Pro Gly Pro Gly Arg
385                 390                 395                 400
Leu Pro Leu Arg Asn Gly Arg Val Ala His Gln Asp Gly Pro Gly Glu
                405                 410                 415
Gly Pro Gly Cys Asn His Met Pro Leu Thr Ile Pro Ala Trp Asn Ile
            420                 425                 430
<210> 3
<211> 180
<212> PRT
<213> Homo sapiens
<400> 3
Met Glu Ile Ile Ser Ser Lys Leu Phe Ile Leu Leu Thr Leu Ala Thr
 1              5                   10                  15
Ser Ser Leu Leu Thr Ser Asn Ile Phe Cys Ala Asp Glu Leu Val Met
            20                  25                  30
Ser Asn Leu His Ser Lys Glu Asn Tyr Asp Lys Tyr Ser Glu Pro Arg

35                      40                      45
Gly Tyr Pro Lys Gly Glu Arg Ser Leu Asn Phe Glu Glu Leu Lys Asp
    50                      55                      60
Trp Gly Pro Lys Asn Val Ile Lys Met Ser Thr Pro Ala Val Asn Lys
65                      70                      75                      80
Met Pro His Ser Phe Ala Asn Leu Pro Leu Arg Phe Gly Arg Asn Val
                85                      90                      95
Gln Glu Glu Arg Ser Ala Gly Ala Thr Ala Asn Leu Pro Leu Arg Ser
            100                     105                     110
Gly Arg Asn Met Glu Val Ser Leu Val Arg Arg Val Pro Asn Leu Pro
        115                     120                     125
Gln Arg Phe Gly Arg Thr Thr Thr Ala Lys Ser Val Cys Arg Met Leu
    130                     135                     140
Ser Asp Leu Cys Gln Gly Ser Met His Ser Pro Cys Ala Asn Asp Leu
145                     150                     155                     160
Phe Tyr Ser Met Thr Cys Gln His Gln Glu Ile Gln Asn Pro Asp Gln
                165                     170                     175
Lys Gln Ser Arg
            180